# EUROPEAN PATENT SPECIFICATION

(11) **EP 4 181 797 B1**
(45) Date of publication and mention of the grant of the patent: **20.08.2025**
(21) Application number: 22729805.6
(22) Date of filing: 25.05.2022
(51) Int. Cl.: A61B 17/072, A61B 17/00, A61B 90/98, A61B 90/00

(54) **STAPLING INSTRUMENT COMPRISING AN ARTICULATION CONTROL DISPLAY**
KLAMMERINSTRUMENT MIT GELENKSTEUERUNGSANZEIGE
INSTRUMENT D'AGRAFAGE COMPRENANT UN DISPOSITIF D'AFFICHAGE À COMMANDE D'ARTICULATION

(30) Priority: 28.05.2021 US 202163194621 P; 18.08.2021 US 202163234396 P; 25.04.2022 US 202217728101
(43) Date of publication of application: 24.05.2023
(73) Proprietor: Cilag GmbH International, 6300 Zug (CH)
(72) Inventor: LEIMBACH, Richard L., Cincinnati, Ohio 45242 (US); MASTROIANNI, Nina, Cincinnati, Ohio 45242 (US); SIDERS, Craig P., Cincinnati, Ohio 45242 (US); GUEST, Timothy L., Cincinnati, Ohio 45242 (US); HUANG, Zhifan F., Cincinnati, Ohio 45242 (US); STOUGHTON, Thomas A., Cincinnati, Ohio 45242 (US); ROSS, Nicholas J., Cincinnati, Ohio 45242 (US); BRUCE, John Kevin, Cincinnati, Ohio 45242 (US)
(74) Representative: Carpmaels & Ransford LLP
(86) International application number: PCT/IB2022/054896
(87) International publication number: WO 2022/249092

(56) References cited:
- EP-A1- 2 839 786
- EP-A1- 3 733 079
- WO-A1-2022/090929
- US-A1- 2011 174 862
- US-A1- 2018 132 850
- US-A1- 2020 054 320

## Description

### BACKGROUND

The present invention relates to surgical instruments and, in various arrangements, to surgical stapling and cutting instruments and staple cartridges for use therewith that are designed to staple and cut tissue.

EP 3 733 079 A1 discloses a surgical instrument comprising a handle, a shaft rotatable relative to the handle, and an articulation joint rotatable relative to the shaft. The instrument comprises a motor-driven articulation system including articulation controls which are flipped by a control system of the surgical instrument when the shaft is rotated upside down relative to the handle.

### SUMMARY OF THE INVENTION

The present invention provides a surgical instrument assembly as recited in claim 1. Optional features are recited in the dependent claims. Aspects, embodiments, examples and methods of the present disclosure which are not claimed per se, are provided for illustrative purposes and are considered useful for giving context to the invention.

### BRIEF DESCRIPTION OF THE DRAWINGS

Various features of the embodiments described herein, together with advantages thereof, may be understood in accordance with the following description taken in conjunction with the accompanying drawings as follows:
FIG. 1 is a perspective view of a surgical instrument in accordance with at least one embodiment;
FIG. 1B is a left side elevation view of the surgical instrument of FIG. 1;
FIG. 1C is a right side elevation view of the surgical instrument of FIG. 1;
FIG. 1D is a front elevation view of the surgical instrument of FIG. 1;
FIG. 1E is a back elevation view of the surgical instrument of FIG. 1;
FIG. 1F is a plan view of the surgical instrument of FIG. 1;
FIG. 1G is a bottom view of the surgical instrument of FIG. 1;
FIG. 2 is a partial perspective view of the surgical instrument of FIG. 1;
FIG. 3 is a partial perspective view of a shaft of the surgical instrument of FIG. 1;
FIG. 4 is a perspective view of a nozzle of the shaft of FIG. 3;
FIG. 5 is an elevational view of an orientation switch of the surgical instrument of FIG. 1;
FIG. 6 is a partial perspective view of a surgical instrument in accordance with at least one embodiment comprising a handle including an orientation sensor and a shaft comprising magnetic elements detectable by the orientation sensor;
FIG. 7 is a partial elevational view of a surgical instrument in accordance with at least one embodiment comprising a handle and articulation actuators on opposing sides of the handle;
FIG. 8 is a partial plan view of the surgical instrument of FIG. 7;
FIG. 9 is a perspective view of a surgical instrument in accordance with at least one embodiment comprising a handle and a rotatable shaft including articulation actuators on opposing sides of the shaft;
FIG. 10 is an end view of the shaft of FIG. 9;
FIG. 11 is a perspective view of a surgical instrument in accordance with at least one embodiment comprising a handle and a rotatable shaft including two articulation actuators on opposing sides of the shaft;
FIG. 12 is an end view of the shaft of FIG. 11;
FIG. 13 is a perspective view of a surgical instrument in accordance with at least one embodiment comprising a slideable articulation actuator including two positions and a detent between the two positions;
FIG. 14 illustrates a capacitive switch including first and second sides, a first light in the first side which illuminates when the first side is contacted, and a second light in the second side which illuminates when the second side is contacted;
FIG. 15 illustrates a two-stage rocker switch for articulating an end effector of a surgical instrument in accordance with at least one embodiment;
FIG. 16 is a partial top view of a surgical instrument in accordance with at least one embodiment comprising an end effector and lights positioned on opposite sides of the end effector which are illuminated to indicate the direction in which the end effector is being articulated;
FIG. 17 is a partial elevational view of the surgical instrument of FIG. 16;
FIG. 18 is a partial elevational view of a surgical instrument in accordance with at least one embodiment comprising directional indicators which are illuminated to indicate which way the end effector is being articulated;
FIG. 19 is a perspective view of a surgical instrument in accordance with at least one embodiment including a slideable articulation switch including three positions - an articulate left position, an articulate right position, and a center, or home, position;
FIG. 20 is an elevational view of a surgical instrument in accordance with at least one embodiment including an articulation joystick actuatable along a longitudinal axis;
FIG. 21 is an elevational view of a surgical instrument in accordance with at least one embodiment including an end effector and an articulation joystick actuatable to articulate the end effector about more than one axis;
FIG. 22A is a front elevational view of a surgical instrument in accordance with at least one embodiment including a plurality of articulation controls;
FIG. 22B is a partial side elevational view of the surgical instrument of FIG. 22A;
FIG. 23 is an elevational view of a surgical instrument in accordance with at least one embodiment including a 4-way tactile articulation control;
FIG. 24 is a partial elevational view of a surgical instrument in accordance with at least one embodiment including a 4-way tactile articulation control including a center, or home, actuator;
FIG. 25 is an elevational view of a surgical instrument in accordance with at least one embodiment including a 4-way capacitive surface;
FIG. 26A illustrates a surgical instrument in accordance with at least one embodiment including an end effector and lights positioned on opposite sides of the end effector which are illuminated to indicate the direction in which the end effector is being articulated;
FIG. 26B is a perspective view of the surgical instrument of FIG. 26A;
FIG. 27 illustrates a surgical instrument in accordance with at least one embodiment including an articulation joint, an end effector articulatable about the articulation joint, and a translatable articulation actuator configured to rotate the end effector about the articulation joint;
FIG. 28 is a partial perspective view of an articulatable end effector, an articulation actuator configured to rotate the end effector about an articulation joint, and demarcations on the articulation actuator which indicate the direction in which an end effector is articulated and/or is being articulated;
FIG. 29 is a perspective view of a surgical instrument in accordance with at least one embodiment comprising a handle, a rotatable shaft extending from the handle, and a rotatable actuator on the handle configured to rotate the shaft about a longitudinal axis;
FIG. 30 is a perspective view of the surgical instrument of FIG. 29 illustrating the shaft in a rotated position;

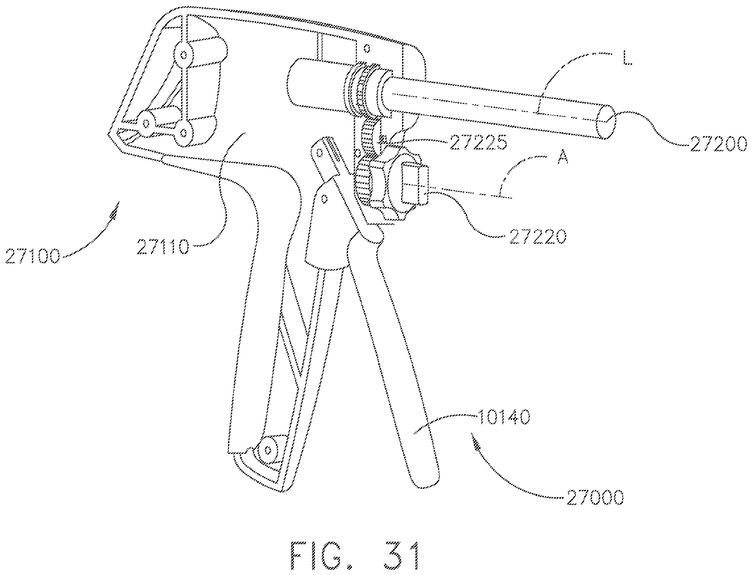

FIG. 31 is a perspective view of the surgical instrument of FIG. 29 illustrated with a portion of the handle housing removed;
FIG. 32 is a partial detail view of the articulation joint of the surgical instrument of FIG. 1 illustrated with some components removed;
FIG. 33 is a partial detail view of an articulation joint in accordance with at least one alternative embodiment usable with the surgical instrument of FIG. 1;
FIG. 34 is a partial perspective view of an articulation drive pin extending from a frame of the end effector of the embodiment of FIG. 33;
FIG. 35 is a partial detail view of the embodiment of FIG. 33 illustrating the end effector in an articulated position;
FIG. 36 is a partial detail view of the embodiment of FIG. 33 illustrating the end effector in another articulated position;
FIG. 37 is a partial detail view of the embodiment of FIG. 33 illustrating the end effector in another articulated position;

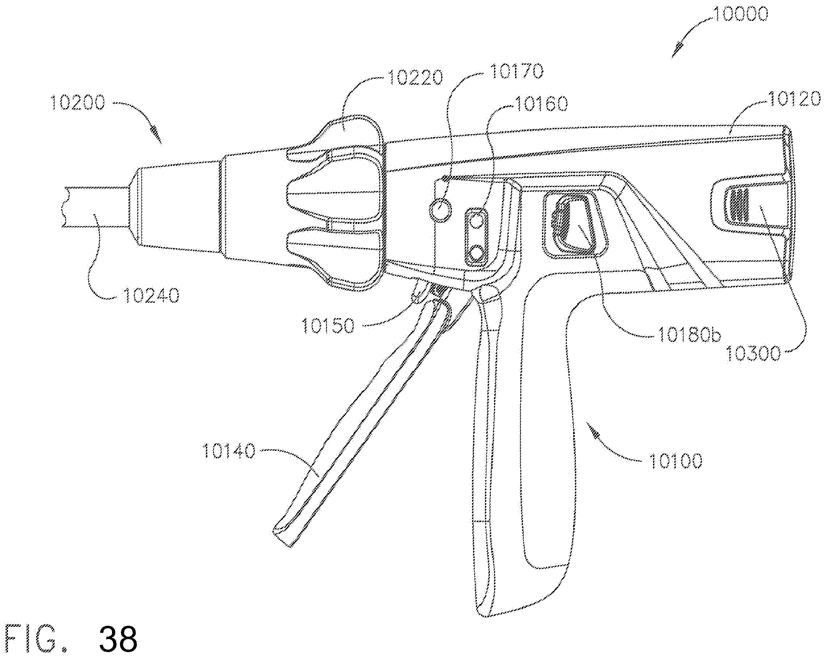

FIG. 38 is a partial elevational view of the surgical instrument of FIG. 1;

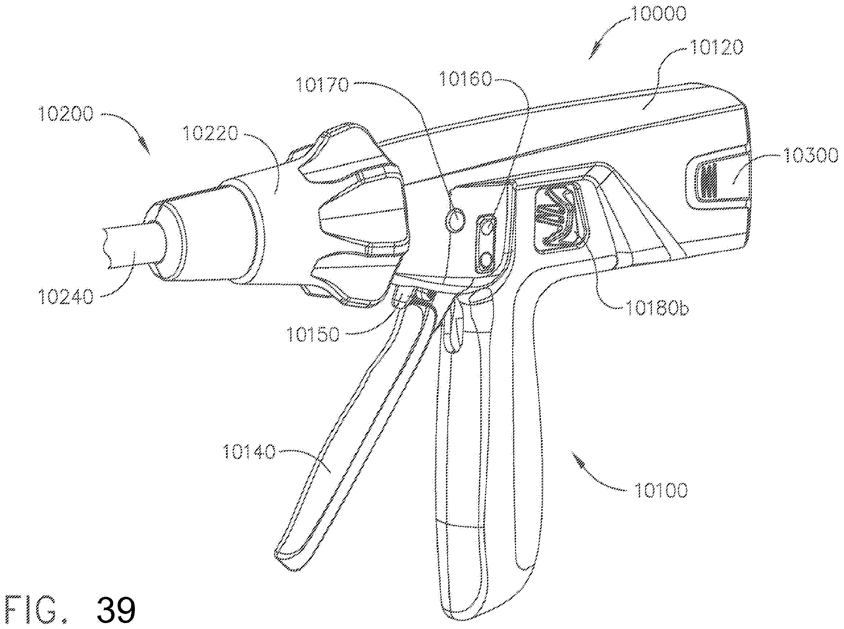

FIG. 39 is a partial perspective view of the surgical instrument of FIG. 1;
FIG. 40 is a partial elevational view of a surgical instrument in accordance with at least one embodiment;
FIG. 41 is a partial perspective view of the surgical instrument of FIG. 40;
FIG. 42 is a partial elevational view of a surgical instrument in accordance with at least one embodiment;
FIG. 43 is a partial perspective view of the surgical instrument of FIG. 42;
FIG. 44 is a perspective view of the surgical instrument of FIG. 1;
FIG. 45 is a partial perspective view of a surgical instrument in accordance with at least one embodiment;
FIG. 46 is a partial perspective view of a shaft of the surgical instrument of FIG. 45;
FIG. 47 is a control algorithm implemented by the surgical instrument of FIG. 45;
FIG. 48 is a partial perspective view of a shaft of a surgical instrument in accordance with at least one embodiment;
FIG. 49 is a partial perspective view of a shaft of a surgical instrument in accordance with at least one embodiment;
FIG. 50 is a partial perspective view of a shaft of a surgical instrument in accordance with at least one embodiment;
FIG. 51 is a partial perspective view of a shaft of a surgical instrument in accordance with at least one embodiment;
FIG. 52 is a perspective view of a slip ring assembly of a surgical instrument in accordance with at least one embodiment;
FIG. 53 is another perspective view of the slip ring assembly of FIG. 52;
FIG. 54 is a perspective view of a shaft component of the surgical instrument of FIG. 52;
FIG. 55 is a partial perspective view of the surgical instrument of FIG. 52;
FIG. 56 is a diagram depicting a shaft orientation sensor array in accordance with at least one embodiment;
FIG. 57 is an elevational view of a surgical instrument including a handle and a shaft in accordance with at least one embodiment;
FIG. 58 is a perspective view of the handle of FIG. 57;
FIG. 59 is a partial perspective view of the handle of FIG. 57 illustrated with some components removed;
FIG. 60 is a partial cross-sectional view of a switch of the handle of FIG. 57;
FIG. 61 is a partial perspective view of a handle and a shaft of a surgical instrument in accordance with at least one embodiment;
FIG. 62 is a partial cross-sectional view of the shaft of FIG. 61 illustrated in a first rotational position;
FIG. 63 is a partial cross-sectional view of the shaft of FIG. 61 illustrated in a second rotational position;
FIG. 64 depicts a control system of the surgical instrument of FIG. 61;
FIG. 65 is an elevational view of the handle of FIG. 57 illustrated with some components removed illustrating a closure actuator of the handle in a partially-closed position;
FIG. 66 is a partial detail view of the closure system of the handle of FIG. 57 illustrated in a partially-closed configuration;
FIG. 67 is a partial detail view of the closure system of the handle of FIG. 57 illustrated in a fully-closed configuration;
FIG. 68 is a partial elevational view of a surgical instrument comprising a handle and a shaft in accordance with at least one embodiment;
FIG. 69 is a partial elevational view of the surgical instrument of FIG. 68 illustrated in a partially-closed configuration;
FIG. 70 is a partial elevational view of the surgical instrument of FIG. 68 illustrated in a fully-closed configuration;
FIG. 71 is a partial elevational view of a surgical instrument comprising a handle and a shaft in accordance with at least one embodiment;
FIG. 72 is a partial elevational view of the surgical instrument of FIG. 71 depicting an actuatable closure lock;
FIG. 73 is a partial elevational view of the surgical instrument of FIG. 57 illustrated with an illuminated articulation control when the closure system is in a fully-closed configuration;
FIG. 74 is a partial elevational view of the surgical instrument of FIG. 57 illustrated with an illuminated articulation control when the closure system is in an open configuration;
FIG. 75 depicts a control system of the surgical instrument of FIG. 57;
FIG. 76 depicts a control system of the surgical instrument of FIG. 57;
FIG. 77 is a perspective view of a surgical instrument comprising a handle, a shaft extending from the handle, and an end effector rotatably connected to the shaft about an articulation joint in accordance with at least one embodiment;
FIG. 78 is an elevational view of the surgical instrument of FIG. 77;
FIG. 79 is a top view of the surgical instrument of FIG. 77;
FIG. 80 is a partial perspective view of the handle of FIG. 77 illustrated with some components removed;
FIG. 81 is a partial perspective view of the handle of FIG. 77 illustrated with additional components removed;
FIG. 82 is a partial perspective view of the handle of FIG. 77 illustrating the motor of the firing drive of the surgical instrument of FIG. 77;
FIG. 83 is an elevational view of the handle of FIG. 77 with the housing removed;
FIG. 84 is a partial perspective view of the surgical instrument of FIG. 77 illustrated with components removed;
FIG. 85 is another partial perspective view of the surgical instrument of FIG. 77 illustrating an orientation switch configured to affect the operation of the articulation controls;
FIG. 86 is a cross-sectional view of the surgical instrument of FIG. 77 illustrating the orientation switch of FIG. 85 in a closed state;
FIG. 87 is a cross-sectional view of the surgical instrument of FIG. 77 illustrating the orientation switch of FIG. 85 in an open state;
FIG. 88 is a partial perspective view of a surgical stapling instrument in accordance with at least one embodiment comprising a handle and a shaft assembly rotatable relative to the handle, wherein the handle comprises an articulation actuator and visual indicators configured to indicate the state of the articulation actuator and to indicate the orientation of the shaft assembly relative to the handle;
FIG. 89 is an elevational view of the surgical stapling instrument of FIG. 88;
FIG. 90 is an elevational view of the surgical stapling instrument of FIG. 88, wherein an indicator light is indicating that the control system of the surgical stapling instrument has modified the output of the control system in response to the articulation actuator;
FIG. 91 is a display of a surgical hub in accordance with at least one embodiment displaying the orientation status of an anvil jaw;
FIG. 92 is a display of a surgical hub in accordance with at least one embodiment displaying the articulation actuator status of a surgical instrument assembly;
FIG. 93 is a partial cross-sectional view of a surgical stapling assembly in accordance with at least one embodiment comprising a handle, a shaft assembly, and a sensing system configured to monitor and detect the orientation of the shaft assembly relative to the handle;
FIG. 94 is a partial cross-sectional view of a surgical stapling assembly in accordance with at least one embodiment comprising a handle, a shaft assembly, and a sensing system configured to monitor and detect the orientation of the shaft assembly relative to the handle;
FIG. 95 is a perspective view of a nozzle of the surgical stapling assembly of FIG. 94;
FIG. 96 is a diagram of a magnet ring of the sensing system of FIG. 94 in the nozzle of FIG. 95;
FIG. 97 is a diagram of a different magnet ring for use with the sensing system of FIG. 94;
FIG. 98 is an elevational view of a surgical stapling instrument in accordance with at least one embodiment comprising a handle, a shaft assembly, and an end effector articulatable relative to the shaft assembly, wherein the surgical stapling instrument further comprises visual indicators configured to indicate to a user of the surgical stapling instrument whether or not the surgical stapling instrument is capable of articulating the end effector; FIG. 98 also illustrates an anvil jaw of the end effector in a fully open position;
FIG. 99 is an elevational view of the surgical stapling instrument of FIG. 98 illustrating the anvil jaw in a first partially-clamped position;
FIG. 100 is an elevational view of the surgical stapling instrument of FIG. 98 illustrating the anvil jaw in a second partially-clamped position;
FIG. 101 is an elevational view of the surgical stapling instrument of FIG. 98 illustrating the anvil jaw in a fully-clamped position; and
FIG. 102 is a partial perspective view of a surgical instrument assembly in accordance with at least one embodiment comprising a closure drive including a closure trigger and a sensing system configured to sense the movement and position of the closure trigger.

Corresponding reference characters indicate corresponding parts throughout the several views. The exemplifications set out herein illustrate various embodiments of the invention, in one form, and such exemplifications are not to be construed as limiting the scope of the invention in any manner.

### DETAILED DESCRIPTION

Numerous specific details are set forth to provide a thorough understanding of the overall structure, function, manufacture, and use of the embodiments as described in the specification and illustrated in the accompanying drawings. Well-known operations, components, and elements have not been described in detail so as not to obscure the embodiments described in the specification. The reader will understand that the embodiments described and illustrated herein are non-limiting examples, and thus it can be appreciated that the specific structural and functional details disclosed herein may be representative and illustrative. Variations and changes thereto may be made without departing from the scope of the claims.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a surgical system, device, or apparatus that "comprises," "has," "includes" or "contains" one or more elements possesses those one or more elements, but is not limited to possessing only those one or more elements. Likewise, an element of a system, device, or apparatus that "comprises," "has," "includes" or "contains" one or more features possesses those one or more features, but is not limited to possessing only those one or more features.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, the reader will readily appreciate that the various methods and devices disclosed herein can be used in numerous surgical procedures and applications including, for example, in connection with open surgical procedures. As the present Detailed Description proceeds, the reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongate shaft of a surgical instrument can be advanced.

A surgical stapling system can comprise a shaft and an end effector extending from the shaft. The end effector comprises a first jaw and a second jaw. The first jaw comprises a staple cartridge. The staple cartridge is insertable into and removable from the first jaw; however, other embodiments are envisioned in which a staple cartridge is not removable from, or at least readily replaceable from, the first jaw. The second jaw comprises an anvil configured to deform staples ejected from the staple cartridge. The second jaw is pivotable relative to the first jaw about a closure axis; however, other embodiments are envisioned in which the first jaw is pivotable relative to the second jaw. The surgical stapling system further comprises an articulation joint configured to permit the end effector to be rotated, or articulated, relative to the shaft. The end effector is rotatable about an articulation axis extending through the articulation joint. Other embodiments are envisioned which do not include an articulation joint.

The staple cartridge comprises a cartridge body. The cartridge body includes a proximal end, a distal end, and a deck extending between the proximal end and the distal end. In use, the staple cartridge is positioned on a first side of the tissue to be stapled and the anvil is positioned on a second side of the tissue. The anvil is moved toward the staple cartridge to compress and clamp the tissue against the deck. Thereafter, staples removably stored in the cartridge body can be deployed into the tissue. The cartridge body includes staple cavities defined therein wherein staples are removably stored in the staple cavities. The staple cavities are arranged in six longitudinal rows. Three rows of staple cavities are positioned on a first side of a longitudinal slot and three rows of staple cavities are positioned on a second side of the longitudinal slot. Other arrangements of staple cavities and staples may be possible.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions by a sled. The sled is movable between a proximal position adjacent the proximal end and a distal position adjacent the distal end. The sled comprises a plurality of ramped surfaces configured to slide under the drivers and lift the drivers, and the staples supported thereon, toward the anvil.

Further to the above, the sled is moved distally by a firing member. The firing member is configured to contact the sled and push the sled toward the distal end. The longitudinal slot defined in the cartridge body is configured to receive the firing member. The anvil also includes a slot configured to receive the firing member. The firing member further comprises a first cam which engages the first jaw and a second cam which engages the second jaw. As the firing member is advanced distally, the first cam and the second cam can control the distance, or tissue gap, between the deck of the staple cartridge and the anvil. The firing member also comprises a knife configured to incise the tissue captured intermediate the staple cartridge and the anvil. It is desirable for the knife to be positioned at least partially proximal to the ramped surfaces such that the staples are ejected ahead of the knife.

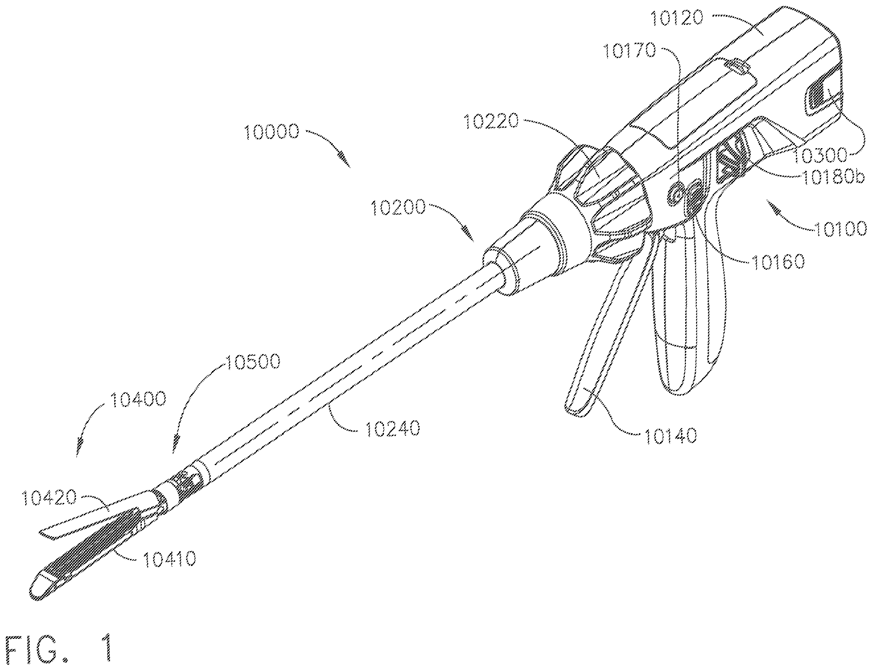

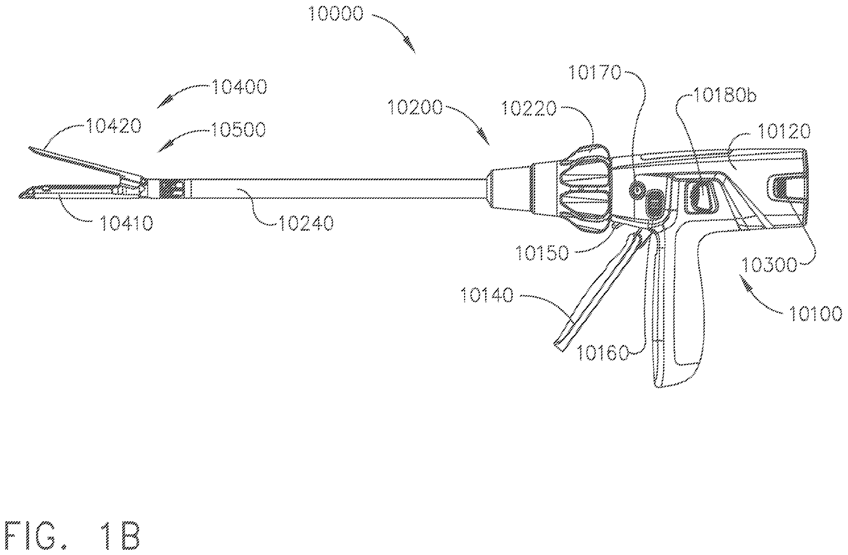

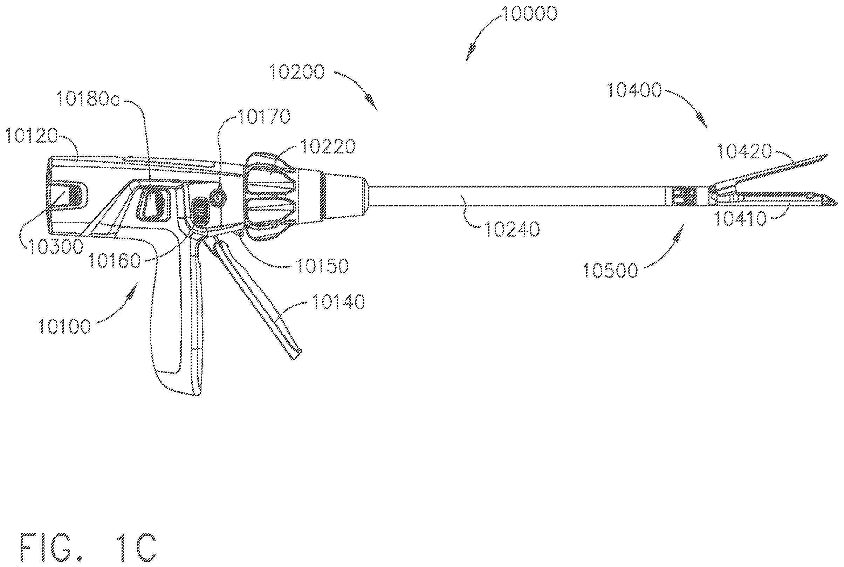

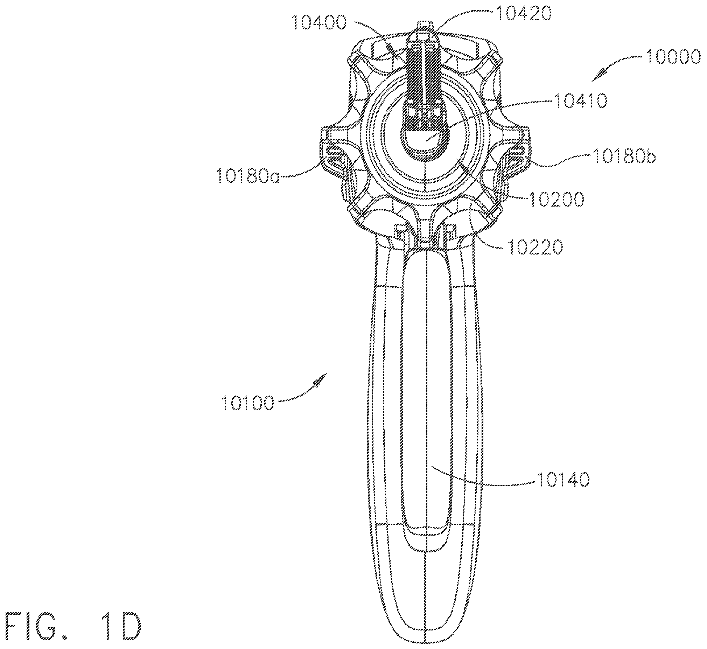

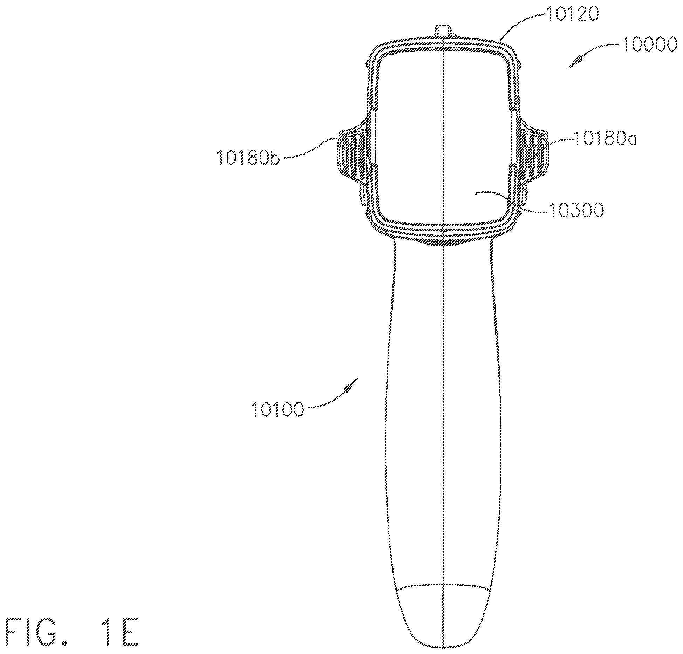

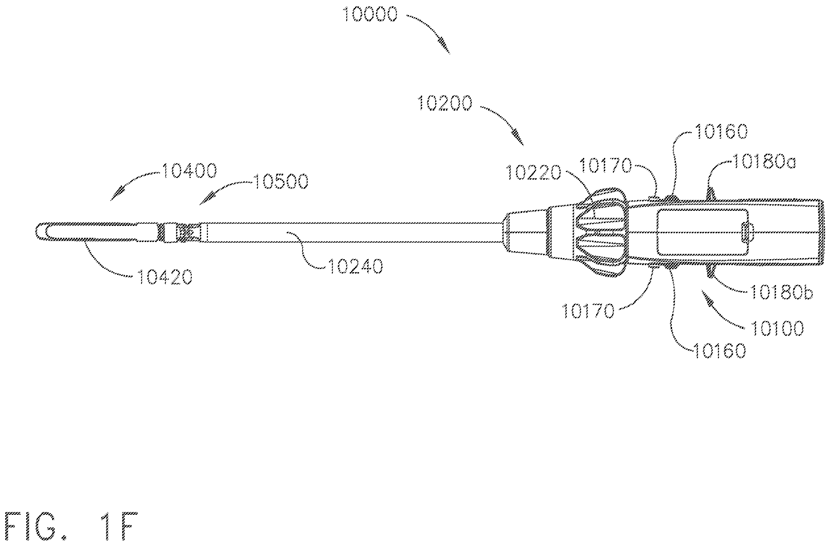

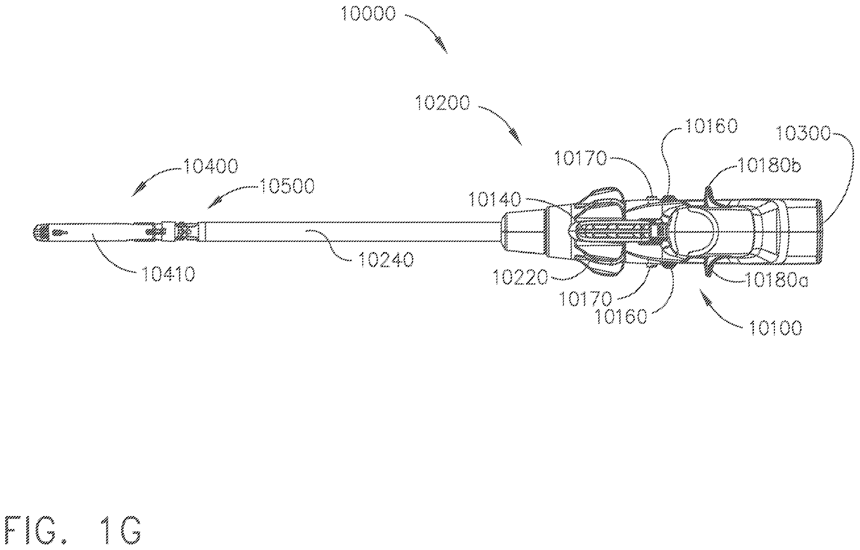

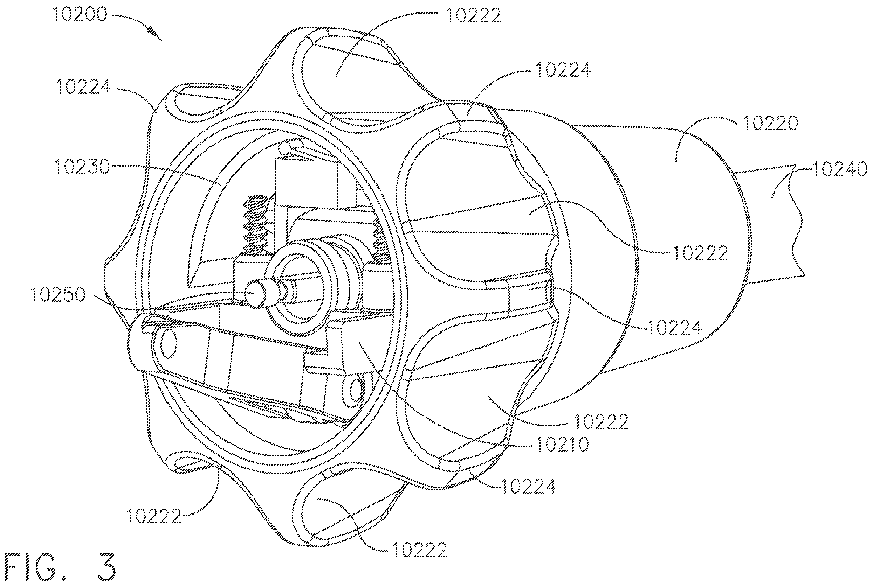

A surgical instrument 10000 is illustrated in FIG. 1. The surgical instrument 10000 comprises a handle 10100, a shaft 10200 extending from the handle 10100, and an end effector 10400. The end effector 10400 comprises a first jaw 10410 configured to receive a staple cartridge and a second jaw 10420 movable relative to the first jaw 10410. The second jaw 10420 comprises an anvil including staple forming pockets defined therein. The surgical instrument 10000 further comprises a closure actuator 10140 configured to drive a closure system of the surgical instrument 10000 and move the second jaw 10420 between an unclamped position and a clamped position. Referring to FIG. 3, the closure actuator 10140 is operably coupled with a closure tube 10240 that is advanced distally when the closure actuator 10140 is closed. In such instances, the closure tube 10240 contacts the second jaw and cams and/or pushes the second jaw 10420 downwardly into its clamped position. The second jaw 10420 is pivotably coupled to the first jaw about a pivot axis. That said, in alternative embodiments, the second jaw can translate and rotate as it is being moved into its clamped position. Moreover, in various alternative embodiments, a surgical instrument comprises a staple cartridge jaw is movable between an unclamped position and a clamped position relative to an anvil jaw. In any event, the handle 10100 comprises a lock configured to releasably hold the closure actuator 10140 in its clamped position. The handle 10100 further comprises release actuators 10180a, 10180b which, when either one is actuated, unlock the closure actuator 10140 such that the end effector can be re-opened. In various alternative embodiments, the handle 10100 comprises an electric motor configured to move the closure tube 10240 proximally and/or distally when actuated by the clinician.

The end effector 10400 is attached to the shaft 10200 about an articulation joint 10500 and is rotatable within a plane about an articulation axis. The shaft 10200 defines a longitudinal axis and the end effector 10400 is articulatable between a position in which the end effector 10400 is aligned with the longitudinal axis and positions in which the end effector 10400 extends at a transverse angle relative to the longitudinal axis. The handle 10100 comprises an electric motor and a control system configured to control the operation of the electric motor. The electric motor comprises a brushless DC motor; however, the electric motor can comprise any suitable motor, such as a brushed DC motor, for example. The entire disclosure of U.S. Patent No. 10,149,683 is relevant. The entire disclosure of U.S. Patent Application Publication No. 2018/0125481 is relevant. The handle 10100 further comprises a replaceable and/or rechargeable battery 10300 attachable to the handle housing which powers the surgical instrument 10000. The entire disclosure of U.S. Patent No. 8,632,525 is relevant. The electric motor is operably coupled with a firing drive 10250 of the surgical instrument 10000 and is configured to drive a firing member of the firing drive 10250 through a staple firing stroke. The electric motor comprises a rotatable output including a gear engaged with a translatable rack of the firing drive 10250. The electric motor is operated in a first direction to drive the firing member through the staple firing stroke and a second, or opposite, direction to retract the firing member and/or reset the firing drive 10250. The surgical instrument 10000 further comprises an actuator 10150 in communication with the motor control system which, when actuated or rotated, signals to the motor control system to operate the electric motor in the first direction and begin the staple firing stroke. If the actuator 10150 is released, the motor control system stops the electric motor. When the actuator 10150 is re-actuated, the motor control system operates the electric motor in the first direction once again to continue the staple firing stroke. When the firing member reaches the end of the staple firing stroke, the control system stops the electric motor awaiting input from the clinician. When the clinician releases the actuator 10150 at such point, the control system reverses the operation of the electric motor to retract the firing member back into its unfired position. The handle 10100 further comprises a retraction actuator in communication with the motor control system that reverses the direction of the electric motor to retract the firing drive when actuated by the clinician. When the retraction actuator is depressed, the staple firing stroke is terminated regardless of whether the firing member had reached the end of the staple firing stroke.

The electric motor of the surgical instrument 10000 is also used to selectively drive an articulation drive system to articulate the end effector 10400. More specifically, the articulation drive system comprises an articulation driver that is selectively engageable with the firing drive and, when the articulation driver is engaged with the firing drive, the articulation driver is movable proximally and distally by the operation of the electric motor to articulate the end effector 10400. When the electric motor is operated in its first direction, in such instances, the end effector 10400 is articulated in a first direction to push the articulation driver distally. Similarly, the end effector 10400 is articulated in a second direction when the electric motor is operated in its second direction to pull the articulation driver proximally. When the articulation driver is not engaged with the firing drive, the operation of the electric motor does not articulate the end effector 10400. Instead, in such instances, the electric motor only moves the firing drive. That said, it should be appreciated that the movement of the firing drive to articulate the end effector 10400 does not cause the staple firing stroke to be performed. The range of motion needed to articulate the end effector 10400 is small, as compared to the range of motion of the staple firing stroke, and occurs proximal to the beginning of the staple firing stroke such that the staples are not ejected and the tissue is not cut while the end effector 10400 is being articulated. The surgical instrument 10000 further comprises an articulation lock which unlocks when the articulation driver is moved longitudinally by the firing drive and then locks the end effector 10400 in position when the articulation driver is not being driven by the firing drive. The entire disclosure of U.S. Patent No. 9,629,629 is relevant. The above being said, a surgical instrument can comprise a separate articulation motor in addition to the firing motor for driving the articulation drive system.

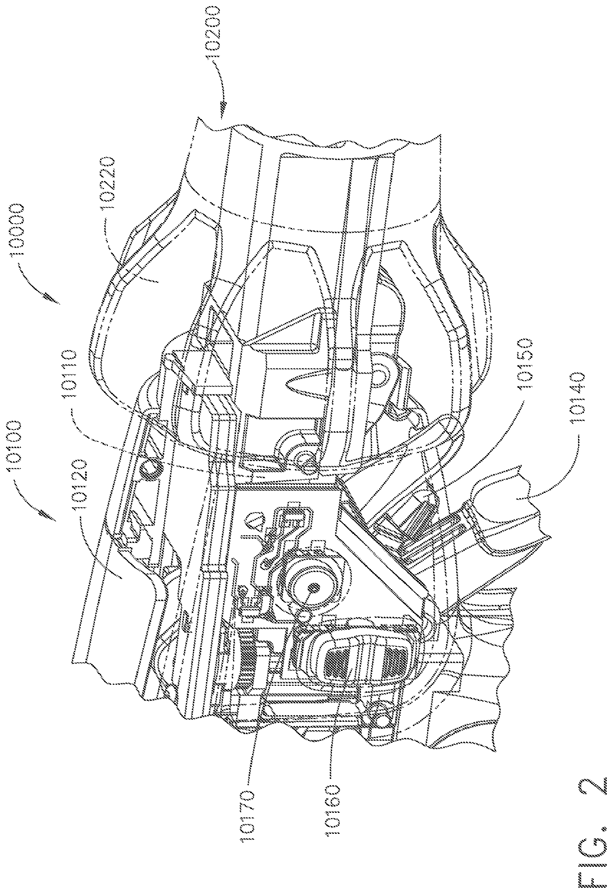

Further to the above, referring to FIG. 2, the handle 10100 comprises a frame 10110, a housing 10120, and an articulation actuator 10160. The articulation actuator 10160 comprises a rocker switch, for example, which is oriented vertically on the housing 10120 and is in communication with the motor control system. The rocker switch is rotatable upwardly and downwardly about an axis to articulate the end effector 10400. The upper portion of the articulation actuator 10160 is pushed by the clinician to articulate the end effector 10400 to the left and the lower portion of the articulation actuator 10160 is pushed to articulate the end effector 10400 to the right. Such an arrangement provides an intuitive interface for the clinician; however, any suitable arrangement could be used. The handle 10100 further comprises a home actuator 10170 in communication with the motor control system. When the home actuator 10170 is actuated by the clinician, the motor control system operates the electric motor to re-center the end effector 10400 along the longitudinal axis of the shaft 10200 of the surgical instrument 10000. To this end, the control system is configured to track the position of the end effector such that, when the home actuator 10170 is actuated, the control system operates the electric motor in the correct direction to articulate the end effector 10400 in the correct direction and the correct amount. In various instances, the surgical instrument 10000 comprises a linear encoder configured to track the position of the articulation driver, for example, such that, when the home actuator 10170 is actuated, the control system can properly center the end effector 10400.

Further to the above, the shaft 10200 is rotatable relative to the handle 10100. The shaft 10200 comprises a frame 10210 attached to the frame 10110 of the handle 10100. In embodiments where the shaft 10200 is readily removable from the handle 10100, the shaft frame 10210 can detach from the handle frame 10110. In embodiments where the shaft 10200 is not removable from the handle 10100, the shaft frame 10210 and the handle frame 10110 can be integrally formed. In any event, the shaft 10200 comprises a nozzle, or grip, 10220 fixedly mounted to the closure tube 10240 of the shaft 10200. The grip 10220 comprises finger grooves 10222 defined therein and ridges 10224 extending between the finger grooves 10222 that provide walls against which a clinician can push their finger and assist the clinician in rotating the shaft 10200 about its longitudinal axis.

Notably, further to the above, the end effector 10400 rotates with the shaft 10200 when the shaft 10200 is rotated about its longitudinal axis. Thus, the end effector 10400 rotates clockwise when the shaft 10200 is rotated clockwise by the clinician and counter-clockwise when the shaft 10200 is rotated counter-clockwise by the clinician. In various alternative embodiments, the surgical instrument 10000 comprises an electric motor configured to rotate the shaft 10200 about its longitudinal axis. In either event, the shaft 10200 is rotatable from a top-dead-center (TDC) position in which the anvil 10420 is positioned directly above the staple cartridge jaw 10410 to any other suitable position within a full 360 degree range of positions. For instance, the shaft 10200 is rotatable into a right 90 degree position in which the anvil 10420 is facing to the right of the handle 10100 or a left 90 degree position in which the anvil 10420 is facing to the left of the handle 10100. The shaft 10200 is also rotatable into a bottom-dead-center (BDC) position in which the staple cartridge jaw 10410 is positioned directly above the anvil 10420.

As described above, the end effector 10400 is both articulatable about the articulation joint 10500 and rotatable with the shaft 10200. When the end effector 10400 is rotated in a plane when the end effector 10400 is in its TDC position, as mentioned above, the articulation control 10160 is intuitive to the user - push up to articulate left and push down to articulate right. This arrangement is also intuitive even after the shaft 10200 - and end effector 10400 - have been rotated 90 degrees to the right or to the left. However, when the shaft 10200 and end effector 10400 have been rotated past 90 degrees in either direction, the articulation control 10160 can become counter-intuitive to the clinician. In fact, the articulation control 10160 can seem backwards. With this in mind, the control system of the surgical instrument 10000 is configured to flip the manner in which the surgical instrument responds to the articulation control 10160 when the shaft 10200 and end effector 10400 have been rotated past 90 degrees in either direction. In such instances, the controls become: push up to articulate right and push down to articulate left. To this end, as described in greater detail below, the surgical instrument 10000 is configured to detect the orientation of the shaft 10200 relative to the handle 10100, i.e., it is configured to detect whether the end effector 10400 is at least partially upside down with respect to the handle 10100 and then enter an alternative operational control mode in which the responsiveness of the surgical instrument 10000 to the articulation control 10160 has been reversed. Such an arrangement can make the surgical instrument 10000 easier to use in various instances.

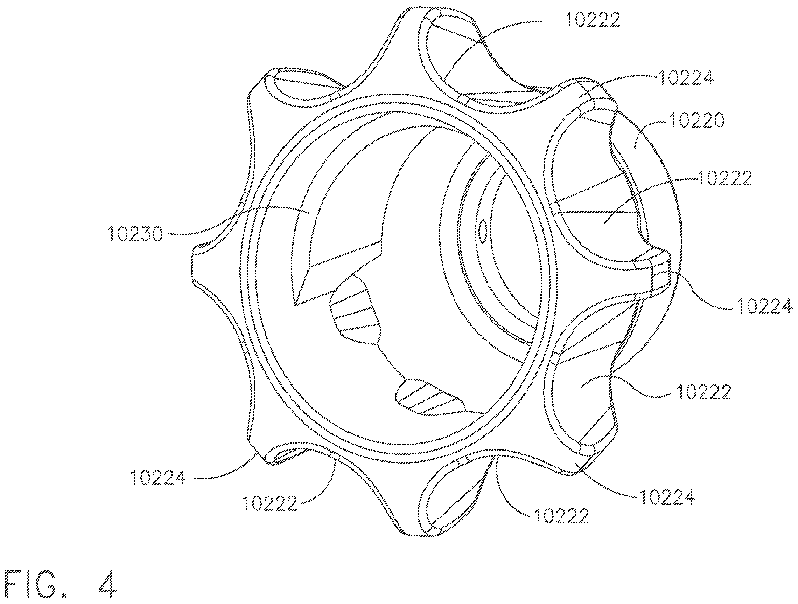

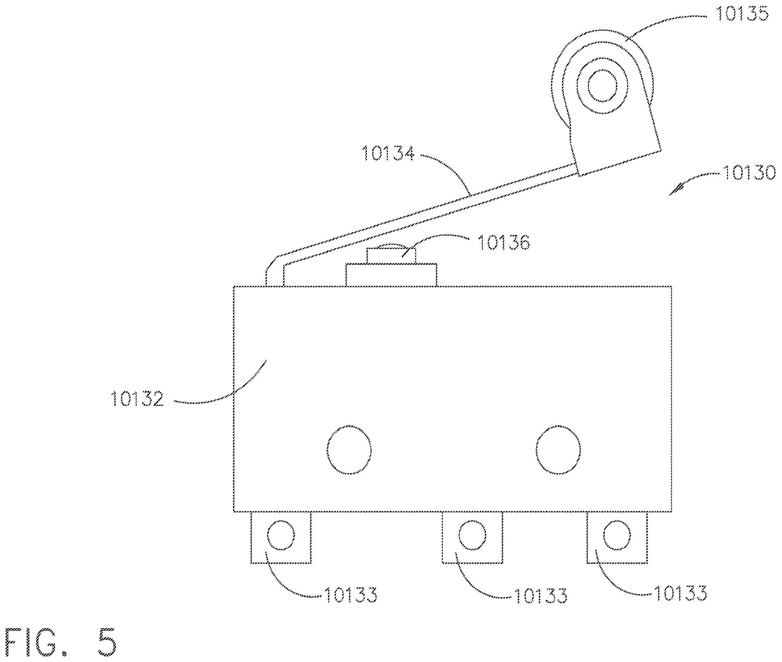

Referring to FIGS. 2-5, the surgical instrument 10000 comprises a switch 10130 mounted to the handle 10100 in communication with the control system which is configured to detect the rotation of the shaft 10200 relative to the handle 10100. The switch 10130 comprises a switch body 10132 fixedly mounted to the handle frame 10110 and three electrical contacts 10133 which are part of a switch circuit in communication with the control system. The switch 10000 further comprises a switch arm 10134 rotatably connected to the switch body 10132 and an electrical contact 10136 positioned on the switch body 10132. The switch arm 10134 is comprised of an electrically-conductive material, such as brass, for example, and closes the switch circuit when the switch arm 10134 comes into contact with the electrical contact 10136. The switch arm 10134 is rotated between an open position (FIG. 5) and a closed position when the shaft 10200 is rotated past the left or right 90 degree positions. More specifically, the grip, or nozzle, 10220 comprises a cam 10230 defined thereon which pushes the switch arm 10134 into its closed position when the shaft 10200 and the end effector 10400 is at least partially upside down. When the shaft 10200 is rotated upwardly past the 90 degree positions, the cam 10230 permits the switch arm 10134 to resiliently move back into its open position and open the switch circuit. The switch arm 10134 comprises a roller 10135 mounted thereto to facilitate relative rotation between the switch arm 10134 and the grip 10220.

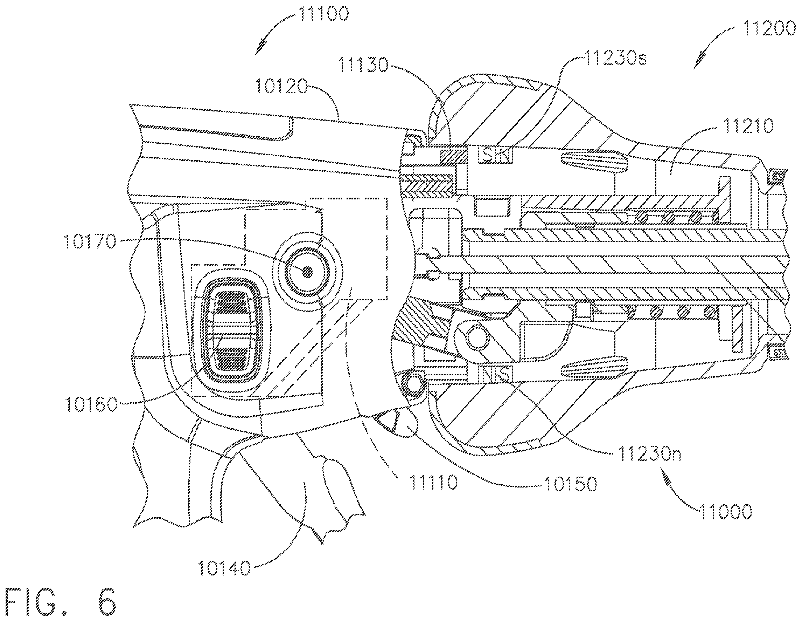

A surgical instrument 11000 is illustrated in FIG. 6. The surgical instrument 11000 is similar to the surgical instrument 10000 in many respects. The surgical instrument 11000 comprises a handle 11100 and a shaft 11200 extending from the handle 11100. The handle 11100 comprises a frame 11110 and the shaft 11200 comprises a frame 11210 attached to the handle frame 11110. The shaft 11200 comprises a grip, or nozzle, 11220, a first magnetic element 11230s positioned on one side of the grip 11220, and a second magnetic element 11230n positioned on the opposite side of the grip 11220. Stated another way, the first magnetic element 11230s and the second magnetic element 11230n are mounted 180 degrees apart. The handle 11100 further comprises a control system including at least one sensor 11130, such as a Hall Effect sensor, for example, mounted to the handle frame 11110 configured to sense the position of the magnetic elements 11230s and 11230n and, with this information, determine the orientation of the shaft 11200 relative to the handle 11100. Notably, the first magnetic element 11230s comprises a permanent magnet with a south pole facing toward the handle 11100 and a north pole facing away from the handle 11100 and the second magnetic element 11230n comprises a permanent magnet with a north pole facing toward the handle 11100 and a south pole facing away from the handle 11100. The magnetic elements 11230s and 11230n disturb the magnetic field emitted by the Hall Effect sensor and, when the shaft 11200 is at least partially upside down, the disturbance associated with such an orientation of the shaft 11200 is detected by the control system of the surgical instrument 11000 via a sensing circuit including the sensor 11130. In such instances, similar to the above, the control system enters into its second operating mode which flips the responsiveness of the surgical instrument 11000 to the articulation control 10160, as described above.

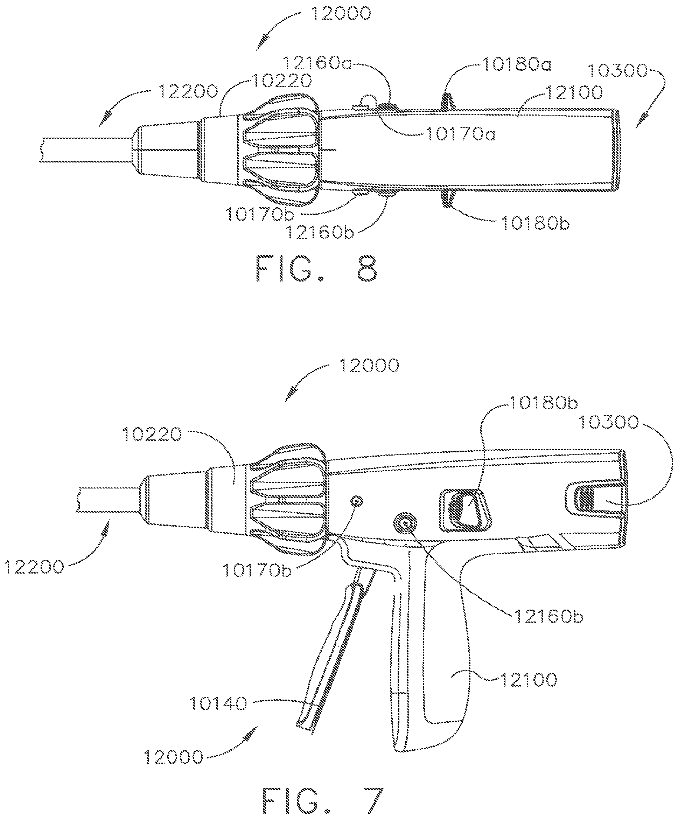

A surgical instrument 12000 is illustrated in FIGS. 7 and 8. The surgical instrument 12000 is similar to the surgical instrument 10000 in many respects. The surgical instrument 12000 comprises a handle 12100 and a shaft 12200 extending from the handle 12100. The handle 12100 comprises a housing, a first articulation control 12160a positioned on a first side of the handle housing, and a second articulation control 12160b positioned on a second, or opposite, side of the handle housing. The first articulation control 12160a is in communication with the control system of the surgical instrument 12000 via a first control circuit and the second articulation control 12160b is in communication with the control system via a second control circuit. The control system is configured to operate the electric motor of the staple firing drive in a first direction to articulate the end effector of the shaft 12200 in a first direction when the first articulation control 12160a is actuated and a second, or opposite, direction to articulate the end effector in a second, or opposite, direction with the second articulate control 12160b is actuated. The handle 12100 further comprises a centering, or home, actuator 10170a positioned on the first side of the handle 12100 and a second centering, or home, actuator 10170b on the second side of the handle 12100. Similar to the above, the actuators 10170a and 10170b are in communication with the control system which is configured such that the actuation of either centering actuator 10170a or 10170b causes the control system to operate the electric motor to re-center the end effector.

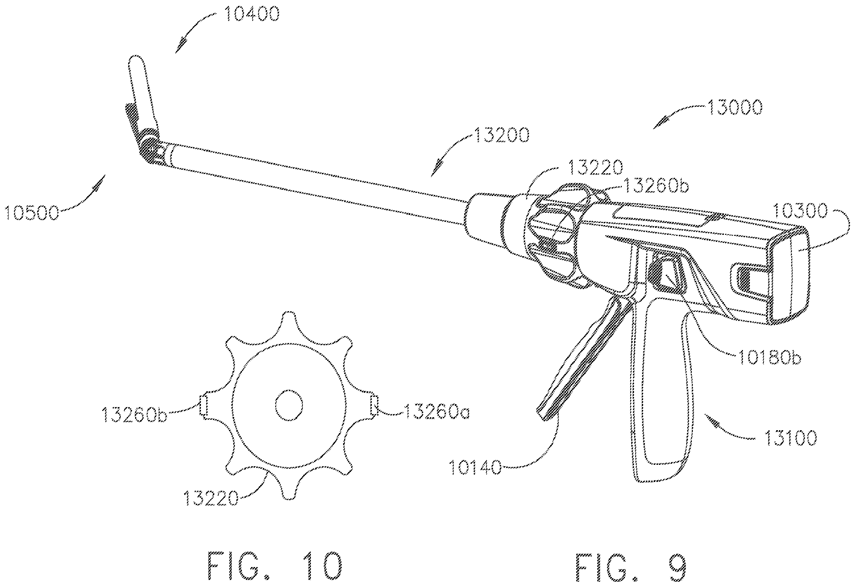

A surgical instrument 13000 is illustrated in FIGS. 9 and 10. The surgical instrument 13000 is similar to the surgical instrument 10000 in many respects. The surgical instrument 13000 comprises a handle 13100 and a shaft 13200 extending from the handle 13100. The shaft 13200 comprises a housing, a first articulation control 13260a positioned on a first side of the shaft housing, and a second articulation control 13260b positioned on a second, or opposite, side of the shaft housing. The first articulation control 13260a is in communication with the control system of the surgical instrument 13000 via a first control circuit and the second articulation control 13260b is in communication with the control system via a second control circuit. The control system is configured to operate the electric motor of the staple firing drive in a first direction to articulate the end effector 10400 of the shaft 13200 in a first direction when the first articulation control 13260a is actuated and a second, or opposite, direction to articulate the end effector 10400 in a second, or opposite, direction when the second articulation control 13260b is actuated. Stated another way, the end effector 10400 articulates in the direction of the articulation control that is actuated. The first articulation control 13260a is positioned on a first finger ridge defined on a grip, or nozzle, 13220 of the shaft 13200 and the second articulation control 13260b is positioned on a second finger ridge defined on the grip 13220. Notably, the articulation controls 13260a and 13260b are positioned 180 degrees apart. Alternatively, the articulation controls 13260a and 13260b can be positioned in the finger grooves defined in the grip 13220, although any suitable arrangement could be used. This arrangement provides an advantage of having the articulation controls in a position which is readily accessible by the hand of the clinician during use and, as a result, they are usable in an intuitive manner as the relative arrangement of the articulation controls 13260a and 13260b and the articulation directions are fixed.

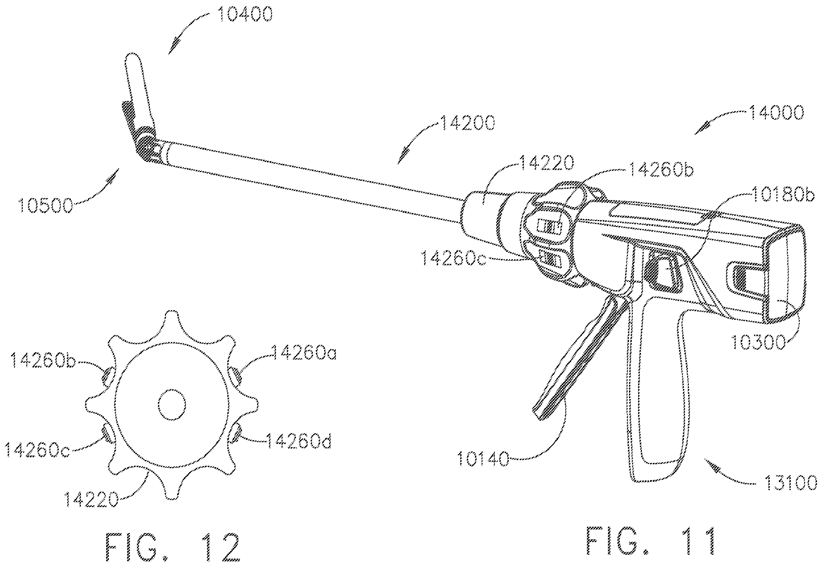

A surgical instrument 14000 is illustrated in FIGS. 11 and 12. The surgical instrument 14000 is similar to the surgical instrument 13000 in many respects. The surgical instrument 14000 comprises a handle 13100 and a shaft 14200 extending from the handle 13100. The shaft 14200 comprises a housing, a first articulation control 14260a positioned on a first side of the shaft housing, and a second articulation control 14260b positioned on a second side of the shaft housing. The first articulation control 14260a is in communication with the control system of the surgical instrument 14000 via a first control circuit and the second articulation control 14260b is in communication with the control system via a second control circuit. The control system is configured to operate the electric motor of the staple firing drive in a first direction to articulate the end effector 10400 of the shaft 14200 in a first direction when the first articulation control 14260a is actuated and a second, or opposite, direction to articulate the end effector 10400 in a second, or opposite, direction when the second articulation control 14260b is actuated. The first articulation control 14260a is positioned in a first finger groove defined in a grip, or nozzle, 14220 of the shaft 14200 and the second articulation control 14260b is positioned in a second finger groove defined in the grip 14220, although any suitable arrangement could be used.

In addition to the above, the shaft 14200 further comprises a third articulation control 14260c positioned on the second side of the shaft housing and a fourth articulation control 14260d positioned on the first side of the shaft housing. The third articulation control 14260c is in communication with the control system of the surgical instrument 14000 via a third control circuit and the fourth articulation control 14260b is in communication with the control system via a fourth control circuit. The control system is configured to operate the electric motor of the staple firing drive in the second direction to articulate the end effector of the shaft 14200 in the second direction when the third articulation control 14260c is actuated and the first direction to articulate the end effector in the first direction when the fourth articulation control 14260d is actuated. The third articulation control 14260c is positioned in a third finger groove defined in the grip 14220 of the shaft 14200 and the fourth articulation control 14260d is positioned in a fourth finger groove defined in the grip 14220, although any suitable arrangement could be used.

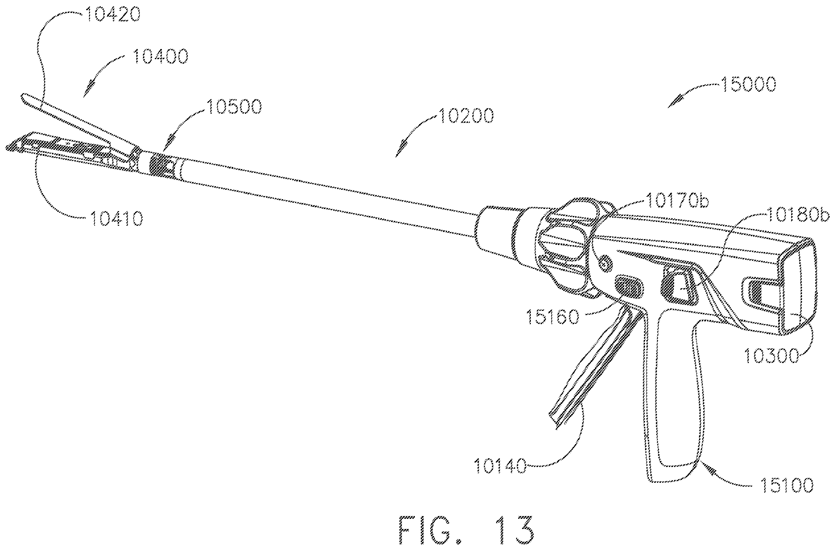

A surgical instrument 15000 is illustrated in FIG. 13. The surgical instrument 15000 is similar to the surgical instrument 10000 in many respects. The surgical instrument 15000 comprises a handle 15100 and a shaft 10200 extending from the handle 15100. The handle 15100 comprises an articulation actuator 15160 in communication with the control system of the surgical instrument 15000. As opposed to the articulation actuator 10160 which is arranged vertically, the articulation actuator 15160 is arranged horizontally. The articulation actuator 15160 comprises a rotatable element which is rotatable within a plane which is parallel to, or at least substantially parallel to, the longitudinal axis of the shaft 10200. The rotatable element is rotatable distally to articulate the end effector 10400 to the right of the handle 15100 and proximally to articulate the end effector 10400 to the left of the handle 15100. This is true regardless of whether the end effector 10400 is rotated upwardly or downwardly owing to the control responsiveness flipping when the end effector 10400 is rotated past 90 degrees from its TDC position in either direction. That said, the controls of the articulation actuator 15160 can be reversed as outlined above. The articulation actuator 15160 comprises a distal contact which is part of a first articulation control circuit and a proximal contact which is part of a second articulation control circuit. The rotatable element engages the distal contact and closes the first articulation control circuit when the rotatable element is in its distal position. The rotatable element is not in contact with the proximal contact when the rotatable element is in its distal position and, as such, the second articulation control circuit is open. Similarly, the rotatable element engages the proximal contact and closes the second articulation control circuit when the rotatable element is in its proximal position. Correspondingly, the rotatable element is not in contact with the distal contact when the rotatable element is in its proximal position and, as such, the first articulation control circuit is open.

Further to the above, the articulation actuator 15160 comprises a detent in the middle of the range of motion of the rotatable element. The detent is configured to resist the motion of the rotatable element as the rotatable element moves from one side of the articulation actuator 15160 to the other. Such resistance to the motion of the rotatable element can signal to the clinician that they will articulate the end effector 10400 in the opposite direction once they move the rotatable element past that point. Moreover, such a detent provides a place to park the rotatable element such that the end effector 10400 is not being articulated in either direction. The rotatable element comprises a ridge alignable with its center, or parked, position which is pushable and pullable by the clinician to move the rotatable element. Such a ridge provides the clinician with a tactile sensation of the direction in which the rotatable element is rotated and, thus, a sense of the direction in which the end effector 10400 is being articulated.

The above being said, various embodiments are envisioned in which the flipping of the control responsiveness of a surgical instrument can be defeated. In at least one instance, the handle of the surgical instrument comprises an actuator in communication with the control system that, when actuated, causes the control system to not enter into its second, or flipped, operational mode. In at least one such instance, the handle further comprises an indicator, such as a light emitting diode (LED), for example, that is illuminated to indicate the status of the surgical instrument, i.e., whether or not the articulation controls will flip when the end effector is rotated past 90 degrees from its TDC position. In certain instances, the surgical instrument comprises an input screen in communication with a microprocessor of the control system which can receive an input to prevent the control system from entering into its second, or flipped, operational mode. In addition to or in lieu of the above, the flip point in which the surgical instrument enters into its second operation mode can be adjusted. In at least one such embodiment, the clinician can modify the flip point to 85 degrees, for example, in either direction from the TDC position of the end effector. Any suitable number, such as 80 degrees, 95 degrees, or 100 degrees, for example, could be used to suit the preference of the clinician. In at least one embodiment, the surgical instrument comprises an input screen in communication with the microprocessor of the control system which is configured to receive an input from the clinician to adjust the articulation control flip point.

During use, it is desirable for the articulation controls not to flip unexpectedly while the clinician is using the articulation controls. When the clinician starts articulating the end effector, the control system maintains the articulation control mode until the clinician releases the articulation control even if the end effector and shaft are rotated past a flip point during the articulation. Once the articulation has stopped, the control system can re-orient the articulation controls, or switch to the flipped articulation control mode if the end effector and shaft are still in an upside-down position. In certain embodiments, the control system does not immediately flip the articulation controls. Instead, the control system comprises a timer circuit and/or the microprocessor of the control system is programmed to wait a certain amount of time before flipping the controls. In at least one instance, the control system waits 5 seconds, for example, from the last time that the articulation controls were used before flipping the articulation controls. Alternatively, the control system can wait 2 seconds or 10 seconds, for example. Such an arrangement can help prevent confusion with the user of the surgical instrument. In various embodiments, the surgical instrument comprises a haptic feedback generator in communication with the control system which is activated by the control system when the articulation controls are flipped. Motor noise, light, sound, and/or a vibratory feedback, for example, can be used. In some embodiments, the shaft and/or handle comprises a mechanical switch which audibly clicks when the shaft is rotated past its flip point in either direction.

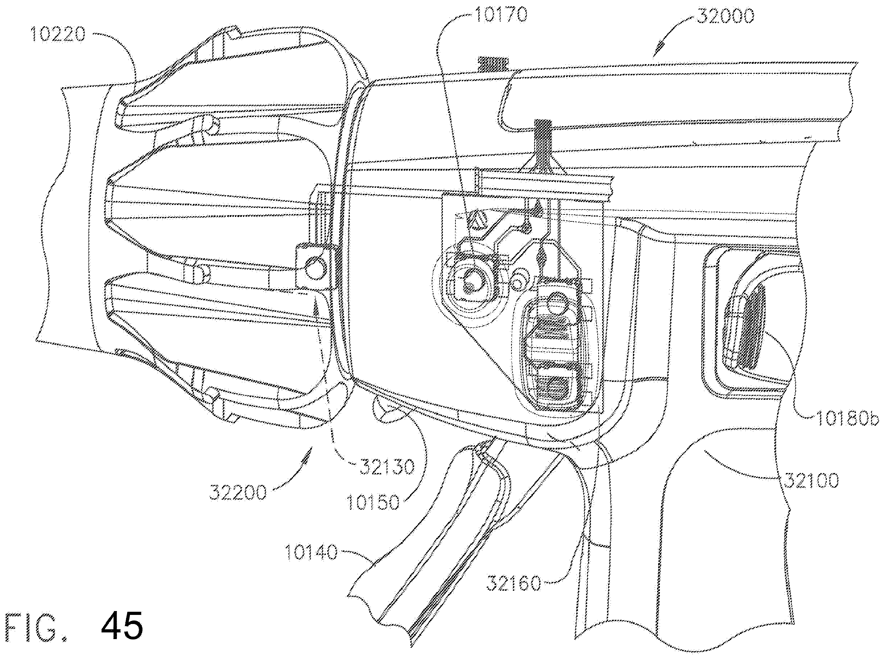

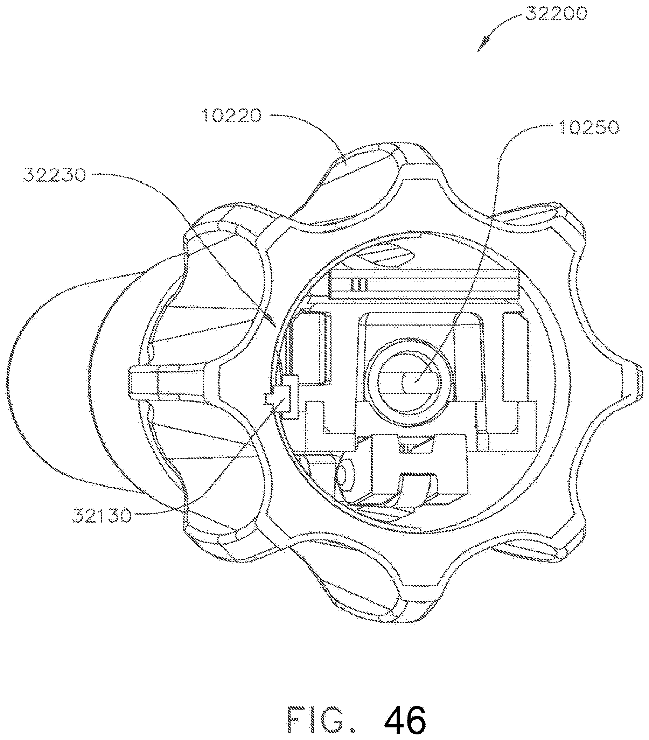

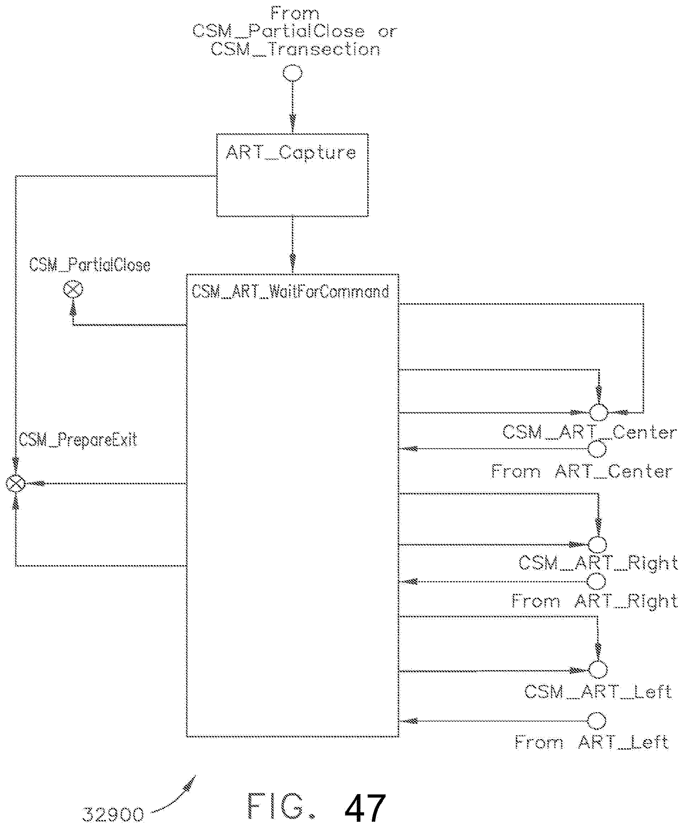

A surgical instrument 32000 is illustrated in FIGS. 45 and 46, the surgical instrument 32000 comprises a handle 32100 and a shaft 32200. The handle 32100 comprises an articulation control 32160 and an articulation flip switch 32130 in communication with the control system of the surgical instrument 32000. The articulation flip switch 32130 is mounted to a control board, such as a printed control board (PCB), for example, which comprises the hardware and software for the control system of the surgical instrument 32000. When the shaft 32200 is rotated past its 90 degree left or right position, the shaft 32200 contacts the articulation flip switch 32130 which is detected by the control system. At this point, the control system follows an algorithm for deciding when, or if, to the flip the articulation controls. An algorithm 32900 is illustrated in FIG. 47 which can control this, although any suitable algorithm could be used. Similar to the above, the shaft 32200 comprises a cam 32230 configured to contact the articulation flip switch 32130. As a result of the above, the articulation flip switch 32130 is open or "off" for 180 degrees of the rotation of the shaft 32200 and closed or "on" for the other 180 degrees of the rotation of the shaft 32200. The cam 32230 is molded into the shroud of the shaft 32200, but could comprise any suitable arrangement. The above being said, the throw of the cam 32230 is designed such that any lateral float or eccentricity in the rotation of the shaft 32200, or cam 32230, does not accidentally close or open the articulation flip switch 32130. To this end, the shaft 32200 comprises a fixed bearing for controlling the rotation of the shaft 32200 and the cam 32230. Notably, the articulation flip switch 32130 is sealed to prevent fluid ingress.

As discussed above, the articulation flip switch 32130 is directly mounted to a control board and is contacted by the shaft assembly as the shaft assembly is rotated related to the handle about its longitudinal axis. When the articulation flip switch 32130 is contacted by the shaft assembly, a force is transmitted to the articulation flip switch 32130 and the control board. Absent more, such a force can tend to deflect the control board and the articulation flip switch 32130 out of position without switching the state of the articulation flip switch 32130. In various embodiments, the control board is mounted to a housing of the handle via one or more screws such that the control board does not translate relative to the handle housing. In at least one embodiment, the screws comprise self-tapping screws that threadably engage the handle housing and/or the control board. In at least one embodiment, the control board is captured between two portions of the handle housing in a press-fit manner such that the control board is tightly held therebetween. In various embodiments, the articulation flip switch 32130 is attached to the control board using one or more fasteners, such as screws, for example, such that the articulation flip switch 32130 does not move relative to the control board. In at least one embodiment, the articulation flip switch 32130 is bonded to the control board using one or more adhesives. In at least one embodiment, the articulation flip switch 32130 is soldered to the control board using any suitable solder, such as eutectic tin-lead solder, for example. In at least one embodiment, the articulation flip switch 32130 comprises a body and electrical terminals that are both soldered to the control board. In at least one embodiment, the electrical terminals comprise pins extending through holes defined in the control board that are soldered into electrical communication with one or more trace circuits defined in and/or on the control board. In at least one embodiment, the electrical terminals comprise surface mount terminals that are soldered into electrical communication with one or more trace circuits defined in and/or on the control board.

In various instances, a surgical instrument comprises an input configured to permit a clinician to select whether the articulation controls operate in their ordinary articulation control mode or their flipped articulation control mode. In at least one instance, the handle of the surgical instrument comprises an input switch in communication with the control system of the surgical instrument. When the input switch is open, for instance, the algorithm controls the orientation of the articulation controls according to a predetermined set of criteria. When the input switch is closed by the clinician, the algorithm does not use the predetermined set of criteria to control the orientation of the articulation controls. Instead, the algorithm uses the orientation of the articulation controls selected by the clinician. In at least one instance, the handle comprises three input switches in communication with the control system - a first switch which instructs the control system to use the "anvil up" articulation controls, a second switch which instructs the control system to use the "anvil down" articulation controls, and a third switch which instructs the control system to use the automatic controls. In some embodiments, the surgical instrument does not have the automatic flip controls described herein and can just comprise the first and second switch inputs. Such an arrangement can greatly reduce the cost and/or complexity of a surgical instrument.

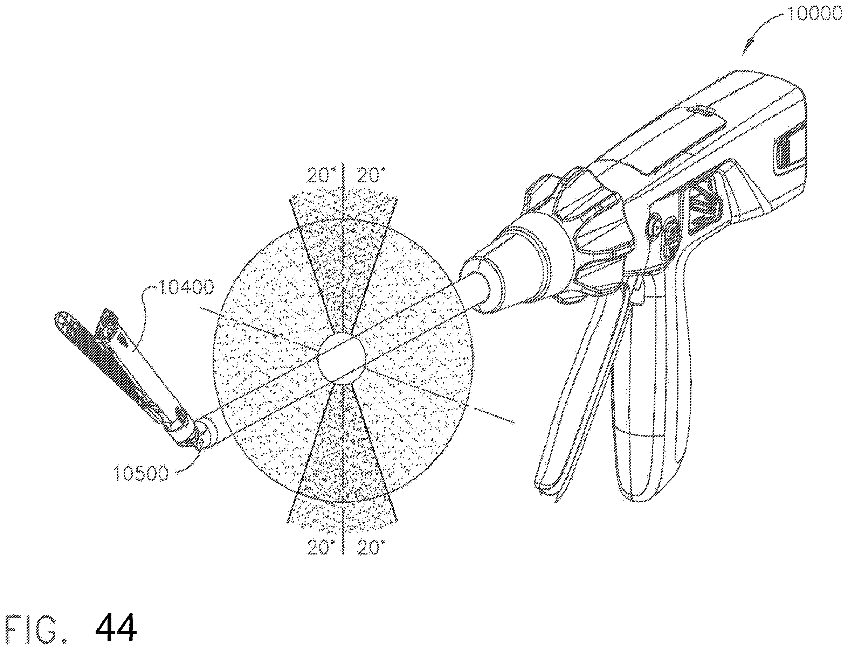

In various instances, further to the above, the flip point can be a specific point in the rotation of the shaft 10200. In certain instances, referring to FIG. 44, a grey zone can exist around the flip point. For instance, the grey zone can include 20 degrees to either side of the flip point, for example. While the shaft 10200 is in the grey zone, the algorithm of the control system is configured to not flip the articulation controls even though the shaft 10200 may have been rotated past the flip point. Such an arrangement allows the shaft 10200 to be rotated back and forth within the grey zone without repeatedly flipping the articulation controls. Once the shaft 10200 is rotated out of the grey zone, however, the control system algorithm flips the articulation controls - subject to any other criteria needed for flipping the articulation controls. In various instances, there is an interface between the range of "anvil up" orientations and the range of "anvil down" orientations. For a shaft that is rotatable 360 degrees, there are two such interfaces - 180 degrees apart from another. Each of these interfaces is positioned within a transition range of orientations that extends into the range of "anvil up" orientations and the range of "anvil down" orientations. When the shaft 10200 is rotated from an "anvil up" orientation into a transition range, the control system does not flip the articulation controls - but further rotating the shaft 10200 out of the transition range into an "anvil down" orientation will cause the articulation controls to flip. Similarly, the control system does not flip the articulation controls when the shaft 10200 is rotated from an "anvil down" orientation into a transition range, but further rotating the shaft 10200 out of the transition range in an "anvil up" orientation will cause the articulation controls to flip. In at least one instance, each transition zone includes 5 degrees of orientations from the "anvil up" range and 5 degrees of orientations from the "anvil down" range, for example. In other embodiments, each transition zone includes 10 degrees of orientations from the "anvil up" range and 10 degrees of orientations from the "anvil down" range, for example.

In various embodiments, further to the above, the up and down orientations of the shaft 10200 are measured with respect to the handle and/or a housing rotatably supporting the shaft. In such instances, a handle comprises a top and a bottom - regardless of its gravitational orientation - and the up orientations of the shaft 10200 are associated with the top of the handle while the down orientations of the shaft 10200 are associated with the bottom of the handle. In at least one such embodiment, the shaft 10200 comprises a gravity sensor, such as an accelerometer and/or a gyroscope, for example, and the handle comprises a gravity sensor. In such embodiments, the shaft gravity sensor and the handle gravity sensor are in communication with the control system which is configured to assess the relative orientation between the shaft and the handle using the data from the gravity sensors. In other embodiments, the up and down orientations of the shaft 10200 are measured with respect to gravity regardless of the gravitational orientation of the handle. In at least one such embodiment, the shaft 10200 comprises a gravity sensor in communication with the control system and the up orientations of the shaft 10200 are associated with vertically up positions while the down orientations of the shaft 10200 are associated with vertically down positions.

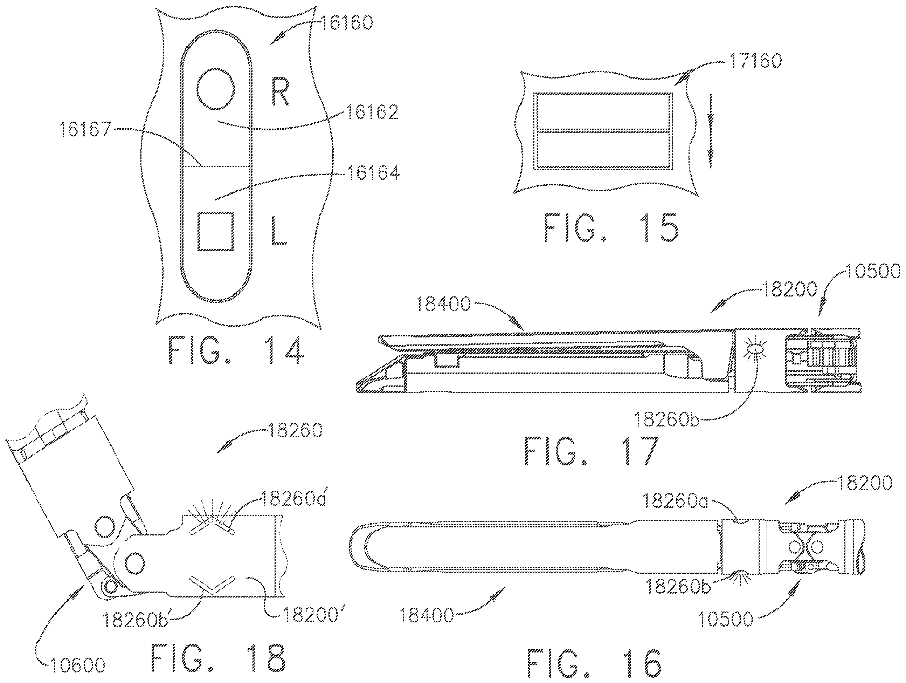

An articulation control 16160 is illustrated in FIG. 14. The articulation control 16160 comprises a first capacitive switch 16162 and a second capacitive switch 16164. The first capacitive switch 16162 and the second capacitive switch 16164 are positioned on opposite sides of an axis 16167. The first capacitive switch 16162 is part of a first articulation control circuit in communication with a control system of a surgical instrument and the second capacitive switch 16164 is part of a second articulation control circuit in communication with the control system. The capacitance of the first capacitive switch 16162 changes when a clinician places their finger on the first capacitive switch 16162 which is detected by the control system and, in response to this change, the control system articulates the end effector of the surgical instrument to the right. The capacitance of the second capacitive switch 16164 changes when a clinician places their finger on the second capacitive switch 16164 which is detected by the control system and, in response to this change, the control system articulates the end effector of the surgical instrument to the left. In various instances, the axis 16167 comprises a dead zone which, if touched by the clinician, does not detectably, or sufficiently, change the capacitance of the first capacitive switch 16162 or the second capacitive switch 16164.

A two-stage switch 17160 is illustrated in FIG. 15. When the switch 17160 is depressed into its first stage, a first articulation control circuit is closed. The first articulation control circuit is in communication with a control system of a surgical instrument. When the control system detects that the first articulation control circuit has been closed, the control system operates an articulation drive motor in a first direction to articulate the end effector of the surgical instrument in a first direction. When the switch 17160 is depressed into its second stage, a second articulation control circuit is closed. In various instances, the first stage comprises a first detent and the second stage comprises a second detent. In at least one such instance, the switch 17160 comprises a dual-detent switch that is depressable to two different depths, for example. In any event, the second articulation control circuit is in communication with the control system of the surgical instrument. When the control system detects that the second articulation control circuit has been closed, the control system operates an articulation drive motor in a second direction to articulate the end effector of the surgical instrument in a second direction. Further to the above, the second articulation control circuit is open when the first articulation control circuit is closed and, likewise, the first articulation control circuit is open when the second articulation control circuit is closed. The above being said, in alternative embodiments, the articulation control circuits can be opened when they are in their respective stages to operate the articulation motor.

Many clinicians, further to the above, prefer to look at the patient when performing an open surgery and/or at an endoscope monitor when performing a laparoscopic surgery. As such, the clinician does not usually look at the surgical instrument that they are holding and, instead, rely on the tactile feel and/or intuitive design of the surgical instrument to operate the surgical instrument. Stated another way, the clinician may not prefer to look down at the handle of the instrument they are holding to verify the direction that they are articulating the instrument. That being said, referring to FIGS. 16 and 17, a surgical instrument can comprise a shaft 18200 comprising indicator lights configured to indicate the direction in which an end effector, such as end effector 18400, for example, is being articulated. The articulation indicator lights are visible to the clinician while they are looking at the end effector 18400 of the surgical instrument - either directly or through an endoscope system monitor. In various instances, an endoscope system comprises an elongate flexible shaft including a camera, a light, and/or any other suitable optical device in communication with a control hub including a control system and/or a video monitor configured to display the output of the camera. In such instances, the end effector 18400 and the indicator lights are visible on the video monitor.

Further to the above, referring again to FIGS. 16 and 17, the shaft 18200 comprises a first indicator light 18260a positioned on the right side of the end effector 18400 in communication with the control system of the surgical instrument via a first electrical circuit. When the control system receives an input to articulate the end effector 18400 to the right, the control system operates the articulation drive motor in a direction which articulates the end effector 18400 to the right and, also, illuminates the first indicator light 18260a. When the control system no longer receives this input, the control system deactivates the articulation drive motor and the first indicator light 18260a. Similarly, the shaft 18200 comprises a second indicator light 18260b positioned on the left side of the end effector 18400 in communication with the control system of the surgical instrument via a second electrical circuit. When the control system receives an input to articulate the end effector 18400 to the left, the control system operates the articulation drive motor in a direction which articulates the end effector 18400 to the left and, also, illuminates the second indicator light 18260b. When the control system no longer receives this input, the control system deactivates the articulation drive motor and the second indicator light 18260b.

As discussed above, the first and second indicator lights 18260a and 18260b are positioned on the end effector 18400 in a position which is readily observable by the clinician when they are looking at the end effector 18400. The indicator lights 18260a and 18260b are positioned distally with respect to the articulation joint 10500; however, in alternative embodiments, the indicator lights 18260a and 18260b are positioned proximally to the articulation joint 10500. In various embodiments, a surgical instrument comprises more than one set of indicator lights. In at least one such embodiment, a first set of indicator lights 18260a, 18260b is positioned distally with respect to the articulation joint 10500 and a second set of indicator lights 18260a, 18260b is positioned proximally with respect to the articulation joint 10500. An alternative embodiment comprising indicator lights 18260a' and 18260b' on a shaft 18200' is illustrated in FIG. 18. The indicator light 18260a' comprises an LED in the shape of a right-facing arrow while the indicator light 18260b' comprises an LED in the shape of a left-facing arrow. The right-facing arrow 18260a' points to the right of the end effector - but not necessarily to the right of the surgical instrument handle and/or the clinician owing to the possible rotation of the shaft 18200'. Similarly, the left-facing arrow 18260b' points to the left of the end effector - but not necessarily to the left of the surgical instrument handle and/or the clinician owing to the possible rotation of the shaft 18200'. Stated another way, the arrows, when illuminated, point in the direction that the end effector is being articulated. Given that the arrows are observable with the end effector on an endoscope monitor, for example, the clinician will develop a sense for the direction that the end effector will move when an arrow is illuminated upon actuating the articulation actuator. If the clinician observes that the illuminated arrow is the opposite of what they expected when they actuate the articulation actuator, the clinician can quickly react and re-actuate the articulation actuator in the correct direction. In various alternative embodiments, the arrows 18260a' and 18260b' can change colors when they are actuated. For instance, the arrow 18260a' is illuminated red when the end effector is not articulated to the right, but is illuminated green when the end effector is articulated to the right. Likewise, the arrow 18260b' is illuminated red when the end effector is not articulated to the left, but is illuminated green when the end effector is articulated to the left.

In various embodiments, further to the above, the articulation indicator lights can be embedded in and/or positioned on the outer housing of the shaft. In certain embodiments, the indicator lights are positioned inside the shaft, but are viewable from outside the shaft through windows and/or openings defined in the shaft, for example.

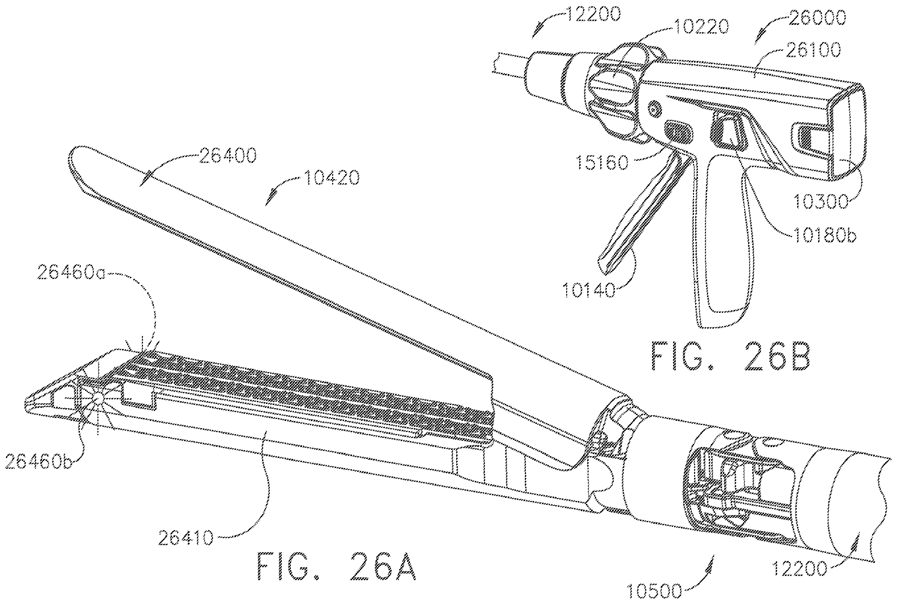

A surgical instrument 26000 is illustrated in FIGS. 26A and 26B. The surgical instrument 26000 comprises a handle 26100 and a shaft 12200 extending from the handle 26100. The shaft 12200 comprises an end effector 26400 including a staple cartridge jaw 26410 and an anvil jaw 10420. The end effector 26400 further comprises a first articulation indicator light 26460a positioned on a first side of the end effector 26400 and a second articulation indicator light 26460b positioned on a second side of the end effector 26400. Similar to the above, the control system of the surgical instrument 26000 illuminates the first articulation indicator light 26460a when the end effector 26400 is articulated in the first direction. In such instances, the control system does not illuminate the second articulation indicator light 26460b. Correspondingly, the control system of the surgical instrument 26000 illuminates the second articulation indicator light 26460b when the end effector 26400 is articulated in the second direction. In such instances, the control system does not illuminate the first articulation indicator light 26460a. The indicator lights 26460a and 26460b are mounted to and/or embedded in the frame of the staple cartridge jaw 26410. That said, the indicator lights 26460a and 26460b can be mounted to and/or embedded in the staple cartridge positioned in the staple cartridge jaw 26410. In such instances, the staple cartridge jaw 26410 comprises an electrical circuit in communication with the control system of the surgical instrument that is placed in communication with an electrical circuit in the staple cartridge when the staple cartridge is seated in the staple cartridge jaw 26410.

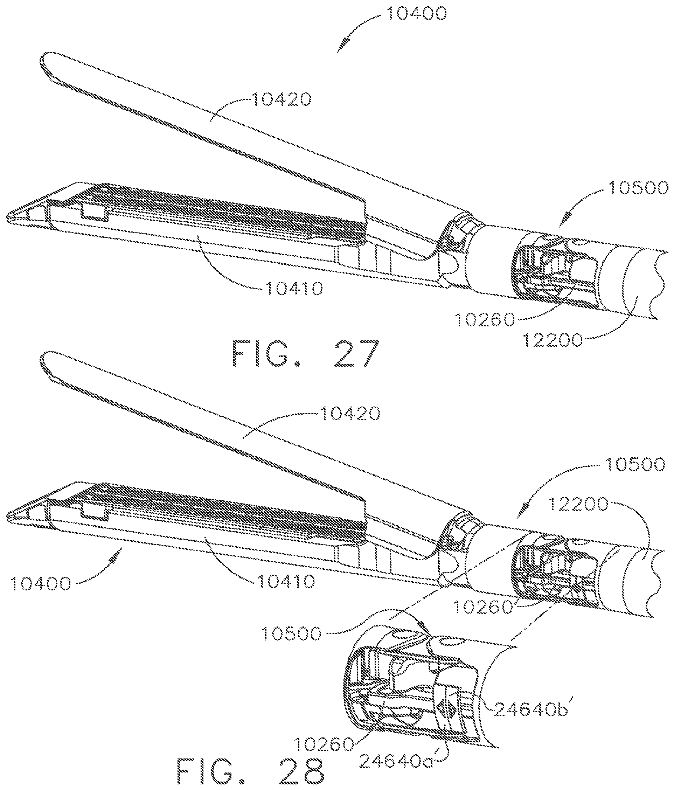

As discussed above, the articulation system of a surgical instrument can include an articulation driver which is movable proximally to articulate the end effector in a first direction and distally to articulate the end effector in a second direction. Referring to FIG. 27, a surgical instrument can comprise a handle 26100, a shaft 12200 extending from the handle 26100, and an end effector 10400 rotatably connected to the shaft 12200 about an articulation joint 10500. The shaft 12200 comprises an articulation driver 10260 comprising a proximal end operably coupled to an articulation drive system and a distal end coupled to the end effector 10400. To this end, the articulation driver 10260 extends distally past the articulation joint 10500 and, in this embodiment, is partially visible to a clinician holding the surgical instrument. The portion of the articulation driver 10260 visible to the clinician is also visible to the clinician through an endoscope monitor. In fact, a clinician may be able to observe the motion of the articulation driver 10260 through the endoscope monitor. The visible portion of the articulation driver 10260 comprises indicia, such as indicia 24640a' and 24640b', for example, thereon which correlates the movement of the articulation driver 10260 to the movement of the end effector 10400. In at least one instance, the indicia can comprise a first set of indicia which includes a distally-directed arrow 24640a' and a circular arrow indicating the direction that the end effector 10400 will be rotated if the articulation driver 10260 is moved distally. The indicia can also comprises a second set of indicia which includes a proximally-directed arrow 24640b' and a circular arrow in the opposite direction indicating the direction that the end effector 10400 will be rotated if the articulation driver 10260 is moved proximally. An alternative articulation driver 10260' is illustrated in FIG. 28 that comprises a laterally-extending portion which can be readily visible to the clinician. In such instances, the above-discussed indicia is positioned on the laterally-extending portion.

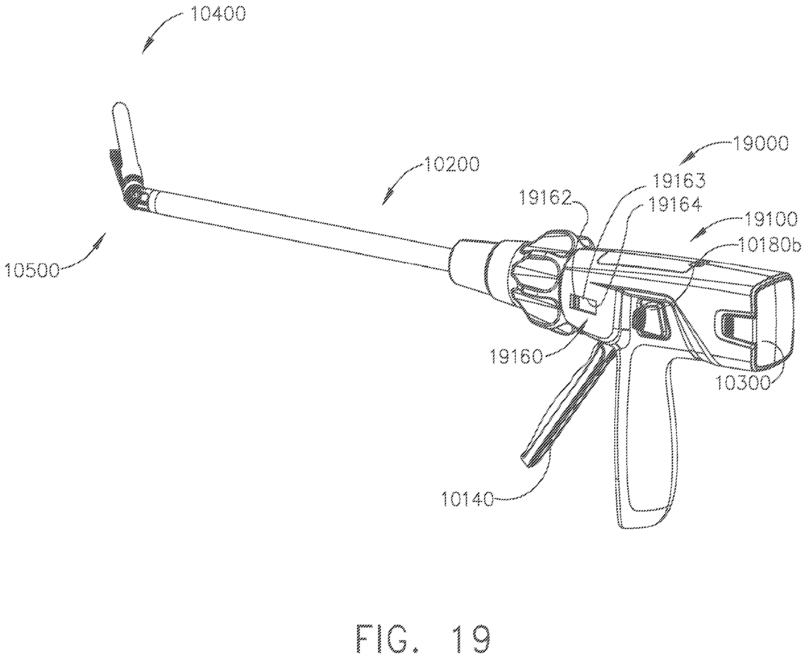

A surgical instrument 19000 is illustrated in FIG. 19. The surgical instrument 19000 is similar to the surgical instrument 15000 in many respects. The surgical instrument 19000 comprises a handle 19100 and a shaft 10200 extending from the handle 19100. The handle 19100 comprises an articulation actuator 19160 in communication with the control system of the surgical instrument 19000. As opposed to the articulation actuator 10160 which is arranged vertically, the articulation actuator 19160 is arranged horizontally. The articulation actuator 19160 comprises a slideable element 19162 which is slideable along an axis which is parallel to, or at least substantially parallel to, the longitudinal axis of the shaft 10200. In at least one instance, the axis of the articulation actuator 19160 is aligned with the longitudinal axis of the shaft 10200. The slideable element 19162 is positioned within a slot 19164 on the handle 19100 of the surgical instrument 19000. The slideable element 19162 is slideable distally to articulate the end effector 10400 to the right of the handle 19100 and proximally to articulate the end effector 10400 to the left of the handle 19100. This is true regardless of whether the end effector 10400 is rotated upwardly or downwardly owing to the control responsiveness flipping when the end effector 10400 is rotated past 90 degrees from its TDC position in either direction. That said, the controls of the articulation actuator 19160 can be reversed as outlined above.

The articulation actuator 19160 comprises a distal contact which is part of a first articulation control circuit and a proximal contact which is part of a second articulation control circuit. The slideable element 19162 engages the distal contact and closes the first articulation control circuit when the slideable element 19162 is in its distal position. The slideable element 19162 is not in contact with the proximal contact when the slideable element 19162 is in its distal position and, as such, the second articulation control circuit is open. Similarly, the slideable element 19162 engages the proximal contact and closes the second articulation control circuit when the slideable element 19162 is in its proximal position. Correspondingly, the slideable element 19162 is not in contact with the distal contact when the slideable element 19162 is in its proximal position and, as such, the first articulation control circuit is open. In any event, the articulation actuator 19160 comprises a detent 19163 in the middle of the range of motion of the slideable element 19162. The detent 19163 is configured to resist the motion of the slideable element 19162 as the slideable element 19162 moves from one side of the articulation actuator 19160 to the other. Such resistance to the motion of the slideable element 19162 can signal to the clinician that they will articulate the end effector 10400 in the opposite direction once they move the slideable element 19162 past that point. Moreover, such a detent 19163 provides a place to park the slideable element 19162 such that the end effector 10400 is not being articulated in either direction.

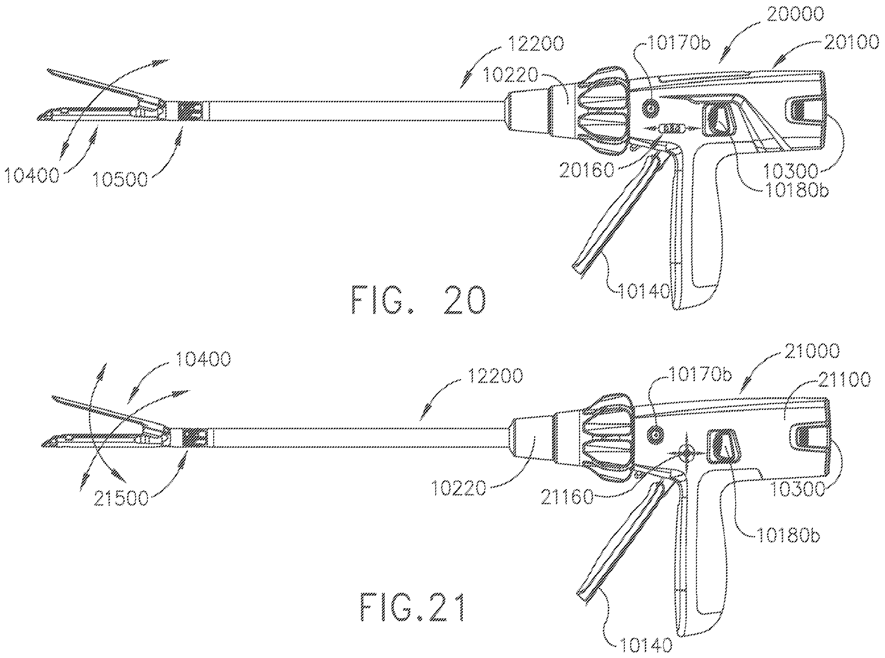

A surgical instrument 20000 is illustrated in FIG. 20. The surgical instrument 20000 is similar to the surgical instrument 10000 in many respects. The surgical instrument 20000 comprises a handle 20100 and a shaft 12200 extending from the handle 20100. The handle 20100 comprises an articulation actuator 20160 in communication with the control system of the surgical instrument 20000. The articulation actuator 20160 comprises a two-dimensional joystick movable within a plane which is aligned with, parallel to, or at least substantially parallel to, the longitudinal axis of the shaft 12200. The joystick is movable distally to articulate the end effector 10400 to the right of the handle 20100 and proximally to articulate the end effector 10400 to the left of the handle 20100. In at least one instance, the joystick comprises a handle having an inner end that is positioned in a sensor seat in communication with the control system of the surgical instrument 20000. The joystick is pivotable within the sensor seat by the clinician when the clinician manipulates the outer end of the joystick handle. Such movement of the joystick is detectable by the control system which operates the articulation system in response to the input from the sensor seat. The articulation actuator 20160 comprises one or more biasing mechanisms, such as springs, for example, configured to bias the joystick handle to a centered, or an at least substantially centered position, in the sensor seat in which the control system does not articulate the end effector 10400.

As discussed above, the end effector 10400 is articulatable within a plane. In alternative embodiments, a surgical instrument comprises a second articulation joint. In such embodiments, the end effector 10400 is rotatable within more than one plane. In various embodiments, a surgical instrument comprises an articulation joint which permits the end effector 10400 to be rotated within a three-dimensional spherical range of positions. Referring to FIG. 21, a surgical instrument 21000 comprises a shaft 21200 including an articulation joint 21500 which allows such articulation motion of the end effector 10400. The surgical instrument 21000 further comprises a handle 21100 including an articulation actuator 21160 in communication with a control system of the surgical instrument 21000. The articulation actuator 21160 comprises a three-dimensional joystick movable proximally, distally, upwardly, downwardly, and in compound directions. The joystick is movable distally to articulate the end effector to the right of the handle 20100 and proximally to articulate the end effector to the left of the handle 21100. The joystick is movable upwardly to articulate the end effector upwardly and downwardly to articulate the end effector downwardly, for example. The joystick is also movable in a direction which is both upward and distal to move the end effector in a direction which is both upward and to the right, for example. The joystick is also movable in a direction which is both downward and proximal to move the end effector in a direction which is both downward and to the left, for example. In at least one instance, the joystick comprises a handle having an inner end that is positioned in a sensor seat in communication with the control system of the surgical instrument 21000. The joystick is orbitable within the sensor seat by the clinician when the clinician manipulates the outer end of the handle. Such movement of the joystick is detectable by the control system which operates the articulation system in response to the input from the sensor seat. The articulation actuator 21160 comprises one or more biasing mechanisms, such as springs, for example configured to bias the joystick handle to a centered, or an at least substantially centered position, in the sensor seat in which the control system does not articulate the end effector 10400.

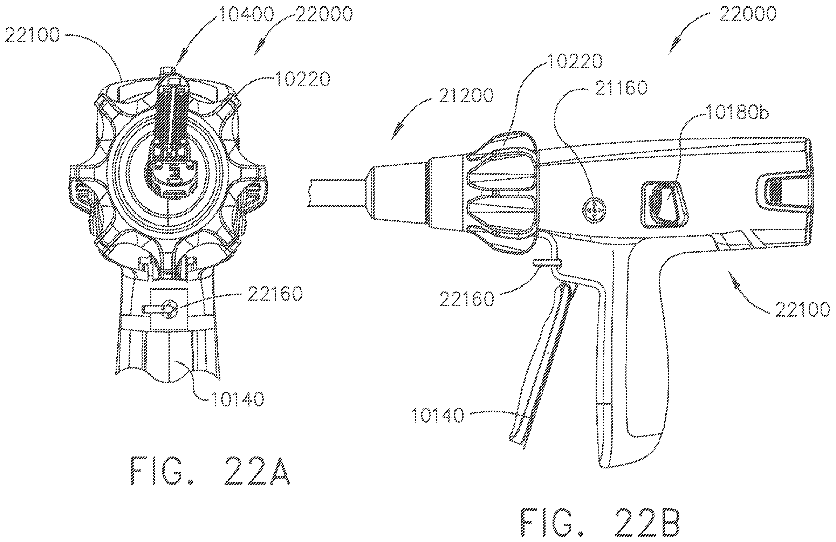

A surgical instrument 22000 is illustrated in FIGS. 22A and 22B. The surgical instrument 22000 is similar to the surgical instrument 21000 in many respects. The surgical instrument 22000 comprises a handle 22100 and a shaft 21200 extending from the handle 22100. The handle 22100 comprises the articulation actuator 21160 positioned on the side of the handle 22100 and, in addition, an articulation actuator 22160 positioned on the front of the handle 22100. Similar to the articulation actuator 21160, the articulation actuator 22160 comprises a three-dimensional joystick in communication with the control system of the surgical instrument 21000 and is capable of articulating the end effector of the surgical instrument 21000 in a three-dimensional field. The front articulation actuator 22160 is readily accessible by the index finger of a clinician holding a pistol grip of the handle 22100. Alternative embodiments are envisioned which comprise the articulation actuator 22160, but not the articulation actuator 22160.

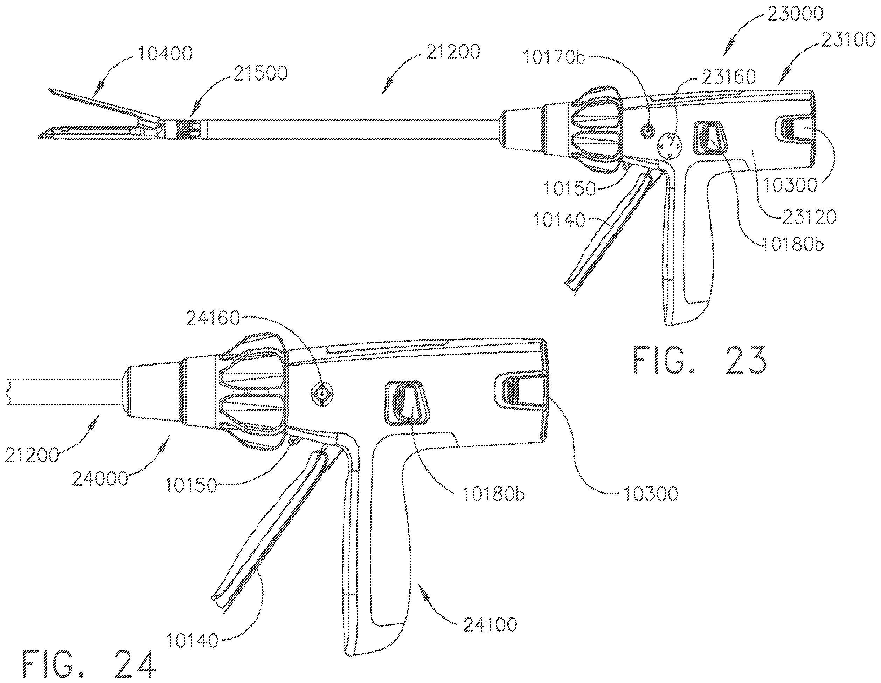

Referring to FIG. 23, a surgical instrument 23000 comprises a shaft 21200 including an articulation joint 21500 which allows for three-dimensional articulation motion of the end effector 10400. The surgical instrument 23000 further comprises a handle 23100 including a housing 23120 and, in addition, an articulation actuator 23160 in communication with a control system of the surgical instrument 23000. The articulation actuator 23160 comprises a four-way tactile control movable proximally, distally, upwardly, downwardly, and in compound directions. The four-way tactile control is movable distally to articulate the end effector to the right of the handle 23100 and proximally to articulate the end effector to the left of the handle 23100. The four-way tactile control is movable upwardly to articulate the end effector upwardly and downwardly to articulate the end effector downwardly. The four-way tactile control is also movable in a compound direction that is both upward and distal to move the end effector in a direction that is both upward and to the right, for example. The four-way tactile control is also movable in a compound direction that is both downward and proximal to move the end effector in a direction that is both downward and to the left, for example. In at least one instance, the four-way tactile control comprises four depressable actuators - one for each direction of right, left, up, and down - and each of which is part of a control circuit in communication with the control system of the surgical instrument 23000. The movement of the four-way tactile control is detectable by the control system which operates the articulation system in a three-dimensional range in response to the input from the articulation actuator 23160. The articulation actuator 23160 comprises one or more biasing mechanisms, such as springs, for example configured to bias the four-way tactile control to a centered, or an at least substantially centered position, in which the control system does not articulate the end effector 10400.

A surgical instrument 24000 is illustrated in FIG. 24. The surgical instrument 24000 is similar to the surgical instrument 23000 in many respects. The surgical instrument 24000 comprises a handle 24100 including an articulation actuator 24160. Similar to the articulation actuator 23160, the articulation actuator 24160 comprises a four-way tactile control. That said, the articulation actuator 24160 comprises an integral re-centering feature. More specifically, the articulation actuator 24160 comprises a depressable actuator positioned in the middle of the articulation actuator 24160 in communication with the control system of the surgical instrument 24000. When the center actuator is depressed, the control system operates to re-align the end effector 10400 with the longitudinal axis of the shaft 10200, much like the actuation of the actuator 10170 discussed above. As a result of the above, the re-centering actuator is positioned in the middle of the four directional actuators making for a compact and intuitive arrangement.

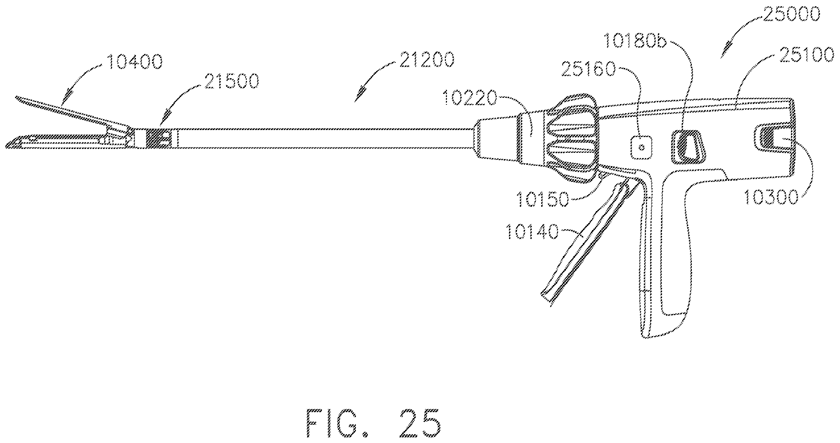

A surgical instrument 25000 is illustrated in FIG. 25. The surgical instrument 25000 is similar to the surgical instrument 24000 in many respects. The surgical instrument 25000 comprises a handle 25100 including an articulation actuator 25160. Similar to the articulation actuator 23160, the articulation actuator 25160 comprises a four-way control in communication with a control system of the surgical instrument 25000. That said, the four-way control comprises a capacitive surface which allows a clinician to tap and/or drag their finger across the surface of the articulation actuator 25160 to control the articulation of the end effector in a three-dimensional range. In at least one instance, the articulation actuator comprises a touchscreen and an array of capacitive sensors positioned under the touchscreen configured to detect the presence and/or motion of the clinician's finger, for example. In use, tapping the top of the capacitive surface articulates the end effector 10400 upwardly, tapping the bottom of the capacitive surface articulates the end effector 10400 downwardly, tapping the distal end of the capacitive surface articulates the end effector 10400 to the right, and tapping the proximal end of the capacitive surface articulates the end effector 10400 to the left, for example. Tapping the center of the articulation screen re-centers the end effector 10400 along the longitudinal axis of the shaft 21200. When a rotating motion is made on the surface of the articulation actuator 25160, the control system rotates the end effector 10400 in the direction and/or speed indicated by the rotating motion. In various instances, the control system of the surgical instrument 25000 comprises a pulse width modulation (PWM) control circuit for controlling the speed of the electric motor used to drive the articulation system of the surgical instrument 25000. In at least one embodiment, the control system comprises a frequency modulation (FM) control circuit in addition to or in lieu of the PWM control circuit for controlling the speed of the articulation motor.

As discussed above, an end effector of a surgical instrument can be rotatable in more than one direction and/or plane. To achieve this, in various embodiments, a surgical instrument comprises a first motor-driven system for moving the end effector in a left-to-right manner and a second motor-driven system for moving the end effector in an up-to-down manner. Both motor-driven systems are in communication with the control system of the surgical instrument and are drivable sequentially and/or concurrently by the control system to position the end effector in the direction indicated by the input from the articulation actuator, or articulation actuators.

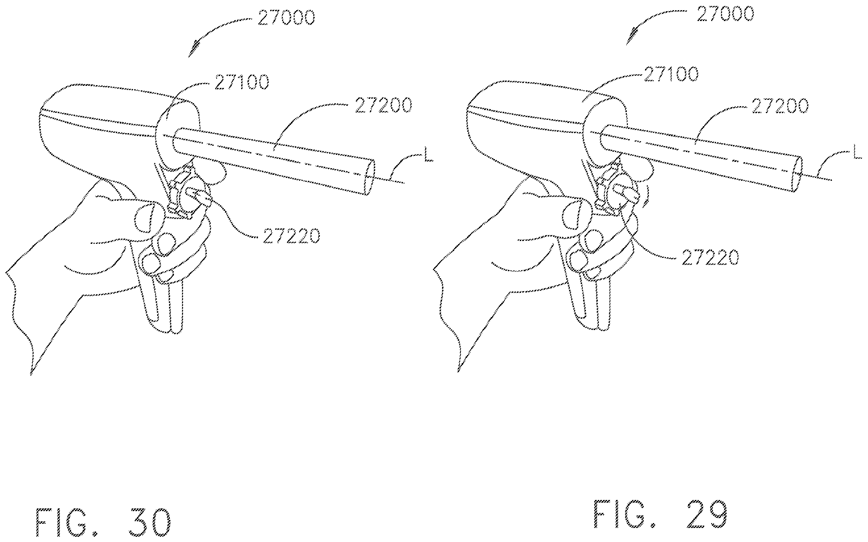

Many of the surgical instruments described above comprise a grip configured to be grasped by a clinician to rotate the shaft about a longitudinal axis. In various instances, the clinician can hold the grip with one hand and can extend their index finger, for example, from that hand to grab the grip and rotate the shaft. Such an arrangement, however, requires the clinician to have a somewhat larger hand. While such a surgical instrument can be operated with one hand, a surgical instrument 27000 is illustrated in FIGS. 29 and 30 that may be easier to use. The surgical instrument 27000 comprises a handle 27100 and a shaft 27200 extending from the handle 27100 that is rotatable about a longitudinal axis. The handle 27100 comprises a handle frame 27110 and a housing that rotatably support the shaft 27200. The handle 27100 further comprises an actuator 27220 positioned on the front side of the handle housing 27110 which, when rotated by the clinician, rotates the shaft 27200 about its longitudinal axis L. The actuator 27220 is rotatably mounted to the handle housing 27110 and is rotatable about an axis A which is parallel to, or at least substantially parallel to, the longitudinal axis of the shaft 27200. The actuator 27220 comprises a ring of gear teeth extending around its perimeter which is operably engaged with a ring of gear teeth extending around the perimeter of the shaft 27200 via a transmission gear 27225 such that, when the actuator 27220 is rotated about its axis, the shaft 27200 is rotated about its longitudinal axis. That said, the gear teeth of the actuator 27220 are not directly engaged with the gear teeth of the shaft 27200; instead, the intermediate gear 27225 - which is rotatably mounted in the handle 27100 - is directly engaged with the gear teeth of the actuator 27220 and the shaft 27200. Such an arrangement synchronizes the motion of the actuator 27220 and the shaft 27200, i.e., rotating the actuator 27220 to the right rotates the shaft 27200 to the right and rotating the actuator 27220 to the left rotates the shaft 27200 to the left. Absent the introduction of the intermediate gear 27225, the shaft 27200 would rotate in an opposite direction, but such an arrangement may provide a torque balance that promotes the stability of the instrument.

Further to the above, embodiments are envisioned in which the rotation of the shaft 27200 is driven by an electric motor. In various embodiments, the actuator 27220, when rotated in the first direction, operates the electric motor to rotate the shaft 27200 in the first direction. Similarly, the electric motor rotates the shaft 27200 in the second direction when the actuator 27220 is rotated in the second direction. In at least one embodiment, the output shaft of the electric motor comprises a pinion gear operably intermeshed with the ring of gear teeth around the shaft 27200. Moreover, in at least one embodiment, the actuator 27220 comprises one or more sensors configured to detect the direction and degree of rotation of the actuator 27220 which are in communication with a control system of the surgical instrument. With this data, the control system is configured to control the direction and speed of the electric motor. In instances where the actuator 27220 is rotated a small amount in the first direction, for example, the shaft 27220 is rotated slowly in the first direction whereas the shaft 27220 is rotated quickly in the first direction when the actuator 27220 is rotated a larger amount in the first direction.

Further to the above, the actuator 27220 comprises a bar including a first end and a second end. The orientation of the bar is synchronized with the orientation of the shaft 27200. When the first end of the bar is directly above the second end, i.e., the first end is closest to the shaft 27200, the shaft 27200 is in its top-dead-center (TDC) position. Correspondingly, the shaft 27200 is in its bottom-dead-center (BDC) position when the second end of the bar is directly above the first end, i.e., the second end is closest to the shaft 27200. As a result of this arrangement, the user of the surgical instrument has an intuitive feel of the orientation of the shaft 27200 based on the orientation of the actuator 27220.

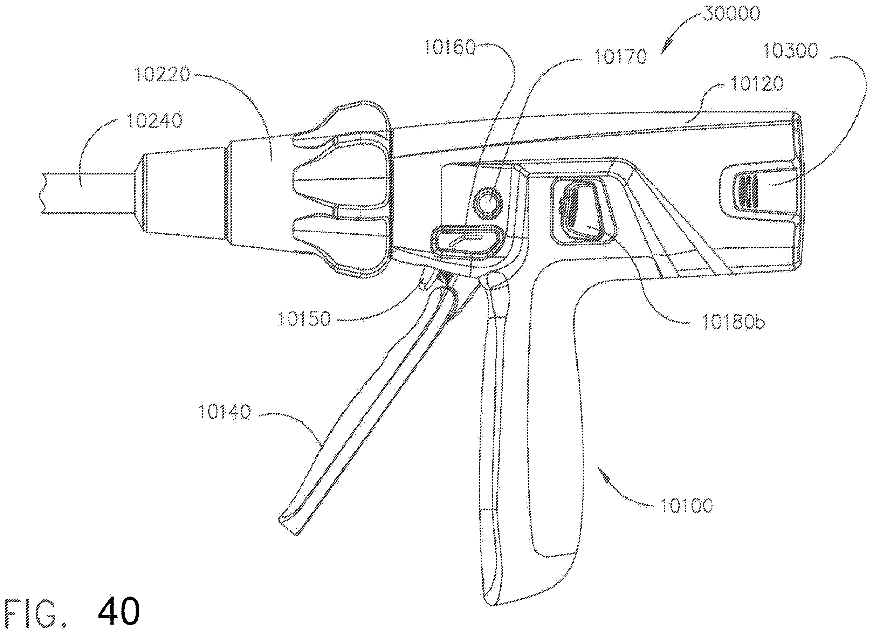

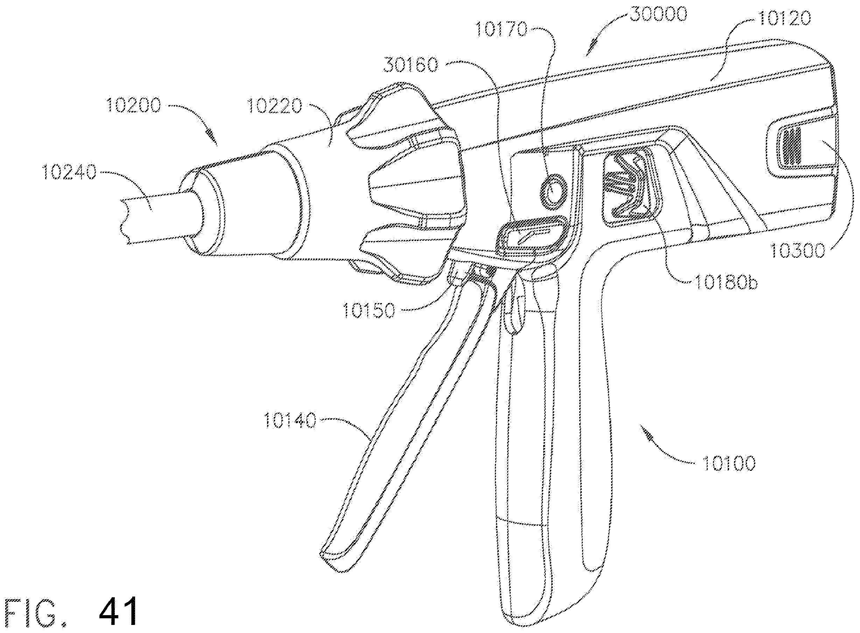

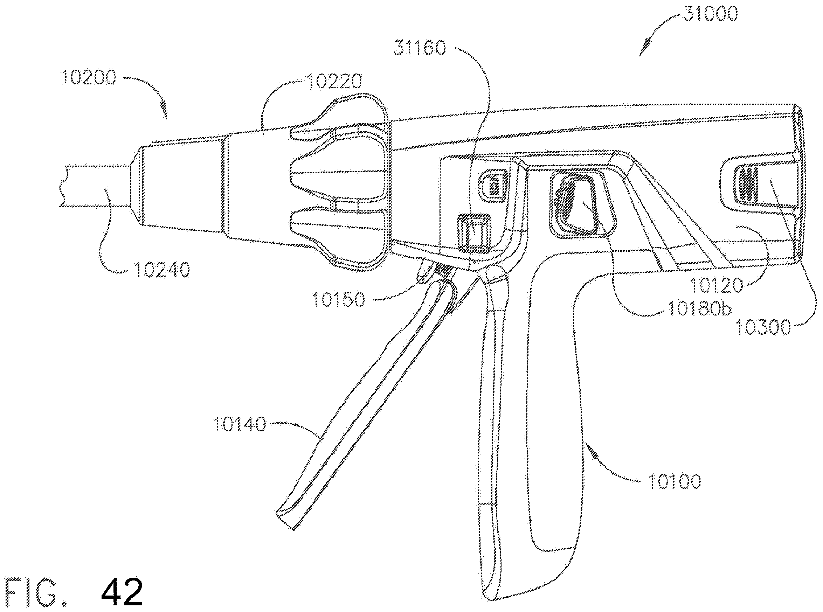

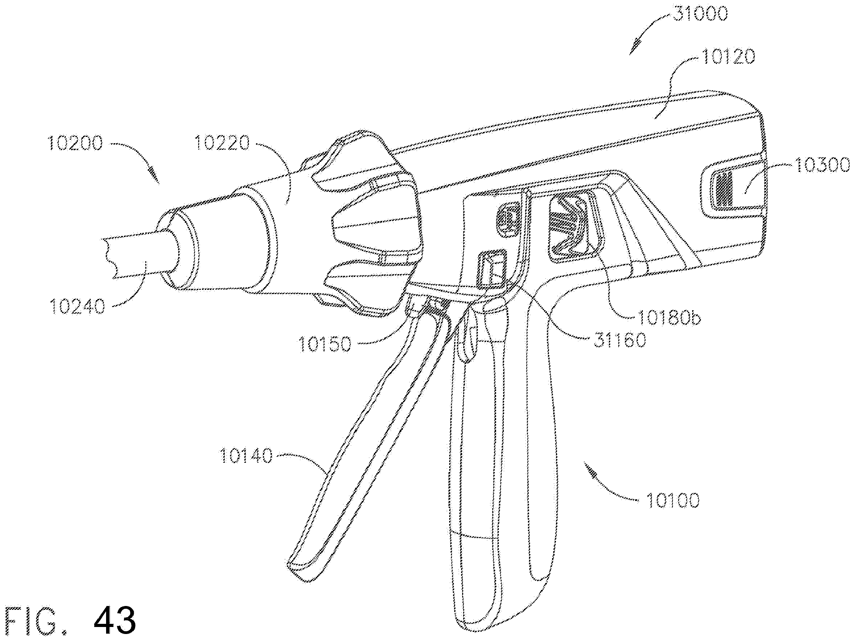

A surgical instrument 30000 is illustrated in FIGS. 40 and 41. The surgical instrument is similar to the surgical instrument 10000 in many respects. As opposed to the vertical articulation actuator 10160, the handle of the surgical instrument 30000 comprises a horizontal articulation actuator 30160. The horizontal articulation actuator 30160 comprises a rocker switch which can be rocked distally to rotate the end effector to the right and rocked proximally to rotate the end effector to the left. A surgical instrument 31000 is illustrated in FIGS. 42 and 43. The surgical instrument is similar to the surgical instrument 10000 in many respects. As opposed to the vertical articulation actuator 10160, the handle of the surgical instrument 31000 comprises an articulation actuator 31160. The articulation actuator 31160 comprises a multi-axis rocker switch that can be rocked proximal-to-distal to articulate the end effector in one plane and up-to-down to articulate the end effector in another plane. In various instances, the articulation planes are orthogonal to one another, but can be arranged in any suitable manner.

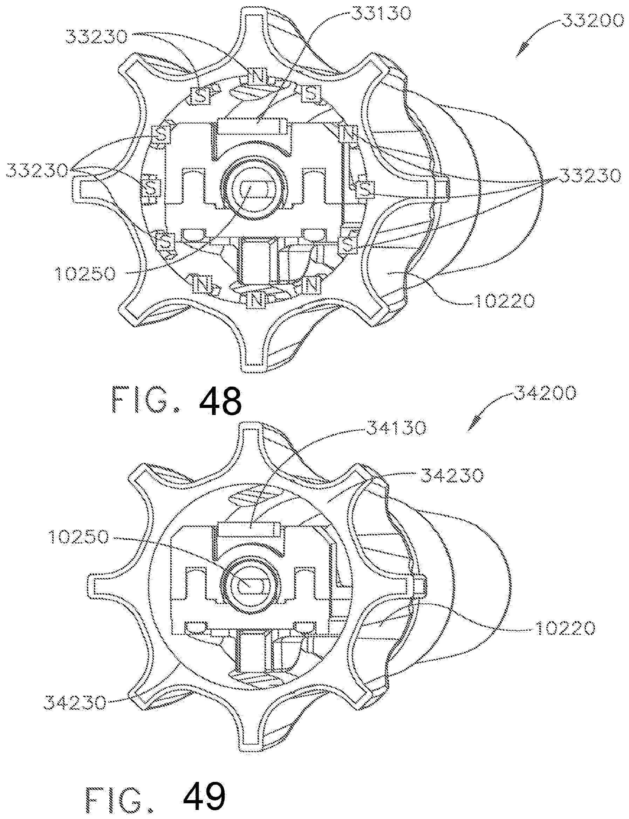

As discussed above, the control system of a surgical instrument can comprise an algorithm which, according to predetermined criteria, flips and/or otherwise re-orients the controls of the surgical instrument in certain instances. In various instances, as also discussed above, the algorithm can be configured to flip the articulation controls of the surgical instrument based on the rotation of the shaft relative to the handle. Referring to FIG. 48, a surgical instrument comprises a handle comprising a Hall Effect sensor 33130, and/or any other suitable sensor, in communication with the control system of the surgical instrument and, in addition, a shaft 33200 including an array of magnets 33230 arranged in a circular, or annular, pattern around the shroud, or grip, 10220 of the shaft 33200. Each magnet 33230 comprises a north pole (N) and a south pole (S) and the magnets 33230 are arranged in the manner indicated in FIG. 5489 - the N poles of some of the magnets 33230 are facing the handle while some S poles are facing toward the handle. When the shaft 33200 is rotated relative to the handle, this arrangement of the magnets 33230 allows the control system to track the position of the shaft 33200 and understand the orientation, or rotation, of the shaft 33200 relative to the handle. Within any three consecutive magnets 33230, for example, the pattern of magnets 33230 create a unique identifiable signature for a given rotation direction. That said, any suitable number and/or arrangement of discrete magnets could be used. Although twelve magnets 33230 are used, less than twelve magnets could be used - such as six magnets, for example. Moreover, more than twelve magnets could be used.

Referring to FIG. 49, a surgical instrument comprises a handle comprising a Hall Effect sensor 34130, and/or any other suitable sensor, in communication with the control system of the surgical instrument and, in addition, a shaft 34200 including a continuous annular magnet 34230 attached to the shroud, or grip, 10220 of the shaft 34200. In various instances, the annular magnet 34230 comprises a disc or ring embedded with magnetic microstructures which is detectable by the Hall Effect sensor. The annular magnet 34230 comprises a continuous, but varying, magnetic pattern around the perimeter thereof which provides a trackable pattern for the control system to assess the orientation, or rotation, of the shaft 34200. In other embodiments, the annular magnet 34230 comprises an intermittent magnetic pattern around the perimeter thereof that is trackable by the control system.

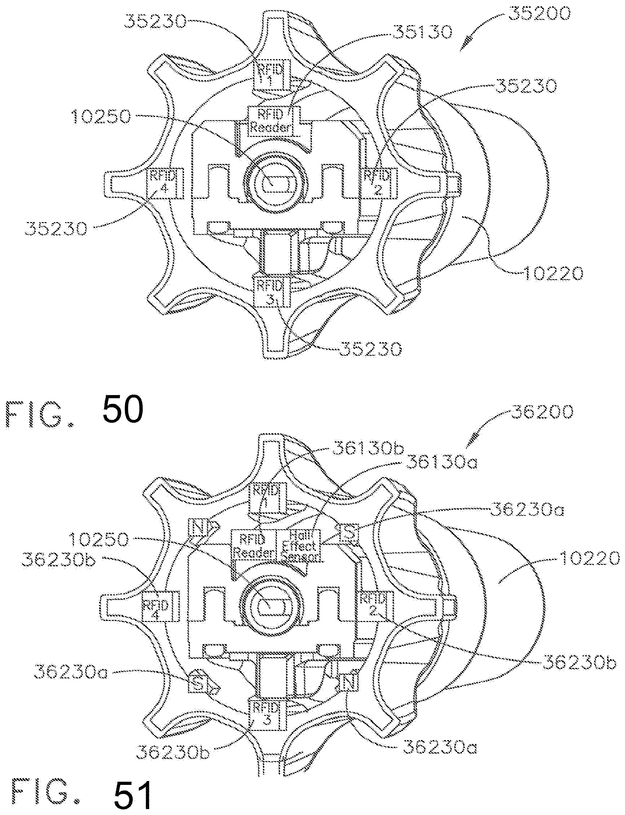

Referring to FIG. 50, a surgical instrument comprises a handle comprising a RFID reader 35130 in communication with the control system of the surgical instrument and, in addition, a shaft 35200 including a circular, or annular, array of RFID chips 35230 around the shroud, or grip, 10220 of the shaft 35200. Each RFID chip comprises a unique identification which is detectable by the RFID reader 35130 and, with this information, the control system is able to assess the orientation, or rotation, of the shaft 35200 relative to the handle. Notably, the RFID reader 35130 has a limited range to read the RFID chips 35230 and, thus, may be only able to read the most-adjacent RFID chip 35230. In some instances, the RFID reader 35130 can have sufficient range to read the two most-adjacent RFID chips 35230. The shaft 35200 comprises four RFID chips 35230, but can comprise any suitable number of RFID chips 35230. That said, the accuracy, or resolution, of the assessment made by the control system can be improved with more RFID chips in various instances.

Referring to FIG. 51, a surgical instrument comprises a handle comprising a Hall Effect sensor 36130a, and/or any other suitable sensor, in communication with the control system of the surgical instrument and, in addition, a shaft 36200 including an array of magnets 36230a arranged in a circular, or annular, pattern around the shroud of the shaft 36200. The handle also comprises a RFID reader 36130b in communication with the control system of the surgical instrument and, in addition, a circular, or annular, array of RFID chips 36230b around the shroud of the shaft 36200. The control system is configured to use the data from the Hall Effect sensor 36130a and the RFID reader 36130b to assess the orientation of the shaft 36200 relative to the handle. Notably, the RFID chips 36230b are positioned intermediate the magnets 36230a which provides the control system with a detectable resolution between adjacent magnets 36230a. Similarly, the magnets 36230a are positioned intermediate the RFID chips 36230b which provides the control system with a detectable resolution between the RFID chips 36230b.

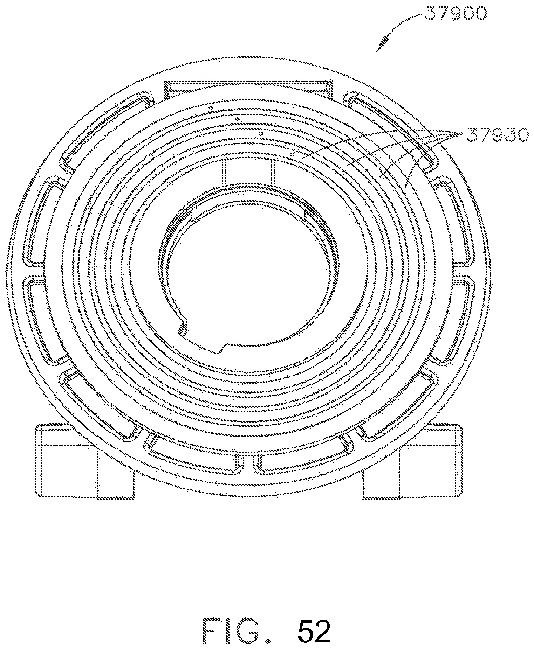

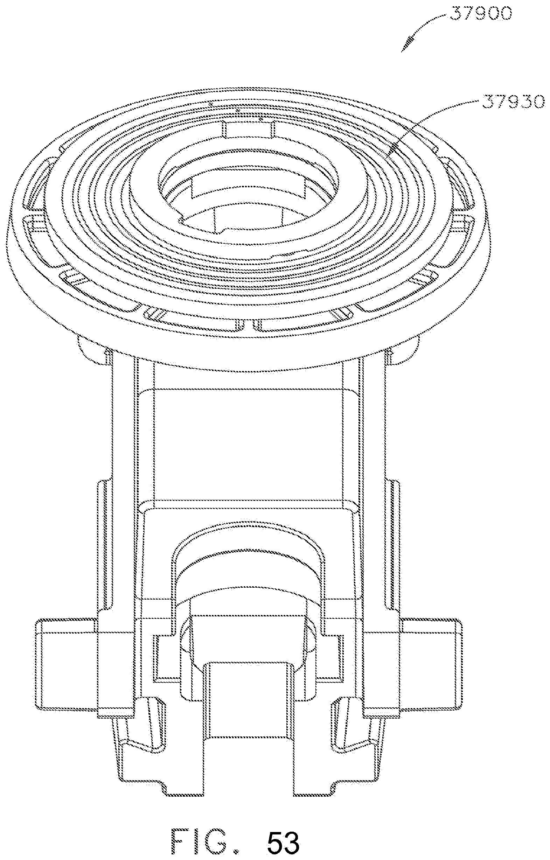

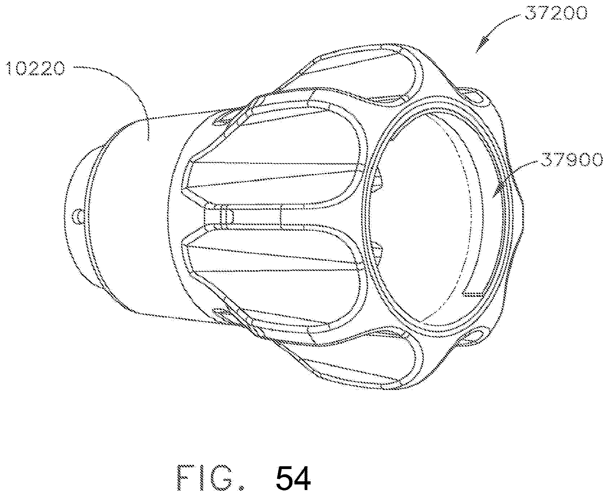

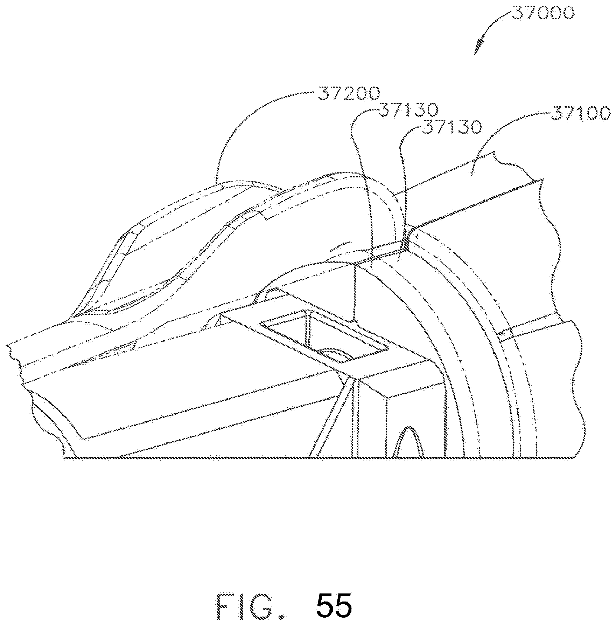

A surgical instrument 37000 is illustrated in FIGS. 52-55. The surgical instrument 37000 comprises a handle 37100 and a shaft 37200 extending from the handle 37100. The surgical instrument 37000 further comprises a slip joint 37900 between the handle 37100 and the shaft 37200. The slip joint 37900 comprises an electrical interface between the handle 37100 and the shaft 37200. The slip joint 37900 comprises annular rings 37930 mounted in the shaft 37200. Four annular rings 37930 are depicted in FIGS. 52 and 53, but a slip joint can comprise any suitable number of rings. The slip joint 37900 further comprises electrical contacts 37130 in the handle 37100. For instance, the slip joint 37900 comprises a first electrical contact 37130 engaged with a first annular ring 37930 and a second electrical contact 37130 engaged with a second annular ring 37930. That said, the slip joint 37900 can comprise any suitable number of electrical contacts to maintain power and/or signal communication between the handle and the shaft. Throughout the rotation of the shaft 37200, i.e., all 360 degrees, the electrical contacts 37130 remain in electrical contact with their respective annular rings 37930. In various instances, each electrical contact 37130 comprises a spring element configured to bias the electrical contact towards its respective annular ring 37930. The electrical contacts 37130 are in communication with the control system of the surgical instrument 37000 - via separate circuits - such that the control system can assess the resistance of the circuits, and/or any other electrical properties of the circuits between the control system and the slip joint 37900. That said, the electrical contacts and rings of the slip joint 37900 can be part of any suitable circuit arrangement.

Further to the above, the slip joint 37900 can be used as an absolute position sensor for the shaft 37200 relative to the handle 37100. More specifically, an intermediate annular ring 37930, i.e., the annular ring 37930 between the first ring 37930 and the second ring 37930, can be used by the control system to assess the orientation of the shaft 37200. To this end, the slip joint 37900 comprises an intermediate electrical contact 37130 in electrical communication with the intermediate annular ring 37930 and the control system as part of an intermediate electrical circuit. The intermediate annular ring 37930 is comprised of a high-resistance material, as compared to the first and second annular rings 37930, and provides a 10,000 Ohm resistance, for example. The intermediate annular ring 37930 has a first portion which is electrically coupled to the first annular ring 37930, a second annular portion which is electrically coupled to the second annular ring 37930, and a small break therebetween. When the shaft 37200 is rotated relative to the handle 37100, the intermediate electrical contact 37130 slides along the intermediate annular ring 37930 and the resistance and voltage of the intermediate electrical circuit changes in a manner which is detectable by the control system owing to the closing and opening of the break by the intermediate contact 37130. The signal from the intermediate electrical circuit is digitized by an analog-digital converter of the control system, the data from which is usable by the control system to assess the orientation of the shaft 37200. In various instances, any suitable number of gaps in the intermediate annular ring 37930 and/or intermediate contacts 37130 can be used to provide a signal with sufficient resolution to determine the orientation, or rotation, of the shaft 37200 relative to the handle 37100.

In various embodiments, a resistive material is embedded in the shaft of a surgical instrument which is part of an electrical circuit that passes through a slip ring. As the shaft rotates, the resistance in the electrical circuit changes - which is detectable by the control system of the surgical instrument to assess the angular orientation of the shaft relative to the handle.

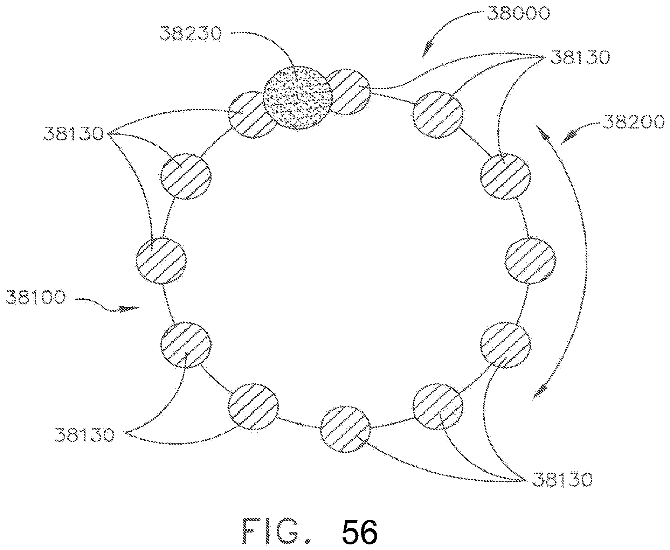

A representation of a surgical instrument 38000 is illustrated in FIG. 56. The surgical instrument 38000 comprises a handle 38100 and a shaft 38200 extending from the handle 38100. The handle 38100 comprises an annular array of Hall Effect sensors 38130 affixed to the frame and/or housing of the handle 38100. The Hall Effect sensors 38130 are positioned along a circumference in the handle 38100, as illustrated in FIG. 56. The Hall Effect sensors 38130 are in communication with the control system via electrical circuits. The shaft 38200 comprises a magnet 38230 mounted to the shroud of the shaft 38200 which is aligned, or at least substantially aligned, with the circumference of the Hall Effect sensors 38130. When the shaft 38200 is rotated about its longitudinal axis, the magnet 38230 moves along the sensor circumference. The sensors 38130 are positioned and arranged such that one or more of the sensors 38130 can detect the position of the magnet 38230 and, thus, the control system can determine the orientation of the shaft 38200 relative to the handle 38100 based on which Hall Effect sensors 38130 have detected the magnetic distortion, and the distortion intensity, created by the magnet 38230.

In various embodiments, a surgical instrument can include one or more optical sensors configured to detect the orientation of the shaft relative to the handle. In at least one embodiment, the handle of the surgical instrument comprises a light emitter and a light detector which are in communication with the control system of the surgical instrument. The shaft comprises a reflective surface that rotates with the shaft. The light emitter emits light onto the reflective surface and the light is reflected back into the light detector. The reflective surface comprises different portions with different reflectivities which creates patterns in the light reflected back to the light detector. With this information, the control system can assess the orientation of the shaft relative to the handle. In various instances, the reflective surface comprises openings and solid areas to create a binary off-on, or low-high, reflection response signal, for example.

In various embodiments, a surgical instrument comprises an electromechanical transducer, such as a linear variable differential transformer, for example, used in connection with a mechanical cam to measure the depth of the cam and relate it to the rotation angle of the shaft. In various embodiments, the handle of a surgical instrument comprises a magnetometer in communication with the control system and, in addition, and the shaft comprises a magnet which is detectable by the magnetometer.

In various embodiments, the shaft of a surgical instrument comprises a gyroscope sensor in the shaft which is used by the control system to assess the orientation of the shaft relative to the handle. In at least one such embodiment, the handle also comprises a gyroscope sensor in communication with the control system such that the relative orientation of the handle and the shaft can be assessed. In various embodiments, the shaft of a surgical instrument comprises a tilt sensor which is used by the control system to assess the orientation of the shaft relative to the handle. In at least one embodiment, a SQ-MIN-200 sensor can be used. A SQ-MIN-200 sensor acts like a normally-closed sensor which chatters open and closed as it is tilted or vibrated. That said, any suitable omnidirectional sensor, for example, could be used.

In various embodiments, a detectable element can be positioned on the clamp drive or closure tube of the shaft. When the shaft is rotated, the closure tube rotates with the shaft. Thus, the one or more sensors of the handle can detect the orientation of the shaft relative to the handle via the detectable element on the shaft. When the closure tube is translated to close the end effector, as described herein, the detectable element moves relative to the one or more sensors. Such translation of the detectable element can also be used to verify the closure of the end effector. In at least one instance, a Hall Effect sensor can be used to detect the rotation and translation of the detectable element. In various instances, the control system of a surgical instrument is configured to prevent the end effector from being articulated while the end effector is closed. This arrangement provides the feedback to the control system to determine not only the responsiveness of the articulation controls, but whether or not the control system should be responsive to the input from the articulation controls at all.

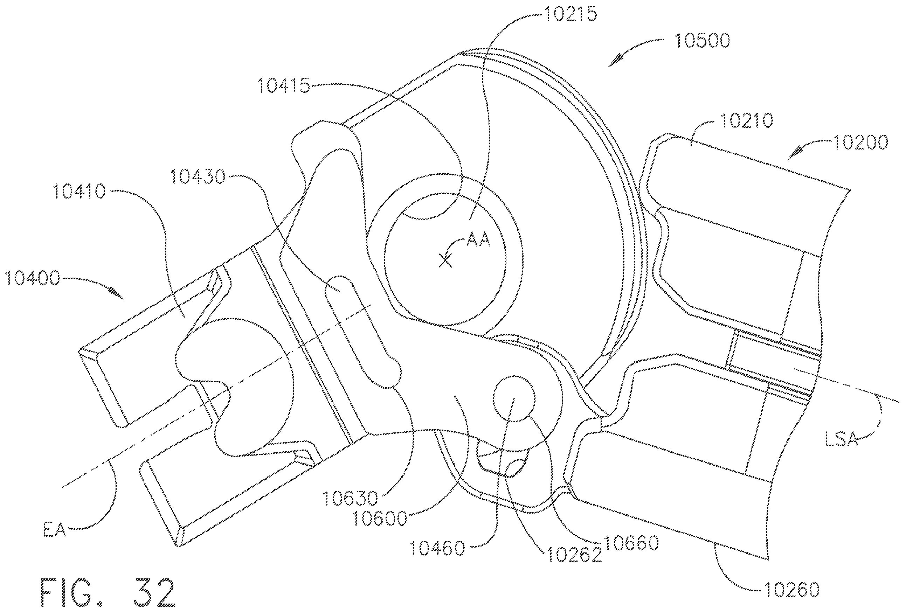

In various embodiments, referring again to FIGS. 27 and 28, the distal end of the articulation actuator 10260 of the surgical instrument 10000 is attached to the end effector 10400 such that the proximal and distal translation of the articulation actuator 10260 rotates the end effector 10400 about the articulation joint 10500. Referring to FIG. 32, the shaft 10200 of the surgical instrument 10000 comprises a shaft frame 10210 which slideably supports the articulation actuator 10260. Although not illustrated in FIG. 32, the shaft 10200 further comprises a pivot pin 10215 extending from the frame 10210. The pivot pin 10215 is closely received within a pivot aperture 10415 defined in the staple cartridge jaw 10410 of the end effector 10400 which defines an articulation axis AA of the articulation joint 10500. The articulation driver 10260 comprises a distal end including an aperture 10262 defined therein and the end effector 10400 further comprises an articulation pin 10460 extending from the proximal end of the staple cartridge jaw 10410 into the aperture 10262. When the articulation actuator 10260 is translated, as described above, the sidewalls of the aperture 10262 engage the articulation pin 10460 and either push or pull the articulation pin 10460 - depending on the direction in which the articulation actuator 10260 is translated. The entire disclosure of U.S. Patent No. 9,101,358 is relevant. The entire disclosure of U.S. Patent No. 5,865,361 is relevant.

Further to the above, the end effector 10400 defines an end effector axis EA and the shaft 10200 defines a longitudinal shaft axis LSA. When the end effector 10400 is in an unarticulated position, the end effector axis EA is aligned, or at least substantially aligned, with the longitudinal shaft axis LSA. When the end effector 10400 is in an articulated position, as illustrated in FIG. 32, the end effector axis EA is transverse to the longitudinal shaft axis LSA. The aperture 10262 is elongate in order to accommodate relative movement between the articulation pin 10460 and the articulation driver 10260; however, for large articulation angles, the articulation driver 10260 may bind and/or flex which can, without more, result in the articulation driver 10260 decoupling from the articulation pin 10460. With that in mind, the end effector 10400 further comprises a retention plate 10600 configured to hold the articulation driver 10260 in engagement with the articulation pin 10460. The retention plate 10600 comprises a planar, or an at least substantially planar portion, which extends over the distal end of the articulation driver 10260 and comprises an aperture 10660 defined therein, the sidewalls of which are engaged with the articulation pin 10460. As a result, the articulation driver 10260 is trapped between the staple cartridge jaw 10410 and the retention plate 10600 such that the articulation driver 10260 does not unintentionally disengage from the staple cartridge jaw 10410. The retention plate 10600 is fixedly mounted to the staple cartridge jaw 10410 such that there is little, if any, relative movement between the retention plate 10600 and the staple cartridge jaw 10410. The staple cartridge jaw 10410 comprises a retention lug 10430 and the retention plate 10600 comprises an aperture 10630 defined therein, the sidewalls of which are engaged with the retention lug 10430 to hold the retention plate 10600 to the staple cartridge jaw 10410. In various instances, the retention plate 10600 can comprise a spring and/or biasing member.

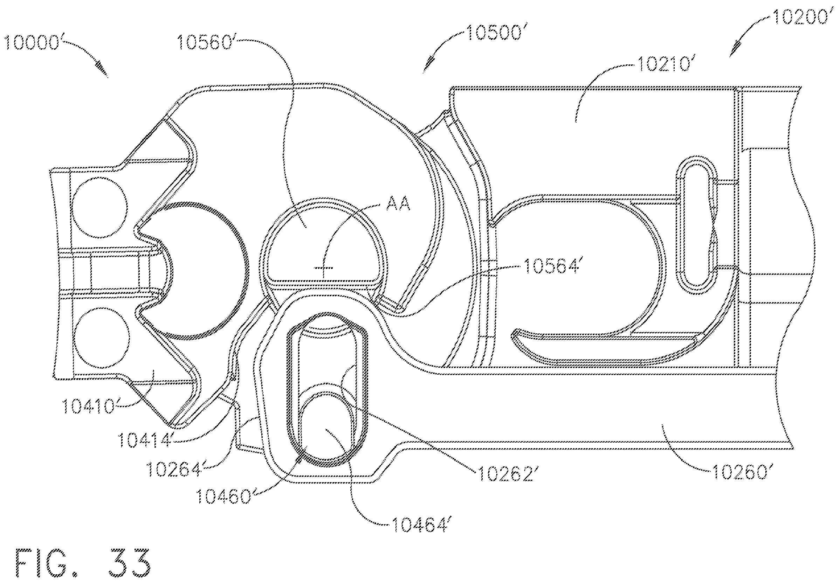

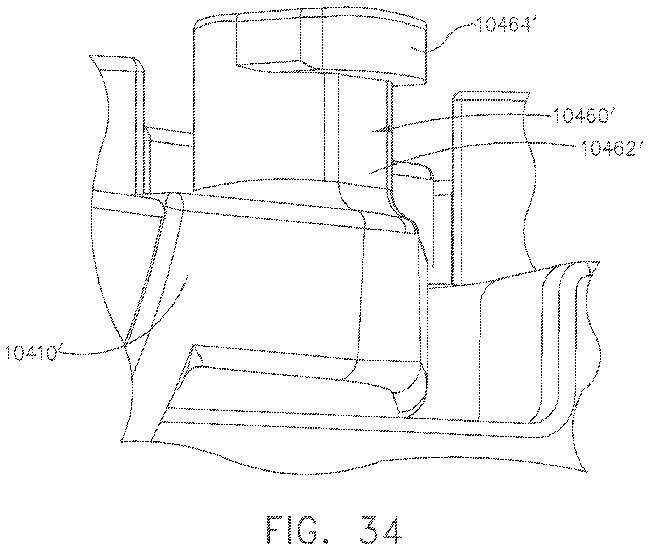

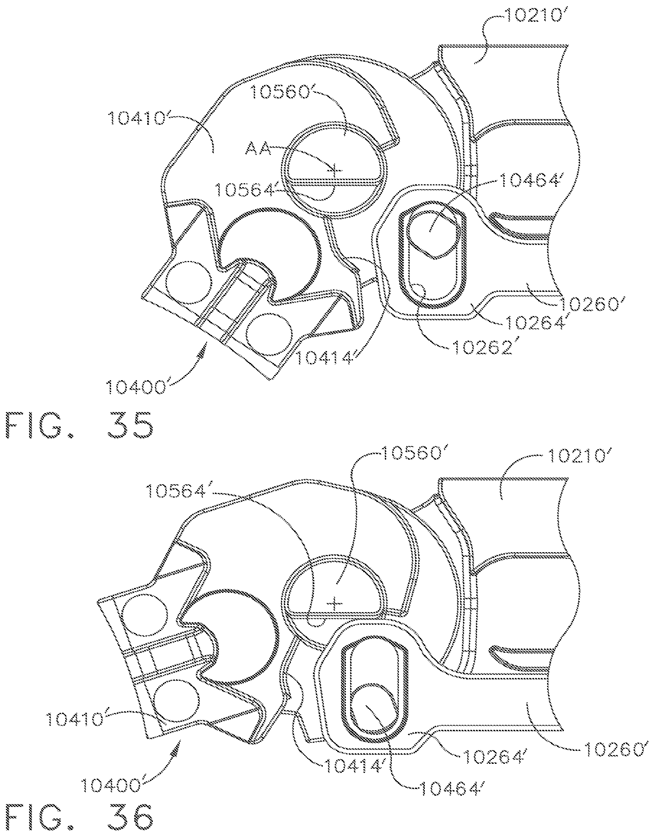

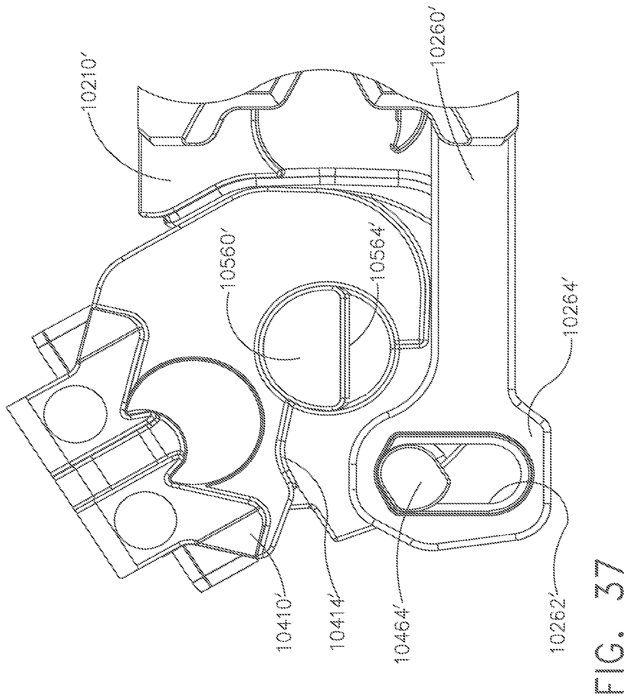

In addition to or in lieu of the retention plate 10600, referring now to FIG. 33, a surgical instrument 10000' comprises an end effector 10400' and an articulation joint 10500' rotatably connecting the end effector to the shaft 10200'. Further to the above, the articulation joint 10500' comprises a pin 10560' extending from a shaft frame 10210' of the shaft 10200' that is closely received within an aperture defined in the staple cartridge jaw 10410' which defines the articulation axis AA for the articulation joint 10500'. The surgical instrument 10000' also comprises an articulation driver 10260' which comprises a distal end 10264' including a slot 10262' defined therein. Similar to the above, the staple cartridge jaw 10410' comprises an articulation pin 10460' extending from the staple cartridge jaw 10410' which extends into the slot 10262' of the distal end 10264' and the interaction between the sidewalls of the slot 10262' and the articulation pin 10460' drive the end effector 10400' about the articulation joint 10500'. Notably, the pin 10560' of the articulation joint 10500' comprises a clearance relief 10564' defined therein to provide clearance for the longitudinal movement of the articulation driver 10260'. The staple cartridge jaw 10410' also comprises a clearance relief 10414' defined therein to permit clearance for the rotation of the staple cartridge jaw 10410' about the articulation joint 10500'. In order to prevent the articulation driver 10260' from becoming decoupled from the staple cartridge jaw 10410', referring to FIGS. 34-37, the articulation pin 10460' comprises a retention shoulder 10464' extending from a cylindrical portion 10462'. The retention shoulder 10464' extends over a portion of the distal end 10264' of the articulation driver 10260' throughout the articulation of the end effector 10400'. Thus, regardless of whether the end effector 10400' is articulated all the way to the left (FIG. 35) or all the way to the right (FIG. 37), or anywhere in between, the retention shoulder 10464' prevents, or at least limits the possibility of, the articulation driver 10260' disengaging from the staple cartridge jaw 10410'.

In various embodiments, further to the above, the clearance relief 10414' comprises a retention shoulder or lip which prevents the articulation driver 10260' from decoupling from the articulation pin 10460'. The retention shoulder 10464' of the articulation pin 10460' is sized and configured such that the width of the retention shoulder 10464' is wider than the width of the slot 10262'. That said, the slot 10262' comprises a length which is larger than its width which permits the retention shoulder 10464' to be interested through the slot 10262' such that the articulation driver 10260' can be assembled to the articulation pin 10460'. The width of the slot 10262' is defined along an axis that is parallel to the longitudinal axis of the shaft while the length of the slot 10262' is defined along an axis that is orthogonal to the longitudinal axis of the shaft. Such an arrangement permits the end effector to articulate relative to the shaft while minimizing binding between the end effector and the articulation driver 10260'. That said, the articulation driver 10260' is comprised of a flexible material that permits the articulation driver 10260' to resiliently flex to accommodate the end articulation of the end effector.

Described herein are various mechanisms and methods for determining the orientation of the shaft relative to the handle. Many of these mechanisms are able to evaluate the orientation of the shaft in real time and without regard to the previous orientation, or orientations, of the shaft. Such arrangements are particularly useful when the surgical instrument loses power, for example. When the surgical instrument re-powers, the control system can immediately assess the orientation of the shaft and the proper responsiveness of the articulation controls, for example. Moreover, the surgical instruments disclosed herein can be configured to immediately assess the articulation angle of the end effector when the surgical instrument is re-powered. Upon re-powering, the control system will evaluate whether the end effector is in a closed configuration or an open configuration. If the end effector is in a closed configuration upon re-powering, the control system will determine that the surgical instrument lost power during the staple firing mode and prompt the clinician to retract the staple firing system. If the end effector is in an open configuration upon re-powering, or once the end effector is in an open position upon re-powering, the control system will seek to make sure that the articulation drive system is coupled to the staple firing system such that the end effector can be straightened, or otherwise suitably oriented by the clinician, to remove the surgical instrument from the patient.

As discussed above, the surgical instruments disclosed herein may comprise control systems. Each of the control systems can comprise a circuit board having one or more processors and/or memory devices. Among other things, the control systems are configured to store sensor data, for example. They are also configured to store data which identifies the type of staple cartridge attached to a stapling instrument, for example. More specifically, the type of staple cartridge can be identified when attached to the stapling instrument by the sensors and the sensor data can be stored in the control system. This information can be obtained by the control system to assess whether or not the staple cartridge is suitable for use.

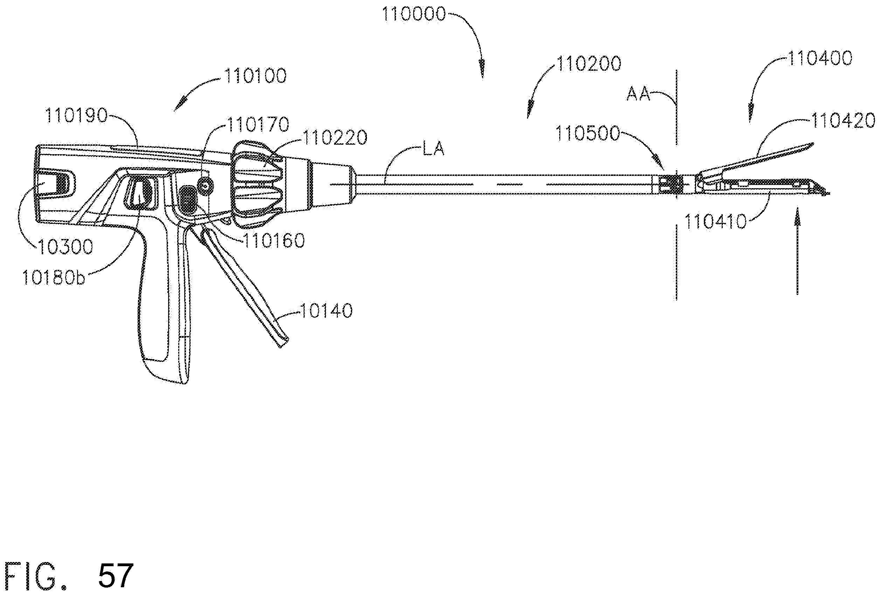

A surgical instrument 110000 is illustrated in FIG. 57. The surgical instrument 110000 comprises a handle 110100, a shaft 110200 extending from the handle 110100, and an end effector 110400 rotatably connected to the shaft 110200 about an articulation joint 110500. The surgical instrument 110000 is similar to the other surgical instruments disclosed herein and such similarities are not discussed herein for the sake of brevity. The shaft 110200 is fixedly attached to the handle 110100.

Further to the above, the surgical instrument 110100 comprises an articulation drive which is actuatable to articulate the end effector 110400 about an articulation axis AA, a closure drive including a closure actuator 10140, described above, which is actuatable to move a jaw 110420 of the end effector 110400 toward a jaw 110410, and a staple firing drive which is actuatable to fire the staples from the staple cartridge seated in the end effector 110400 during a staple firing stroke. The staple firing drive comprises an electric motor configured to advance a firing member distally through the staple firing stroke and retract the firing member proximally back into its unfired position. Similar to other embodiments described herein, the articulation drive is selectively engageable with the staple firing drive. An articulation member of the articulation drive is driveable by the staple firing drive when the articulation drive is engaged with the staple firing drive and, correspondingly, the articulation drive is not driveable by the staple firing drive when the articulation drive is not engaged with the staple firing drive. As described further below, the closure drive decouples the articulation drive from the staple firing drive when the closure drive is sufficiently actuated.

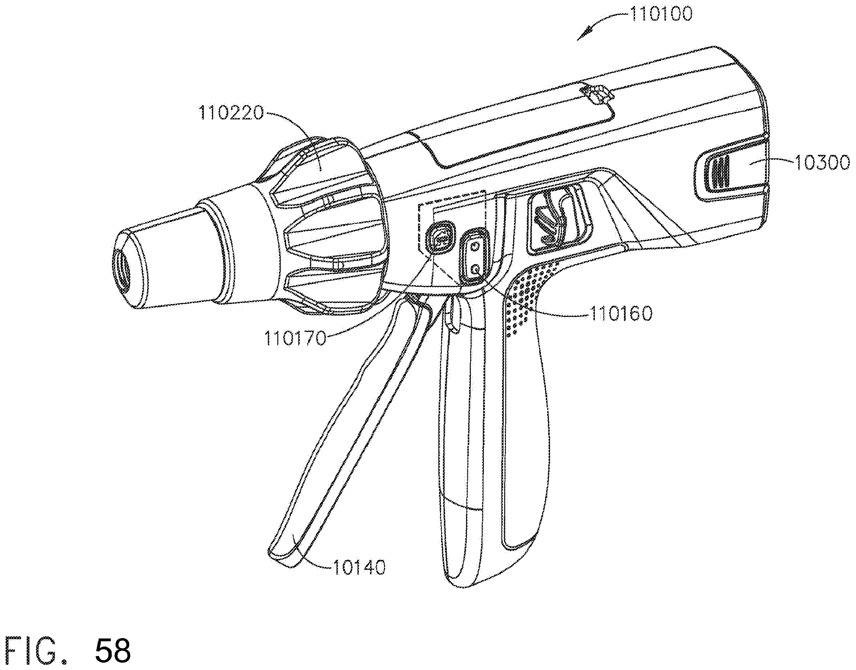

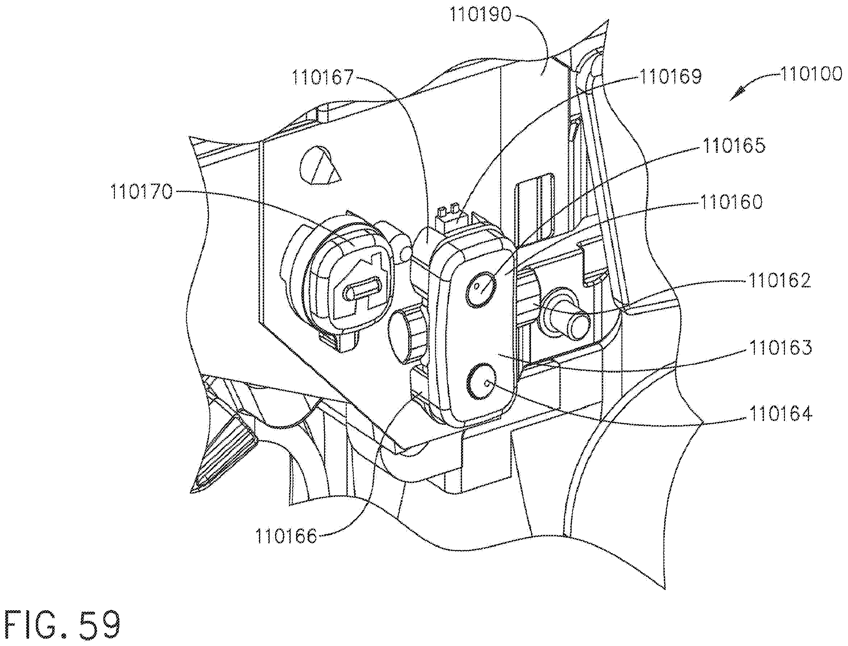

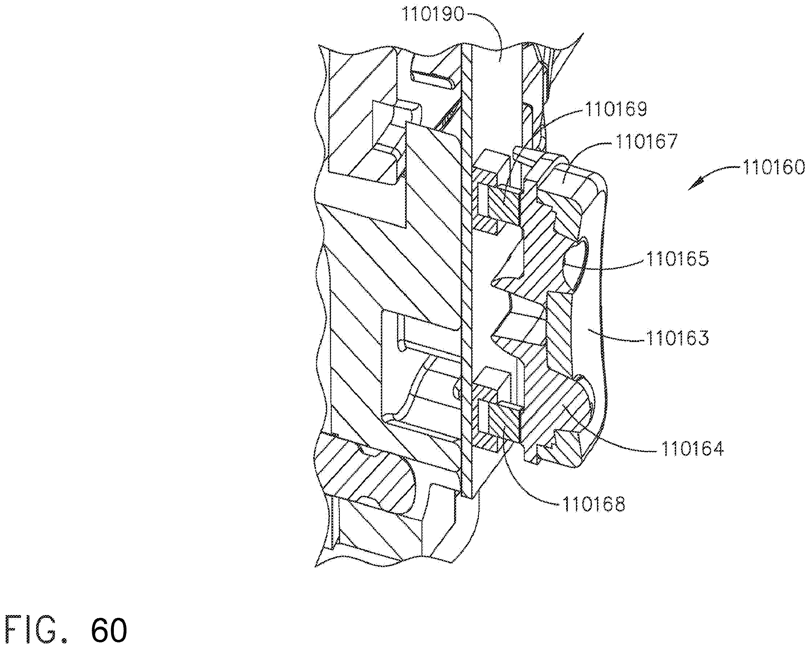

Referring to FIGS. 58-60, the handle 110100 comprises an articulation actuator 110160 which is actuatable to articulate the end effector 110400. The articulation actuator 110160 comprises a rocker switch, for example, including a rocker body 110163 which is rotatably mounted to a circuit board 110190 about a pivot 110162. The articulation actuator 110160 further comprises a first contact 110168 mounted to the circuit board 110190 which is moved from an open state to a closed state when a first end 110164 of the rocker body 110163 is depressed. When the first end 110164 is released, a biasing member in the first contact 110168 returns the first contact back into its open state. The articulation actuator 110160 also comprises a second contact 110169 mounted to the circuit board 110190 which is moved from an open state to a closed state when a second end 110165 of the rocker body 110163 is depressed. When the second end 110165 is released, a biasing member in the second contact 110169 returns the second contact back into its open state. The first contact 110168 and the second contact 110169 are in communication with a control system of the surgical instrument 110000. When the control system detects that the first contact 110168 has been closed, the control system operates the electric motor of the staple firing system to articulate the end effector 110400 in a first direction. Correspondingly, the control system operates the electric motor of the staple firing system to articulate the end effector 110400 in a second direction when the control system detects that the second contact 110169 has been closed.

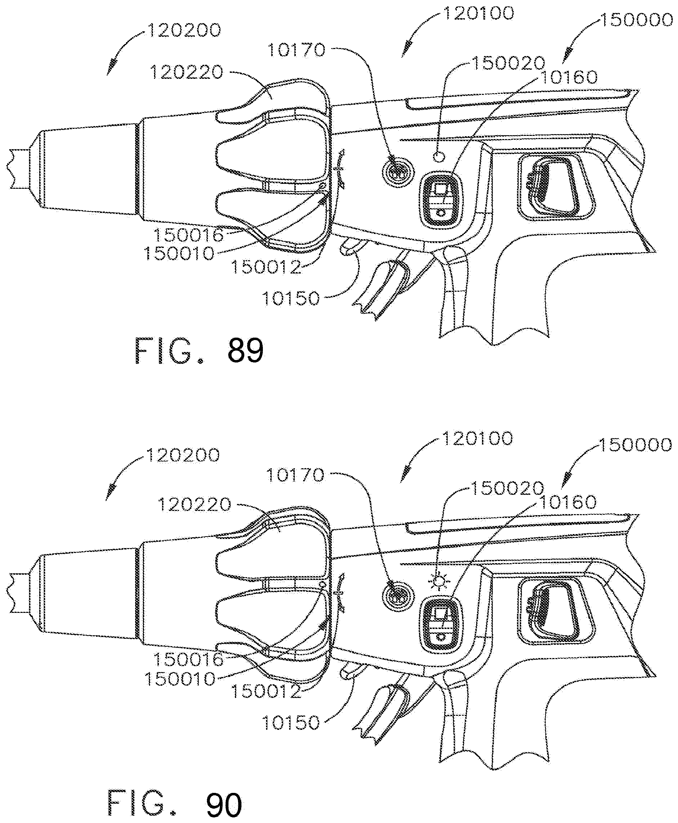

Further to the above, the rocker body 110163 comprises a first stand-off 110166 that contacts the circuit board 110190 when the rocker body 110163 is depressed in the first direction and limits the travel of the rocker body 110163. Similarly, the rocker body 110163 comprises a second stand-off 110167 that contacts the circuit board 110190 when the rocker body 110163 is depressed in the second direction and limits the travel of the rocker body 110163. Such an arrangement prevents or reduces the possibility of the articulation actuator 110160 from being damaged. Such an arrangement can also be adapted to other actuators on the handle 110100, such as an actuator 110170, for example. The actuator 110170 comprises a switch in communication with the control system of the surgical instrument 110100 which, when closed, causes the control system to automatically re-center the end effector 110400 along a longitudinal axis LA (FIG. 90) of the shaft 110200.

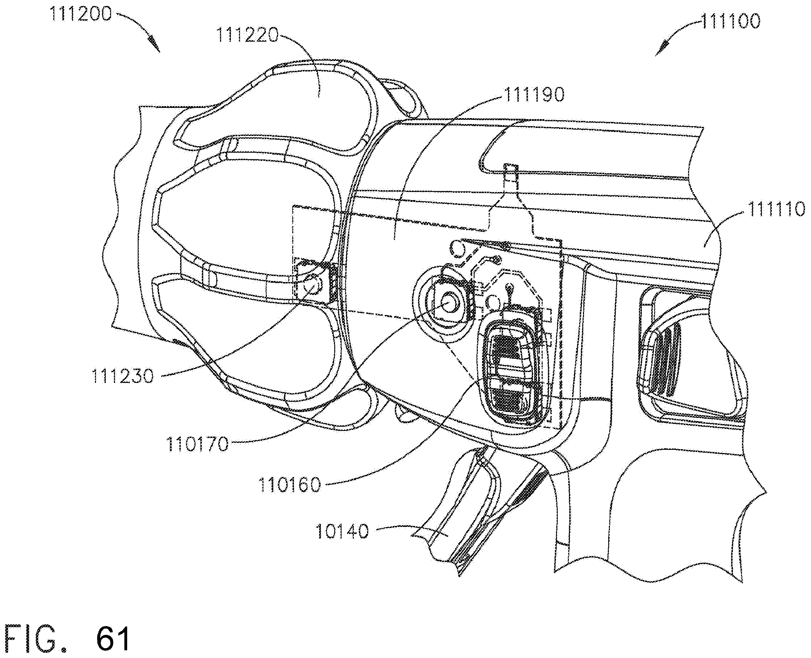

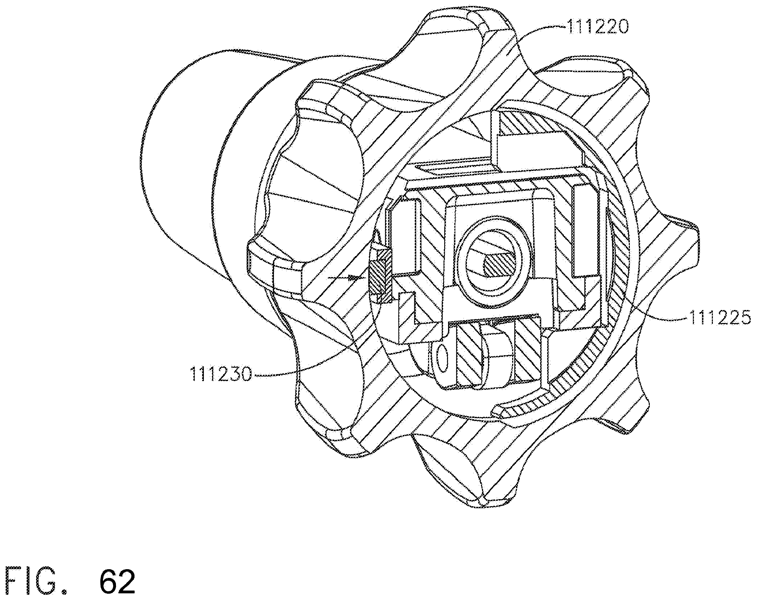

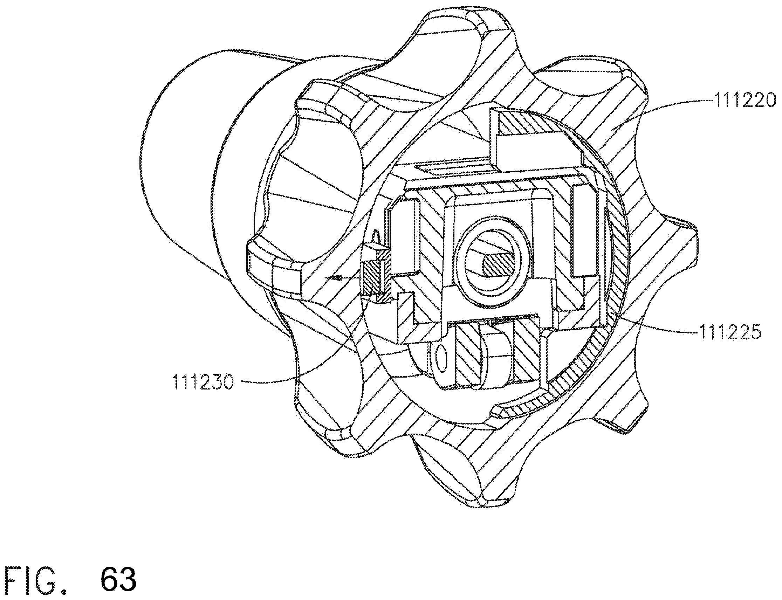

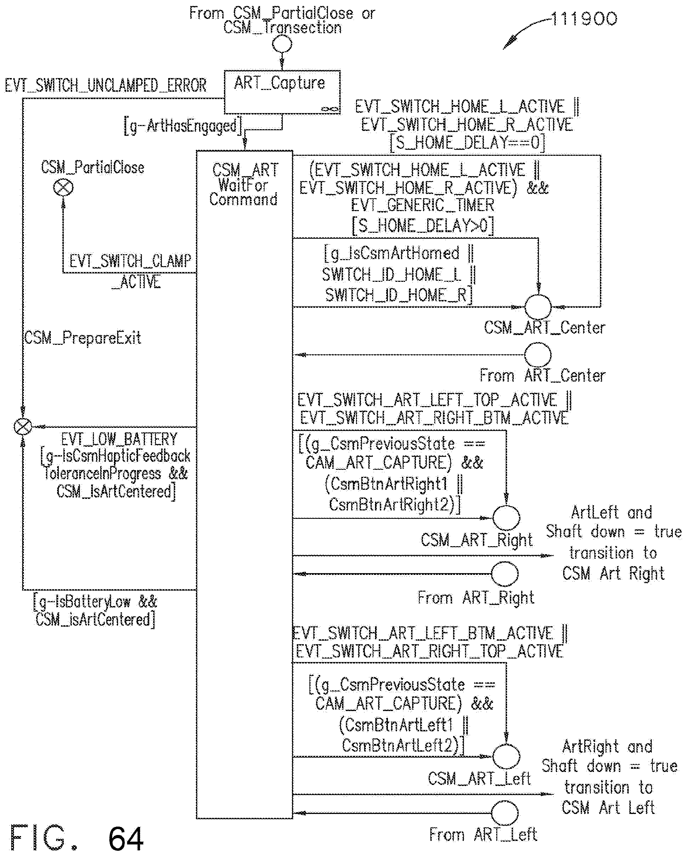

Further to the above, the shaft 110200 and the end effector 110400 are rotatable relative to the handle 110100 about the longitudinal axis LA. In use, a clinician can grasp a nozzle-shaped portion, or nozzle, 110220 of the shaft 110200 to rotate the shaft 110200 about the longitudinal axis. Similar to the above, referring to FIGS. 61-64, a surgical instrument can comprise a handle 111100 and a shaft 111200 rotatable relative to the handle 111100 about a longitudinal axis LA where the rotation of the shaft 111200 relative to the handle 111100 can be sensed by a sensor, or switch, 111230. The switch 111230 is mounted to a circuit board 111190 and, similar to the above, the switch 111230 is switched between a first, or open, state and a second, or closed, state when a cam 111225 of the nozzle 111220 comes into contact with the switch 111230. As a result, the rotation of the shaft 111200 is divided into two ranges - a first range of orientations in which the switch 111230 is in the first state and a second range of orientations in which the switch 111230 is in the second state. The switch 111230 is in communication with the control system of the surgical instrument 111000 and, depending on the input provided by the switch 111230, the control system controls the articulation of the end effector in a first response state and a second response state. In the second response state, the response of the articulation drive to the actuation of the articulation actuator 110160 is reversed, or flipped, as compared to the first response state. As described above, such an arrangement provides a more intuitive operation of the surgical instrument 111000 when the shaft 111200 is in a flipped, or upside-down, orientation. See the control system 111900 of FIG. 64, for example. This control system can be used in connection with any of the embodiments disclosed herein, such as the surgical instrument 110000, for example.

In various embodiments, further to the above, the control system of the surgical instrument 110000 becomes unresponsive to the articulation actuators 110160 and 110170 when the closure trigger 10140 is initially actuated to close the end effector 110400. Moreover, in such embodiments, the initial actuation of the closure trigger 10140 causes the articulation drive to decouple from the staple firing drive. Such embodiments entirely avoid the possibility of the end effector 110400 articulating while the end effector 110400 is clamped onto the tissue. That said, such embodiments require the clinician to estimate where the second jaw 110420 will contact the tissue when the second jaw 110420 is eventually closed after the end effector 110400 has been articulated. If the clinician has already partially-closed the end effector 110400, in such embodiments, the clinician must re-open the end effector 110400 to re-articulate the end effector 110400. In such embodiments, re-opening the end effector 110400 re-engages the articulation drive with the staple firing drive and the control system becomes responsive once again to the articulation actuators 110160 and 110170. In alternative embodiments, the end effector 110400 of the surgical instrument 110000 can be articulated while the end effector 110400 is in a partially-closed, or partially-clamped, configuration. Once the end effector 110400 is closed more than the partially-closed configuration, in these embodiments, the articulation drive is decoupled from the staple firing drive and the control system is no longer responsive to the articulation controls 110160 and 110170 until the end effector 110400 is re-opened or at least returned back to its partially-closed configuration.

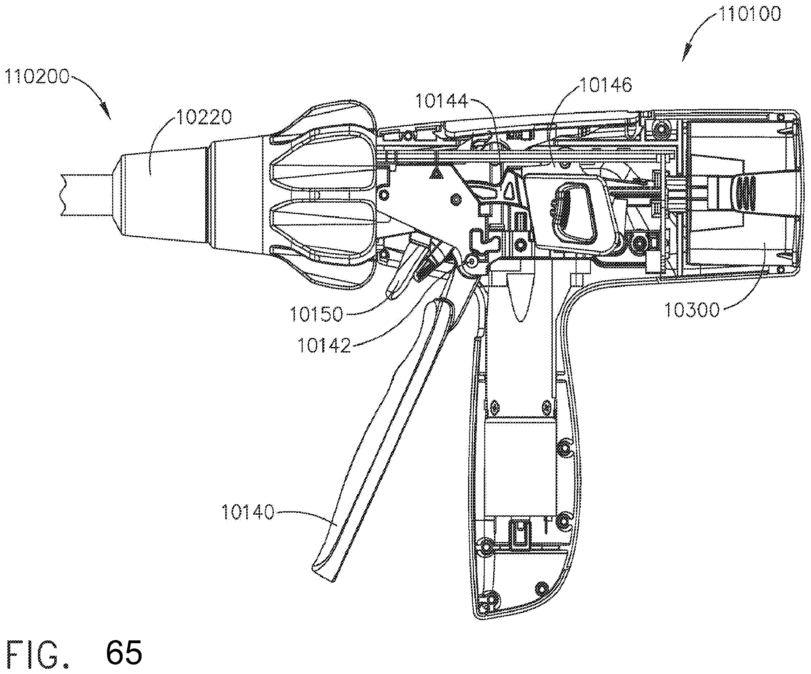

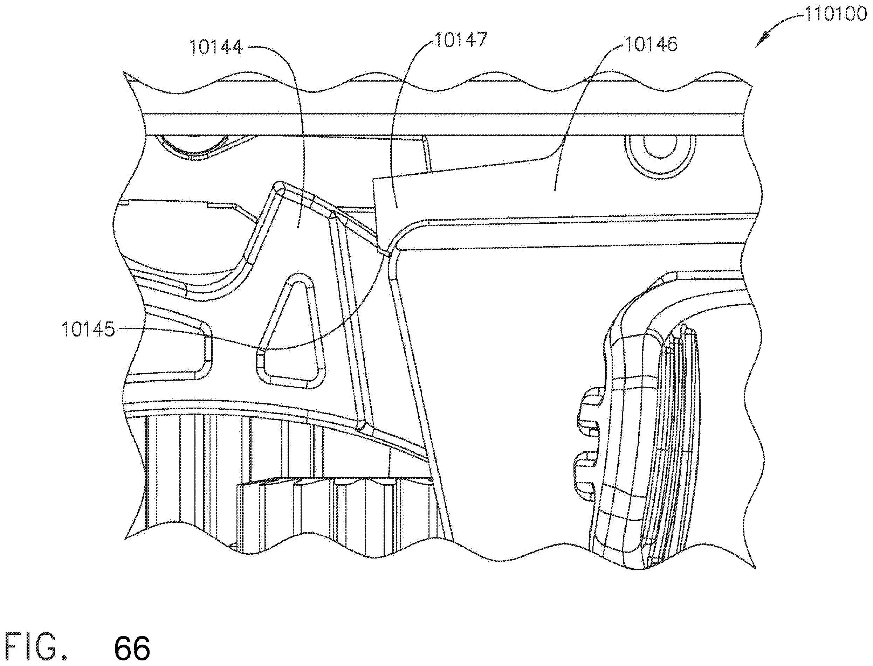

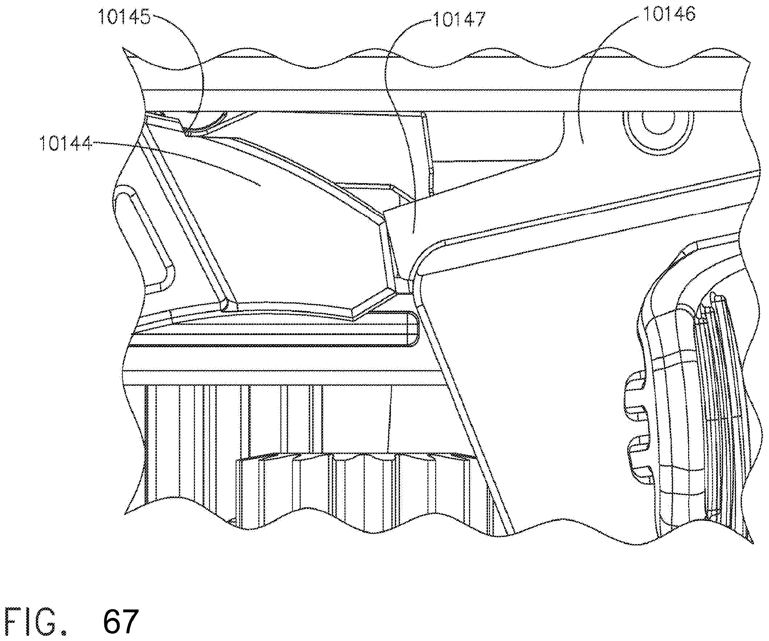

Further to the above, the partially-closed configuration of the end effector 110400 is a predefined, or predetermined, position of the second jaw 110420. In at least one such embodiment, referring to FIGS. 65-67, the surgical instrument 110100 comprises a closure lock 10146 configured to releasably hold the closure actuator 10140 in the pre-defined, partially-closed position. When the closure actuator 10140 is in this partially-closed position, the articulation drive is still engaged with the staple firing drive and the control system is responsive to the articulation controls 110160 and 110170. Stated another way, the articulation drive is engaged with the staple firing drive and the control system is responsive to the articulation controls 110160 and 110170 when the closure actuator 10140 is in a position between, and including, the open position and the pre-defined, partially-closed position. FIG. 66 illustrates a lock arm 10147 of the closure lock 10146 seated in a notch, or recess, 10145 defined in a top portion 10144 of the closure actuator 10140. The lock arm 10147 engages the notch 10145 as the closure actuator 10140 is being closed, i.e., when the closure actuator 10140 reaches the partially-closed position discussed above. In various instances, the lock arm 10147 entering the notch 10145 can make an audible click which can indicate to the clinician closing the closure actuator 10140 that any additional closure of the closure actuator 10140 will disable the articulation drive and controls. The lock arm 10147 entering into the notch 10145 can also provide a tactile feedback to the clinician. At such point, the clinician is afforded an opportunity to observe the articulated position of the end effector 110400 and the partially-closed configuration of the end effector 110400 while the closure actuator 10140 is held in position. If the clinician is unsatisfied with the position of the end effector 110400 in this instance, the clinician is afforded an opportunity to articulate the end effector 110400 once again using the articulation controls 110160 and 110170 without having to re-open the end effector 110400. Closing the closure actuator 10140 beyond this position, however, decouples the articulation drive from the staple firing drive and makes the control system unresponsive to the articulation controls 110160 and 110170. In such instances, the lock arm 10147 flexes out of engagement with the notch 10145 such that the top portion 10144 rotates past the lock arm 10147 until the closure actuator 10140 reaches the end of its stroke. At such point, referring to FIG. 67, the lock arm 10147 unflexes and falls in behind the top portion 10144 to releasably hold the closure actuator 10140 in its fully-closed position. Applying a force to the closure actuator 10140 can flex the lock arm 10147 out of the way once again so as to return the closure actuator 10140 to its above-discussed partially-closed position and/or fully-open position. When the closure actuator 10140 is returned to the partially-closed position, and/or anywhere in-between the partially-closed position and the open position, the articulation drive is re-engaged with the staple firing drive and the control system is once again responsive to the articulation controls 110160 and 110170.

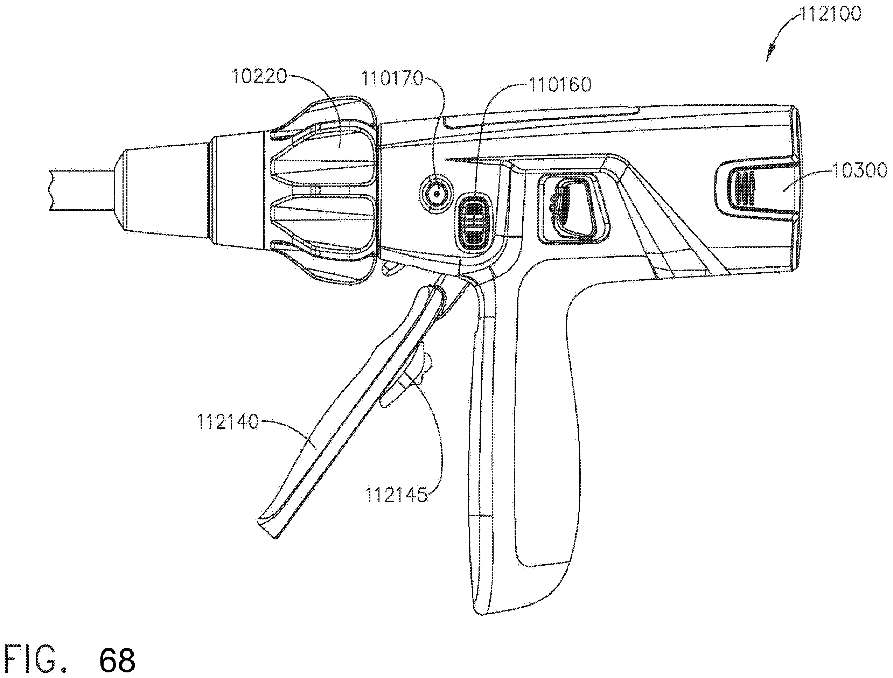

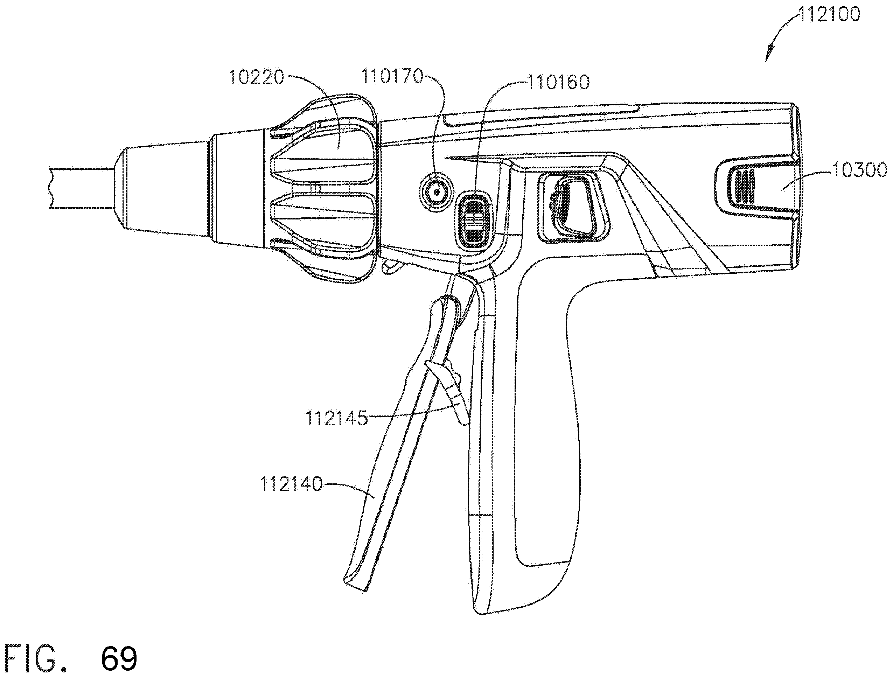

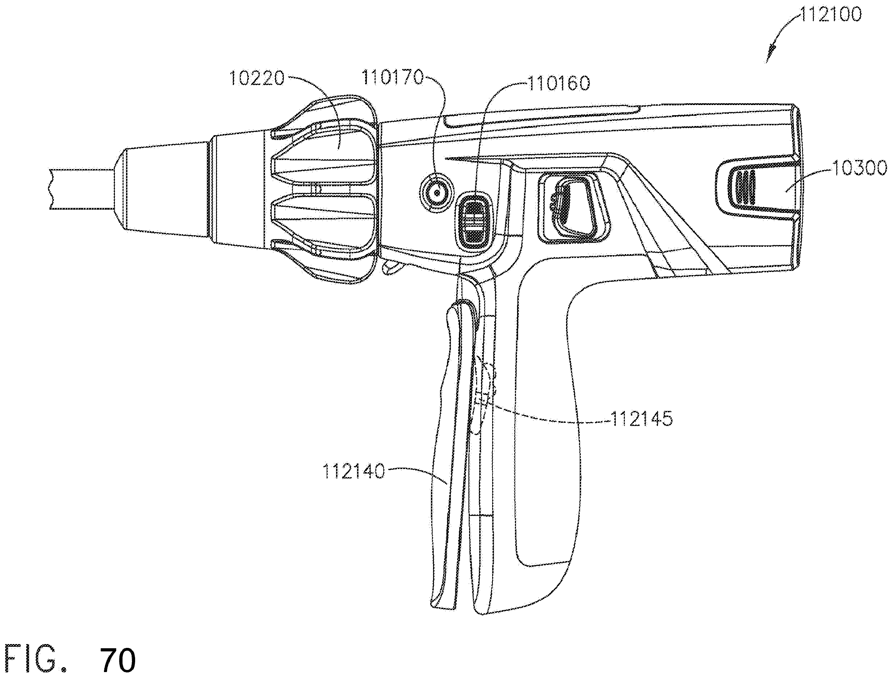

A surgical instrument including a handle 112100 is illustrated in FIGS. 68-70 which comprises a selectively actuatable closure actuator block. The handle 112100 comprises a closure actuator 112140 which, similar to the closure actuator 10140, is rotated from a fully-open position (FIG. 104) to a fully-clamped position (FIG. 106) to close the end effector 110400. The closure actuator 112140 comprises a deployable block 112145 rotatably mounted thereto which is rotatable between a stowed position (FIG. 104) to a deployed position (FIG. 105) which can support the closure actuator 112140 in a partially-closed position. In this partially-closed position of the closure actuator 112140, similar to the above, the articulation drive is still operably engaged with the staple firing drive and the control system is still responsive to the articulation controls 110160 and 110170. At such point, the clinician can choose to deactivate the closure block 112145 and fully close the end effector 110400. Doing so, similar to the above, will decouple the articulation drive from the staple firing drive and make the control system non-responsive to the articulation controls 110160 and 110170. The clinician can decide whether or not to deploy the closure block 112145. If the closure block 112145 is not deployed, the closure actuator 112140 will not be stopped in its predefined, partially-closed position and the articulation drive will be deactivated as the end effector 110400 is closed. When the closure actuator 10140 is returned to the its partially-closed position, and/or anywhere in-between the partially-closed position and the open position, the articulation drive is re-engaged with the staple firing drive and the control system is once again responsive to the articulation controls 110160 and 110170. The control system comprises a sensor system configured to assess whether the closure actuator 112140 is in its open position, partially-closed position, and/or fully-closed position.

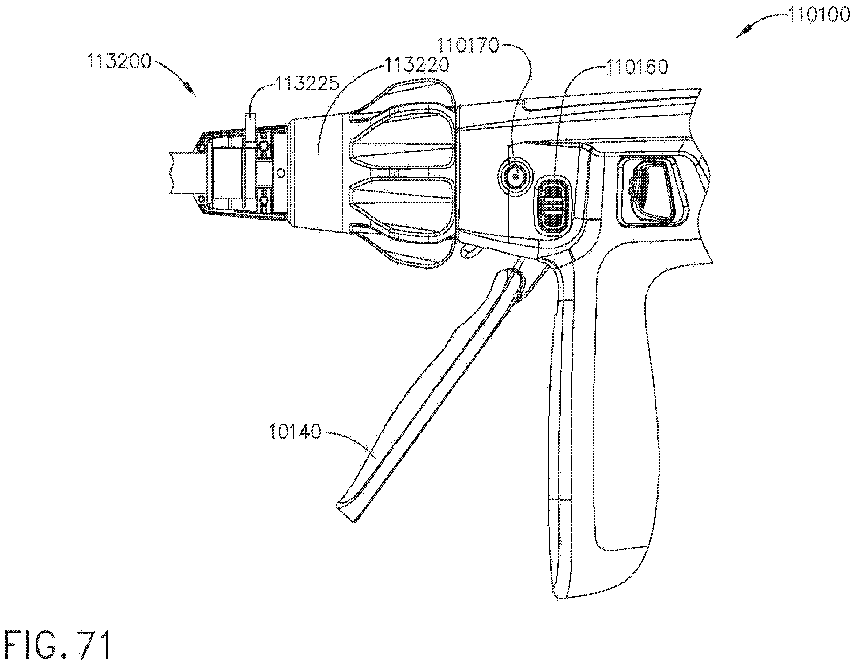

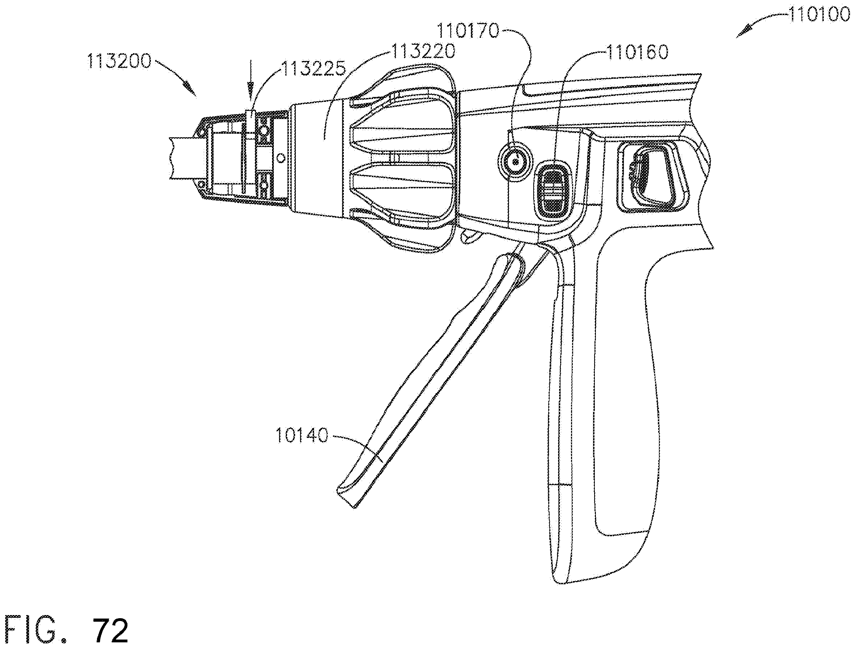

Further to the above, automatic locks and/or deployable blocks can be used separately and/or together in various embodiments. Another example is illustrated in FIGS. 71 and 72 which includes a shaft 113200 extending from a handle 110100. The shaft 113200 comprises a nozzle 113220 which is used to rotate the shaft 113200 about a longitudinal axis. The nozzle 113220 includes an actuator 113225 which is manually depressed by the clinician to block the closure drive in a state which corresponds to the above-discussed predefined, partially-closed position.

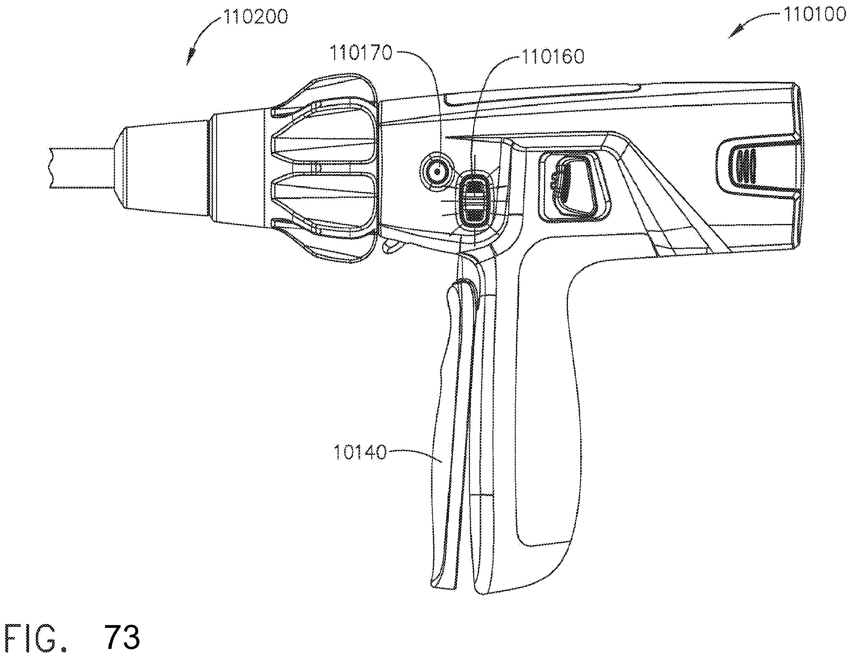

Referring to FIG. 73, the surgical instrument 110000 comprises a visual indicator which indicates that the articulation drive has been decoupled from the staple firing drive and that the control system is no longer responsive to the articulation controls 110160 and 110170. The rocker body 110163 of the articulation actuator 110160 is comprised of a translucent material, such as a translucent plastic, for example. In at least one embodiment, the rocker body 110163 is comprised of clear polycarbonate, for example. The articulation actuator 110160 further comprises a light, such as a light emitting diode (LED), for example, positioned within and/or underneath the rocker body 110163. The light is in communication with the control system of the surgical instrument 110000 and is illuminated by the control system when the articulation drive is not engaged with the staple firing drive. In such instances, the clinician is provided with visual feedback that the articulation control 110160 is no longer responsive to inputs. Similarly, the articulation control 110170 comprises a button housing comprised of a translucent material and a light in communication with the control system. Similar to the articulation control 110160, the light of the articulation control 110170 is illuminated by the control system when the articulation drive is not engaged with the staple firing drive. In various embodiments, the articulation actuator 110160 is not illuminated when the closure actuator 10140 is in within a range of positions between, and including, its fully-open position and a predetermined partially-closed position, discussed above. When the closure actuator 10140 is closed beyond the predetermined partially-closed position, the articulation actuator 110160 is illuminated - at least until the closure actuator 10140 is returned back into the predetermined partially-closed position.

In various alternative embodiments, the light of the actuator 110160, and/or the actuator 110170, is illuminated with a first color, such as green, for example, when the articulation drive is engaged with the staple firing drive and a second color, such as red, for example, when the articulation drive is not engaged with the staple firing drive. In at least one such embodiment, the light in the articulation actuator 110160 comprises a two-color LED, for example.

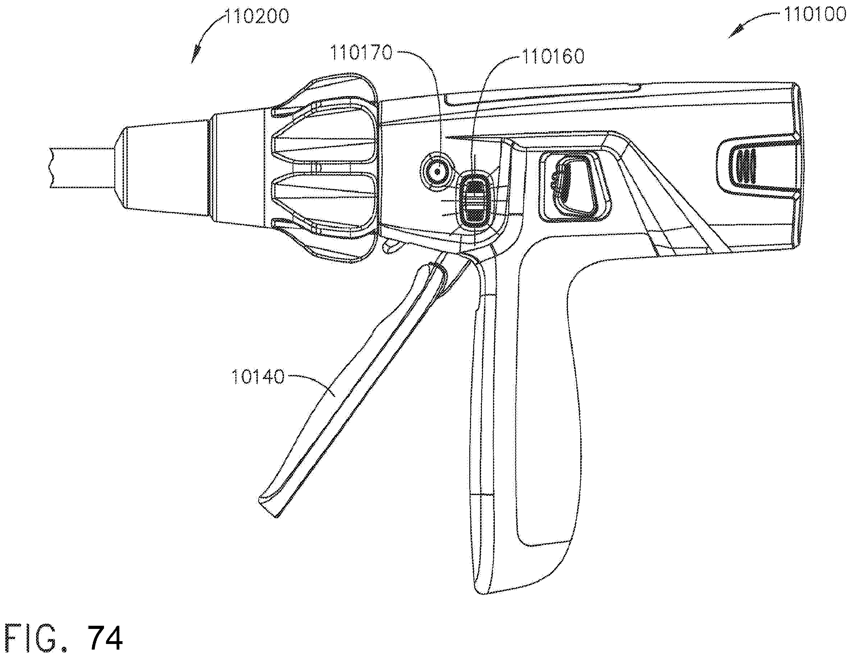

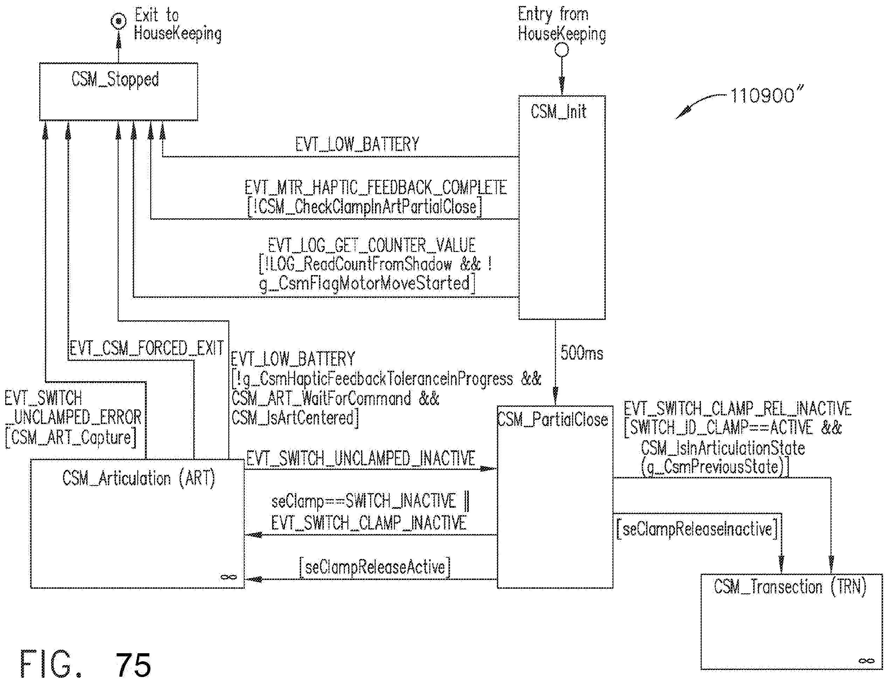

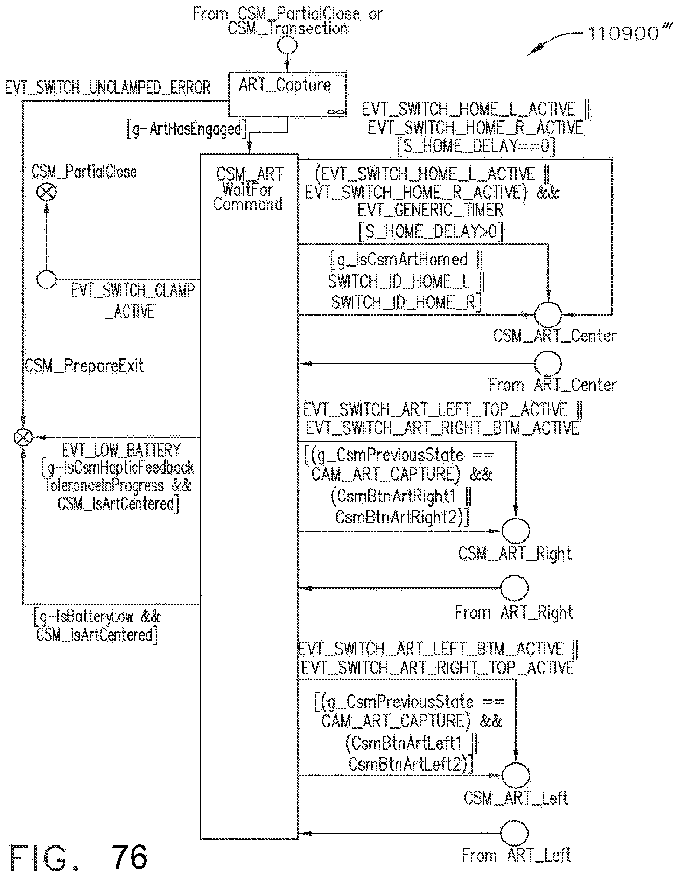

Referring to FIG. 74, the surgical instrument 110000 can comprise a visual indicator which indicates that the articulation drive is engaged with the staple firing drive and that the control system is responsive to the articulation controls 110160 and 110170. The light in the articulation control 110160 is in communication with the control system of the surgical instrument 110000 and is illuminated by the control system when the articulation drive is engaged with the staple firing drive. In such instances, the clinician is provided with visual feedback that the articulation control 110160 is responsive to inputs. Similar to the articulation control 110160, the light of the articulation control 110170 is illuminated by the control system when the articulation drive is engaged with the staple firing drive. In various embodiments, the articulation actuator 110160 is illuminated when the closure actuator 10140 is in within a range of positions between, and including, its fully-open position and a predetermined partially-closed position, discussed above. When the closure actuator 10140 is closed beyond the predetermined partially-closed position, the articulation actuator 110160 is deilluminated - at least until the closure actuator 10140 is returned back into the predetermined partially-closed position. Further details are provided in the control system schematics 110900" and 110900‴ illustrated in FIGS. 75 and 76, respectively.

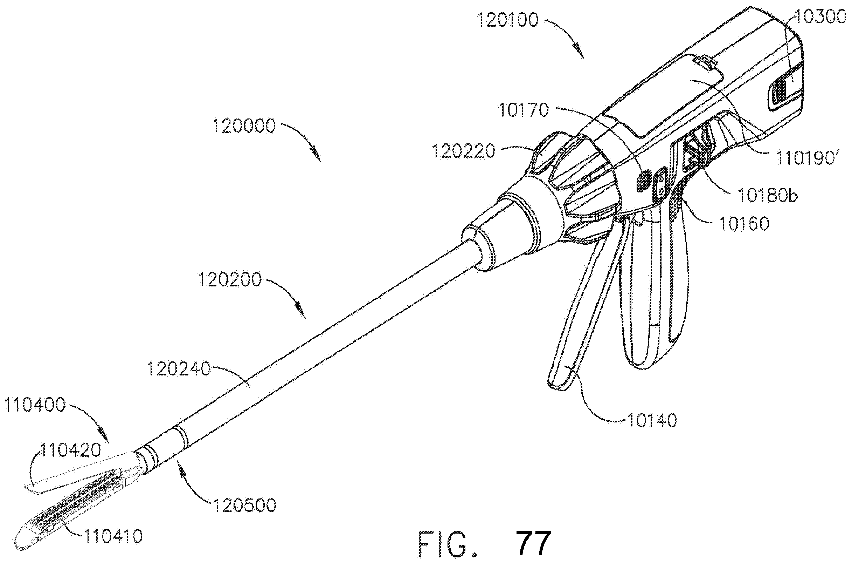

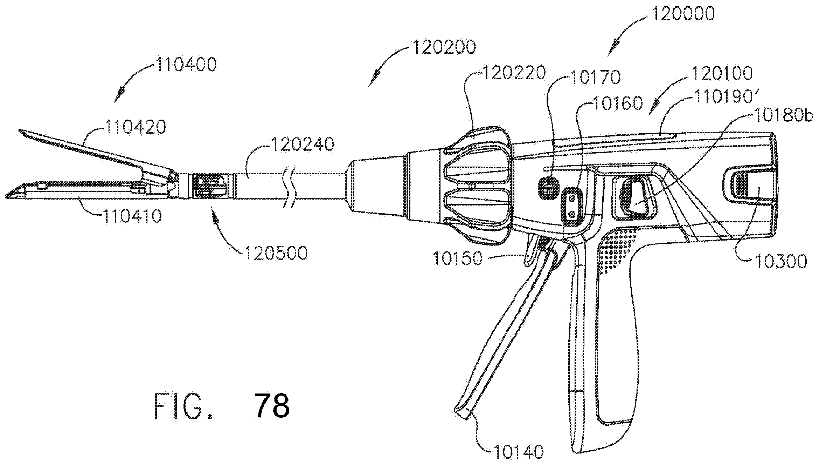

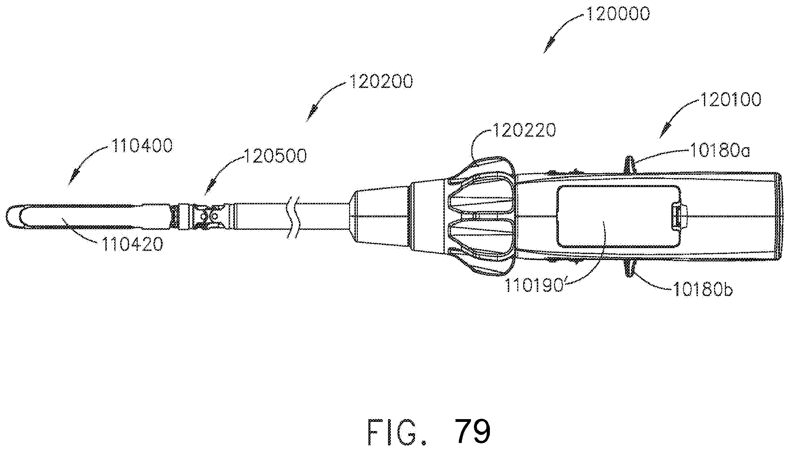

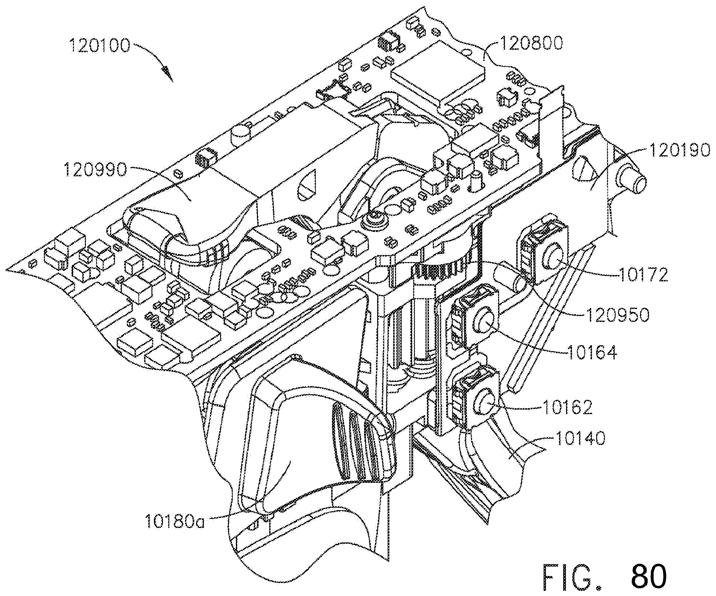

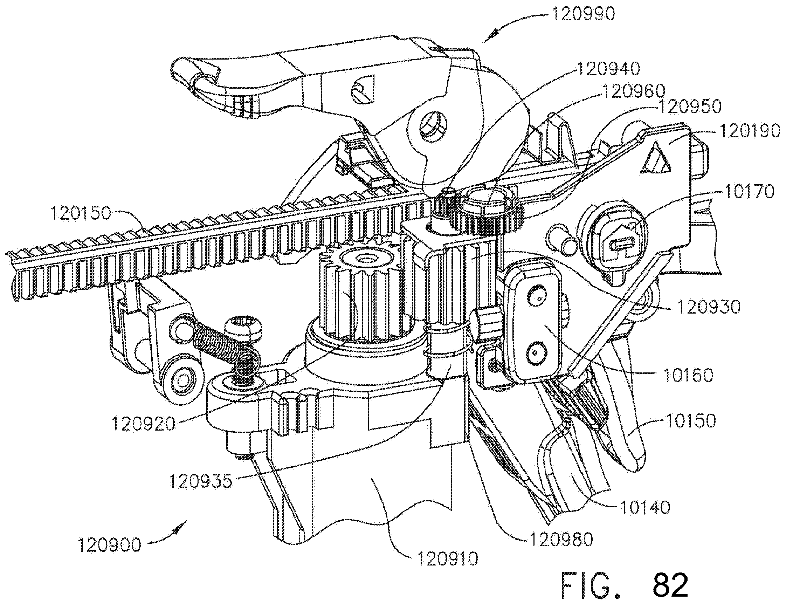

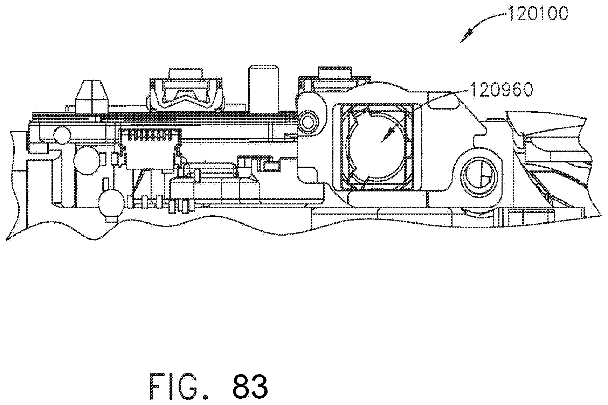

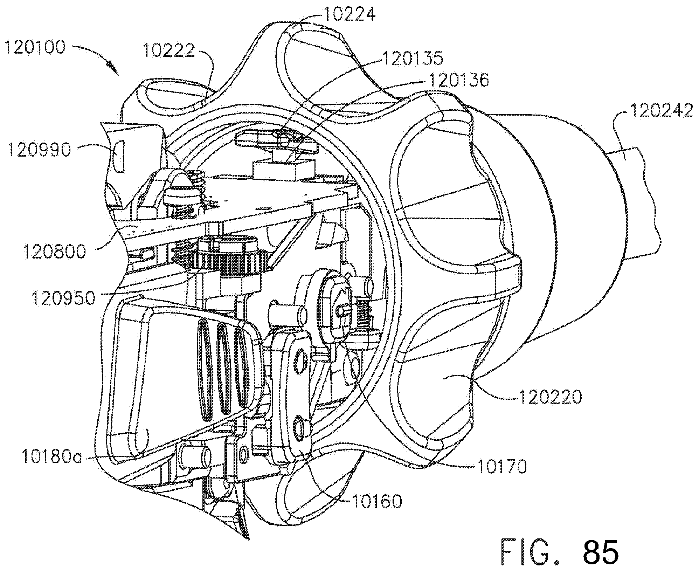

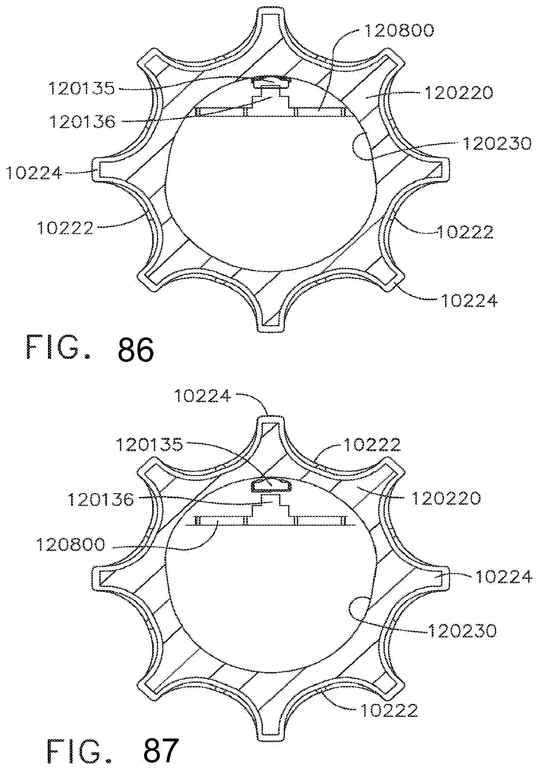

A surgical instrument 120000 is illustrated in FIGS. 77-87 and is similar to the other surgical instruments disclosed herein. The surgical instrument 120000 comprises a handle 120100, a shaft 120200 extending from the handle 120100, and an end effector 110400 rotatably coupled to the shaft 120200 about an articulation joint 120500. The handle 120100 comprises a frame, an outer housing 120110, and controls for operating the surgical instrument. Further to the above, referring to FIGS. 80-83, the handle 120100 comprises a drive system 120900 including an electric motor 120910 that is actuatable to articulate the end effector 120500, among other things.

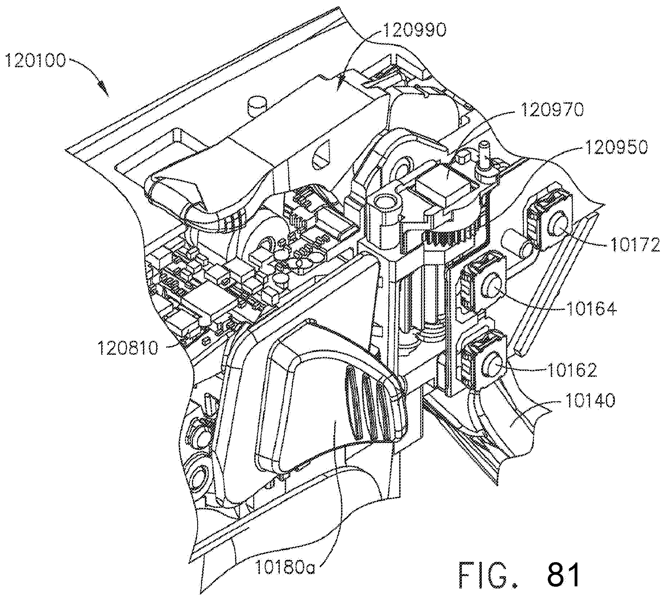

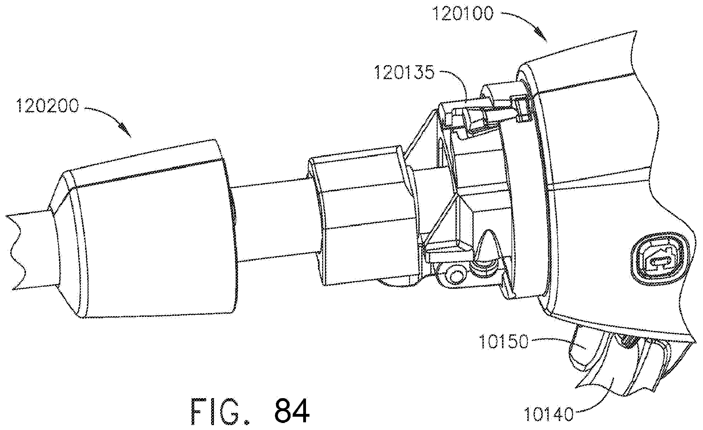

Further to the above, referring to FIGS. 80 and 81, the control system of the surgical instrument 120000 comprises a control board 120800 including a processor, microchips, memory devices, and/or circuitry, for example, which controls the operation of the surgical instrument 120000 in response to inputs from the instrument controls and/or sensors of the surgical instrument 120000. In addition to the control board 120800, the control system can comprise a sub-board 120810 in communication with the control board 120800. In at least one embodiment, the sub-board 120810 can comprise a dedicated control circuit, such as a motor control circuit, for example. Referring to FIGS. 84-87, the surgical instrument 120000 further comprises an orientation sensor, or switch, 120136 mounted to the control board 120800 and a movable switch element 120135. In at least one embodiment, the switch element 120135 comprises a pivotable lever, for example. Further to the above, the shaft 120200 is rotatable relative to the handle 120100 about a longitudinal axis through an entire rotation - 360 degrees - about the longitudinal axis that is divided into a first orientation range in which a nozzle 120220 of the shaft 120200 is in contact with the switch element 120135 and the switch 120136 is in a closed state (FIG. 182) and a second orientation range in which the nozzle 120220 is not in contact with the switch 120136 and the switch 120136 is in an open state (FIG. 183). Similar to the above, the inner surface of the nozzle 120220 comprises an inner cam profile 120230 which comprises a small diameter region for contacting the switch element 120135 when the shaft 120200 is in the first orientation range and a large diameter region which does not contact the switch element 120135 when the shaft 120200 is in the second orientation range. When the control system of the surgical instrument 120000 detects that the switch 120136 is closed, further to the above, the control system responds to control inputs in a manner which is intuitive for the anvil jaw 110420 being in an up orientation. Correspondingly, the control system responds to control inputs in a manner which is intuitive for the anvil jaw 110420 being in a down orientation when the control system detects that the switch 120136 is open. For instance, the control system can run the electric motor 120910 in the first direction when an articulate-right control is actuated and the switch 120136 is in its closed state and a second, or opposite, direction when the articulate-right control is actuated and the switch 120136 is in its open state.

In various embodiments, as discussed in greater detail herein, surgical instruments, such as surgical stapling instruments, for example, comprise a handle, a shaft, and an end effector articulatable relative to the shaft by way of articulation controls positioned on the handle and/or another portion of the surgical instrument. The articulation controls can comprise any suitable combination of buttons and/or switches, for example, which allow a user to articulate the end effector in different directions relative to the shaft. For example, a first button on the handle, when pressed, will articulate the end effector in a left direction relative to the handle and a second button, when pressed, will articulate the end effector in a right direction relative to the handle. In at least one instance, a rocker switch, for example, can be used where the first button and the second button each comprise opposite ends of the rocker switch.

In addition to the above, the shaft of the surgical instrument is also rotatable about a longitudinal axis relative to the handle. Such rotation also causes the end effector to rotate about the longitudinal axis. When the shaft of a stapling instrument is rotated 180 degrees about the longitudinal axis, the end effector can flip from an anvil-up orientation to an anvil-down orientation, for example. In such a scenario, the top jaw effectively becomes the bottom jaw. The shaft may then be rotated 180 degrees in the same or opposite direction, for example, to return the end effector to its original orientation. Regardless of the orientation of the shaft and end effector, one or both of the end effector jaws can be opened and closed to clamp tissue therebetween and the staple firing drive can be operated to fire staples from a staple cartridge seated in the cartridge jaw of the end effector.

To make using the surgical stapling instrument intuitive as possible, the articulation controls can also be flipped, or re-mapped, for example, when the end effector, and/or jaws of the end effector, are flipped. This can ensure that regardless of the orientation state of the end effector relative to the handle (anvil up/anvil down, for example), that the articulation controls always articulate the end effector in, not only the desired direction, but also the same direction relative to the handle. For example, when the anvil is in an anvil up orientation relative to the handle, a first articulation control, or button, will articulate the end effector in a right direction relative to the handle and a second articulation control, or button, will articulate the end effector in a left direction relative to the handle. When the shaft is rotated about its longitudinal axis to place the anvil in the anvil down orientation relative to the handle, the articulation controls can automatically be flipped by, for example, a control circuit. When the articulation controls are flipped, actuation of the first articulation control will still articulate the end effector in the right direction and actuation of the second articulation control will still articulate the end effector in the left direction. If the articulation controls are not flipped, or re-mapped, however, the articulation controls will articulate the end effector in the opposite direction when the end effector is upside-down (the anvil is in an anvil down position) as compared to when the end effector is right-side-up (the anvil is in the anvil up position).

Flipping, or remapping, the articulation controls on the fly as the shaft is rotated relative to the handle can be advantageous in that the user does not have to think about how the end effector originally articulated before the user rotated the end effector into an upside down orientation. The end effector will always articulate in the same direction relative to the handle. That being said, the end effector will not always be in a perfectly anvil up position or a perfectly anvil down position. Rather, the end effector may be rotated into any orientation between the anvil up position and the anvil down position. In such in-between positions, however, the anvil may be in a generally up position or a generally down position. If the anvil is in a generally up position, the control system is configured to respond to the articulation controls as if the anvil is in the anvil up position. Similarly, the control system is configured to respond to the articulation controls as if the anvil is the anvil down position if the anvil is in a generally down position. When the end effector is rotated right or left into, or near, a 90 degree position, however, it may be difficult, absent more, for the user to visibly discern whether or not the anvil is in a generally up or a generally down position and ascertain how the end effector may respond to the articulation controls.

In at least one instance, providing visual feedback of the flipping, or remapping, of articulation controls can help alert a user that the controls have been flipped, or, are about to be flipped, when the end effector rotation reaches a threshold orientation. The threshold orientation can comprise a scenario where, owing to shaft rotation, the end effector has been rotated in 90 degrees about the longitudinal shaft axis in either direction. Both of these orientations involve the anvil jaw being pivotable side-to-side to clamp tissue. It is at both of these orientations where end effector articulation can become completely vertical. This may cause some confusion as to whether or not a 'right articulation control' would articulate the end effector up or down and whether or not a 'left articulation control' would articulate the end effector up or down. It is also at these orientations where the articulation controls would be flipped at, and/or, upon passing these orientations.

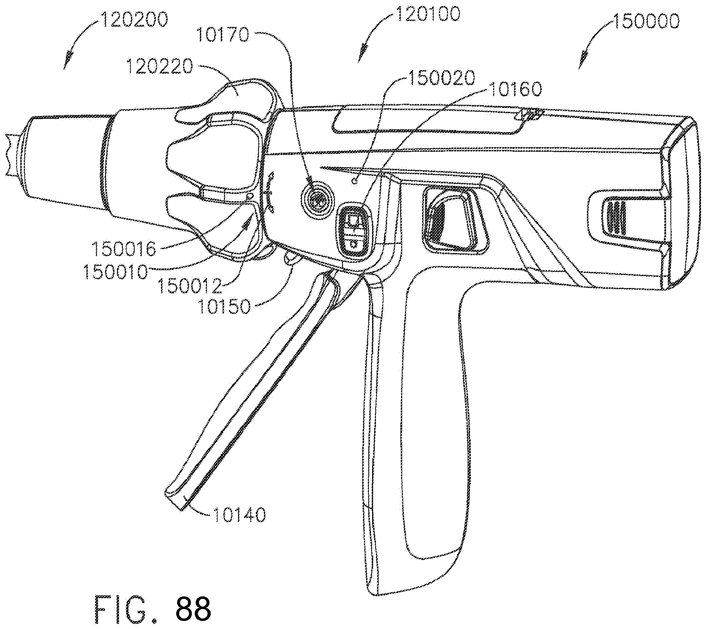

FIGS. 88-90 depict a surgical stapling instrument 150000 which is similar in many respects to the surgical stapling instruments and assemblies described herein. The surgical stapling instrument 150000 comprises a visual indicator 150010 configured to show a user of the surgical stapling instrument 150000 when the shaft 120200, and thus the end effector, reach and/or pass an orientation threshold which causes the articulation actuator, or controls, 10160 to be flipped, or remapped. The visual indicator 150010 comprises a mark 150016 defined on a rib of the nozzle 120220 and a threshold mark 150012 defined on the handle 120100. The mark 150016 can comprise any suitable pattern or visual. In various instances, the mark 150016 comprises an indent, a raised surface, a painted surface, and/or a different texture surface, for example. In at least one instance, the mark 150016 is detectable by a user when the user touches the mark 150016. In various instances, the mark 150012 comprises an indent, a raised surface, a painted surface, and/or a different texture surface, for example.

Further to the above, the mark 150012 is affixed to the handle and is stationary during use of the instrument 150000 while the mark 150016 rotates with the shaft 120200 and the nozzle 120220. As the nozzle 120200 is rotated through a range of rotational motion, by a motor and/or manually by a user, for example, the mark 150016 can approach, reach, and then pass by the mark 150012. The mark 150012 indicates a threshold orientation where, when the mark 150016 on the nozzle 120220 passes the mark 150016 on the handle 120100, the articulation controls 10160 are flipped, or re-mapped. In at least one instance, the articulation controls 10160 comprise a rocker switch where the top switch articulates right and the bottom switch articulates left. When the threshold orientation is crossed, the user will know, based on seeing that the mark 150016 has passed the mark 150012 that the articulation controls are flipped, or remapped, and that the control system has compensated for the flipped orientation of the shaft and end effector. When the mark 150016 passes the mark 150012, as a result, the top switch still articulates the end effector to the right and the bottom switch still articulates the end effector to the left.

In various embodiments, the surgical stapling instrument 150000 further comprises an indicator light 150020 in communication with a control circuit of the surgical stapling instrument 150000 that is configured to alert a user when the output of the articulation controls 10160 has been adjusted. In at least one embodiment, the control circuit comprises a processor including one or more input gates and one or more output gates. The control circuit further comprises one or more sensors in communication with the input gates of the processor which deliver a voltage potential or signal to the processor that the shaft and end effector have reached a flipped orientation. Such sensors are disclosed throughout the Subject Application. The indicator light 150020 comprises a light emitting diode (LED), but could comprise any suitable indicator light. When illuminated, the indicator light 150020 emits one color and, when not illuminated, the indicator light 150020 does not emit a color. The indicator light 150020 is in communication with at least one of the processor output gates and is illuminated by the processor when the processor receives an input signal that the shaft and end effector have reached a flipped orientation. If the light 150020 is not on, the user will know that the output response of the articulation actuator 10160 is not flipped. In at least one alternative embodiment, the indicator light 150020 comprises a multicolor LED that emits one color when the indicator light 150020 receives a low, or off, voltage signal from the processor and emits a different color when the indicator light 150020 receives a high, or on, voltage signal from the processor.

In at least one instance, the indicator light 150020 alerts a user when the articulation actuator 10160 is about to be flipped and/or has been flipped. In at least one instance, the indicator light 150020 is illuminated by the processor of the control circuit in a pulsing, or flashing, manner when the shaft 120200 is in an orientation just before the threshold orientation indicating that the output response of the articulation actuators has not yet been flipped but may become flipped if the shaft and end effector are rotated further. The indicator light 150020 is illuminated by the processor in a constant manner when the shaft 120200 is at the threshold orientation and past the threshold orientation indicating that the output response of the control circuit has been switched. The processor does not illuminate the indicator light 150020 when the shaft and end effector are not near the threshold orientation and the output response of the articulation actuators has not been flipped. In at least one instance, the pulsing is configured to gradually change speed based on how close the shaft 120200 is to the orientation threshold. For example, the closer the shaft 120200 is to the orientation threshold, the faster the indicator light 150020 will flash.

Further to the above, the indicator light 150020 is configured to be illuminated with different colors corresponding to the different states of the orientation of the shaft 120200 relative to the handle 120100. For example, the light 150020 can be illuminated green indicating that the shaft 120200 is not near the threshold orientation and that the output response to the articulation controls has not been changed. The light 150020 can be changed to amber when the shaft 120200 starts approaching the threshold orientation and the output response to the articulation controls has not been changed. The light 150020 can be changed to red when the output response to the articulation controls has been changed. In at least one embodiment, the light 150020 is pulsated red when the shaft 120200 passes the threshold orientation. In at least one embodiment, the light 150020 is pulsated amber when the shaft 120200 gets further from the threshold orientation.

In at least one instance, auditory feedback is used in addition to or in lieu of visual indicators and/or lights, for example, to indicate that the rotation of the shaft is near, at, and/or beyond the threshold orientation. For example, a speaker in communication with the processor can be employed to audibly alert a user with pulsating sounds, different sounds, sounds varying in volume and/or rhythm, and/or sounds comprising spoken information, for example.

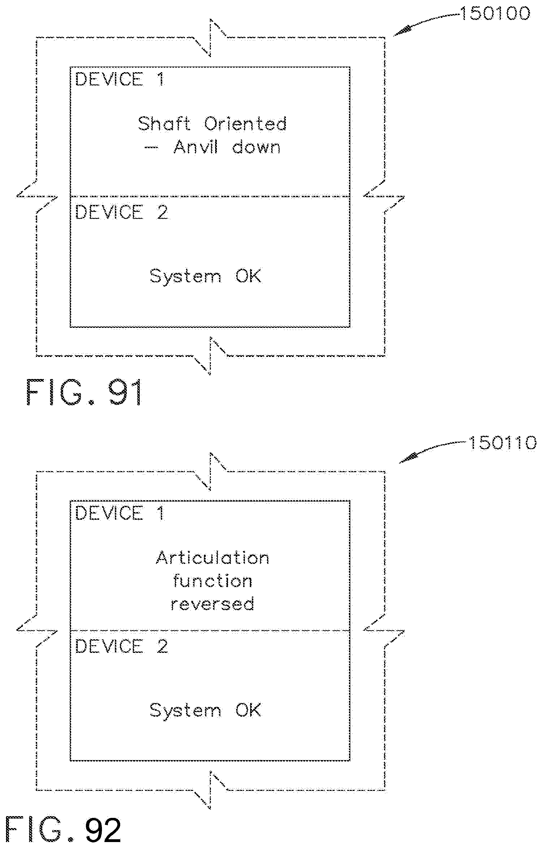

In various instances, various types of surgical instruments are used with a surgical hub in a surgical operating suite. Some surgical hubs are disclosed in U.S. Patent Application Publication No. 2019/0201141 A1. In at least one embodiment, a surgical instrument, such as the stapling instrument 150000, for example, comprises a wireless signal transmitter that transmits data regarding the stapling instrument 150000 to the surgical hub. In various instances, the surgical hub comprises at least one display screen visible to one or more clinicians in the surgical operating suite. The display screen may comprise a variety of information comprising instrument status, patient data, and/or operational procedural data, for example. FIGS. 91 and 92 each depict a display option 150100 and 150110 where the display can show the status of various devices connected to the hub. This information can include the orientation state of the end effector relative to the handle, for example. In at least one instance, the display is configured to alert a user of the current status regarding the orientation state of the end effector relative to the handle and whether or not the articulation controls are flipped. For example, the display 150100 shows that the connected surgical instrument, by way of wireless communication, for example, is in an anvil down orientation and that the articulation actuator has been flipped, or reversed. The display 150100 can be used in addition to or in lieu of the indicator light 150020. In various embodiments, the display 150100 and the indicator light 150020 are co-ordinated and/or updated simultaneously to show the change, or lack of change, in the output response of the control system to the control actuators. In various embodiments, the information displayed on the handle of the surgical instrument is also displayed on the hub display 150100. In at least one such embodiment, the manner in which the information is displayed on the handle is the manner in which the data is displayed on the hub display 150100.

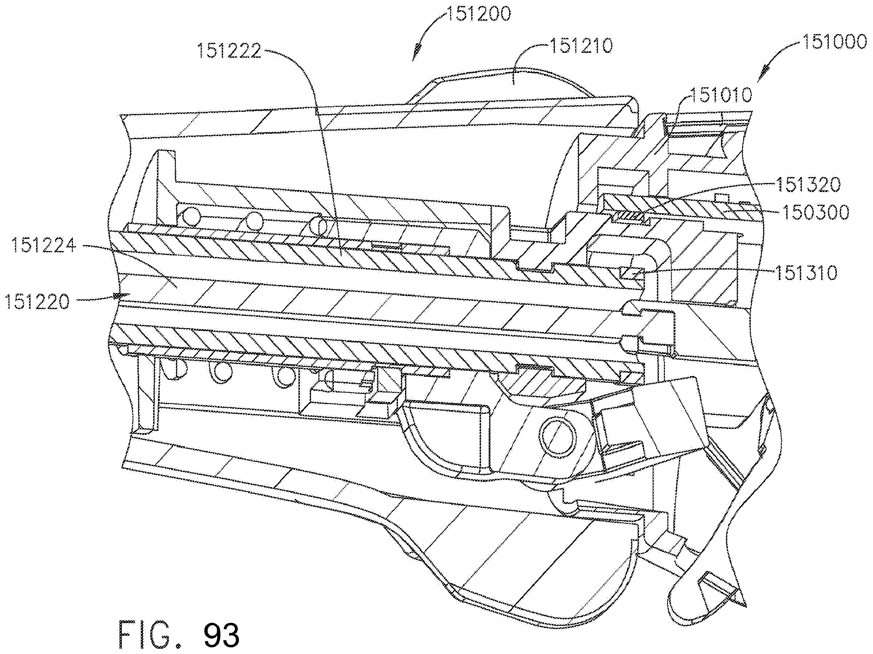

As discussed above, sensors and/or switches are used in conjunction with one or more components of a surgical stapling instrument to determine the orientation of the shaft relative to the handle. FIG. 93 depicts a surgical stapling assembly 151000 comprising a handle 151010 and a shaft assembly 151200 rotatable relative to the handle 151010. The shaft assembly 151200 comprises a proximal nozzle 151210 and a plurality of shafts 151220 extending longitudinally through the shaft assembly 151200 along a longitudinal axis toward an end effector extending from the shaft assembly 151200. The shafts 151220 comprise a firing rod 151220 and a frame, or channel retainer, 151222. All of the components of the shaft assembly 151200, and the end effector, are rotatable together relative to the handle 151010 about the longitudinal axis. The stapling assembly 151000 further comprises a sensing, or detecting, system configured to monitor the orientation of the shaft assembly 151200 relative to the handle 151010. Data from the sensing system is fed to a control circuit of the surgical stapling assembly 151000, and/or the surgical hub, constantly or at predetermined sample rate that the control circuit uses to assess whether or not to change the output response of the articulation controls of the stapling assembly 151000. The sensing system comprises a magnetic element, such as magnet 151310, for example, mounted on the frame 151222 and a Hall Effect sensor 151320 mounted directly on a printed circuit board 151300 positioned within the handle 151010. As discussed further below, the Hall Effect sensor 151320 is in communication with the control circuit of the stapling assembly 151000. In addition to or in lieu of the Hall Effect sensor 151320, a light sensor, a proximity sensor, an infrared sensor, a pressure sensor, a position sensor, a force sensor, and/or a piezo sensor, for example, can be utilized.

As the shaft assembly 151200 is rotated relative to the handle 151010, the magnet 151310 rotates with the shaft assembly 151200. This rotation of the magnet 151310 is detectable by the Hall Effect sensor 151320 which produces a different voltage output for each rotated orientation of the shaft assembly 151200. This information can be utilized by a control circuit to automatically switch, or remap, articulation controls positioned on the handle when the shaft assembly 151200 has reached or crossed an orientation threshold. In various instances, two or more magnets can be used so as to provide a discrete output that is uniquely paired with a discrete orientation of the shaft assembly 151200.

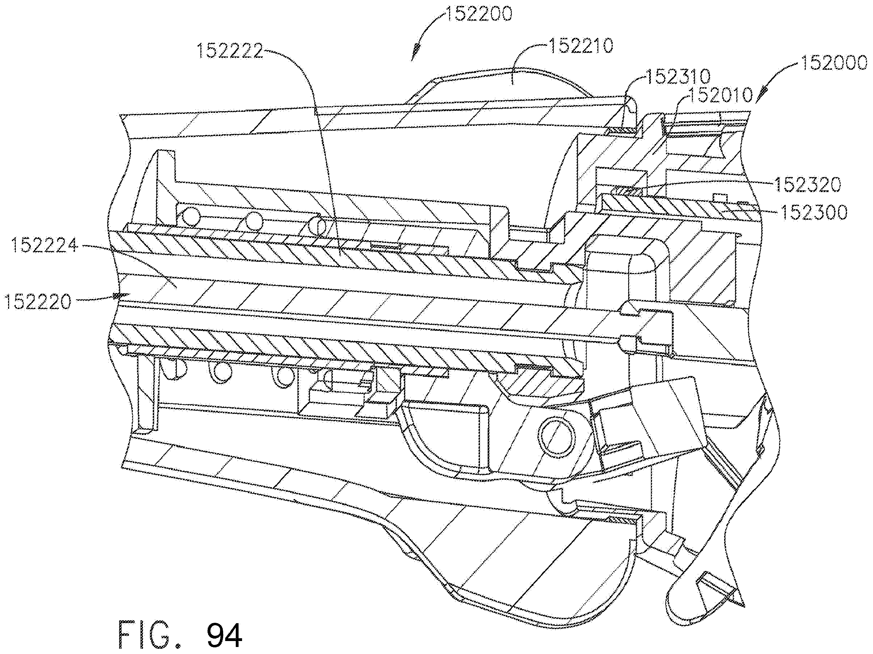

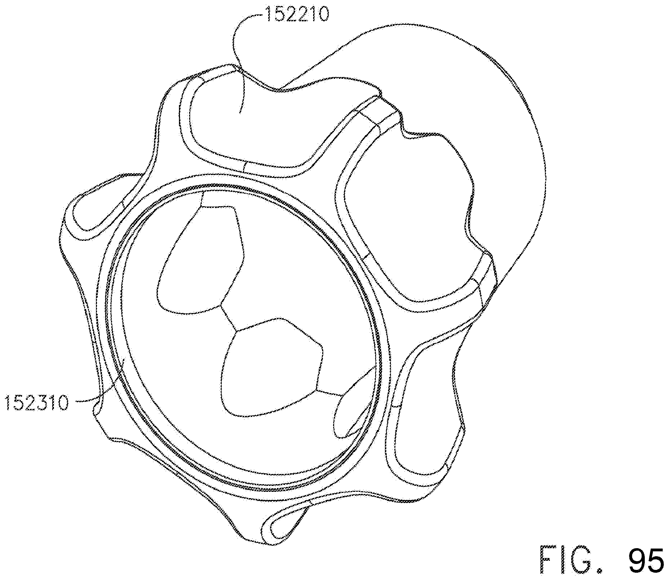

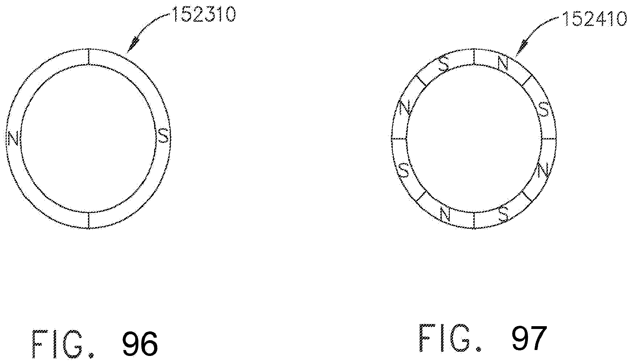

FIGS. 94-96 depict a surgical stapling assembly 152000 which is similar in many respects to various other surgical stapling assemblies disclosed herein, such as the surgical stapling assembly 151000 depicted in FIG. 93, for example. The stapling assembly 152000 comprises a handle 152010 and a shaft assembly 152200 rotatable relative to the handle 152010. The shaft assembly 152200 comprises a proximal nozzle 152210 and a plurality of shafts 152220 extending through the handle 152010 and the shaft assembly 152200 toward an end effector. The shafts 152220 comprise a firing rod 152220 and a frame, or channel retainer, 152222. Because the end effector is rotatable about a longitudinal shaft axis, all of the components of the shaft assembly 152200 are rotatable relative to the handle 152010. The surgical stapling assembly 152000 further comprises a sensing system comprising a magnet ring 152310 mounted within the nozzle 152210 and a Hall Effect sensor 152320 mounted directly on a printed circuit board 152300 positioned within the handle 152010. As can be seen in FIG. 96, the magnet ring 152310 is divided into two halves. One half of the magnet ring 152310 comprises a first polarity and the other half of the magnet ring 152310 comprises a second polarity opposite to the first polarity. The differing polarities can be aligned within the shaft assembly such that the Hall Effect sensor can detect when the shaft assembly 152200 passes the threshold orientation. One polarity can indicate the anvil up position and the other polarity can indicate the anvil down position. This information can be fed to a control circuit of the stapling assembly 152000 to automatically remap the articulation controls of the stapling assembly 152000 and/or alert a user of the switched output of the articulation controls. FIG. 97 depicts an alternative magnet ring 152410 which uses a plurality of alternating polarities along the circumference of the magnet ring 152410. In at least one instance, the alternating polarities are monitored by a control circuit with the Hall Effect sensor. Utilizing a magnet and a Hall Effect sensor in the manner discussed above can eliminate the resistance caused when physically contacting a switch element, for example, and increase the longevity the stapling assembly.

In various embodiments, a surgical stapling assembly comprises a handle, a shaft extending from the handle, and an end effector attached to the shaft by way of an articulation joint. The end effector is articulatable relative to the shaft by way of an articulation drive that is controlled by articulation controls, or actuators, positioned on the handle, for example, that are used to orient the end effector within a patient when the end effector is in an open, or unclamped, configuration. As discussed in greater detail herein, the surgical stapling assembly comprises a staple firing drive that is used to drive the articulation drive when the articulation drive system is coupled to the staple firing drive. The stapling assembly further comprises a closure drive configured to move the end effector from its open configuration toward a fully-clamped configuration. More specifically, the closure drive, when actuated, moves a movable jaw of the end effector from an unclamped position to a fully-clamped position. The stapling assembly comprises a clutch which decouples the articulation drive from the firing drive when the movable jaw passes a partially-closed threshold position. Until the movable jaw reaches the threshold position, however, the end effector can be articulated while partially closed. Such an arrangement can permit the end effector to be articulated while in a partially closed position yet prevent a user from articulating the end effector when the end effector is fully clamped onto the patient tissue. Moreover, allowing a user to articulate a partially clamped end effector can be helpful when an end effector with a wide jaw aperture is being used as such end effectors may be more difficult to maneuver in tight spaces. However, absent more, a user may not know when the end effector has passed the partial closure threshold position and is no longer capable of being articulated.

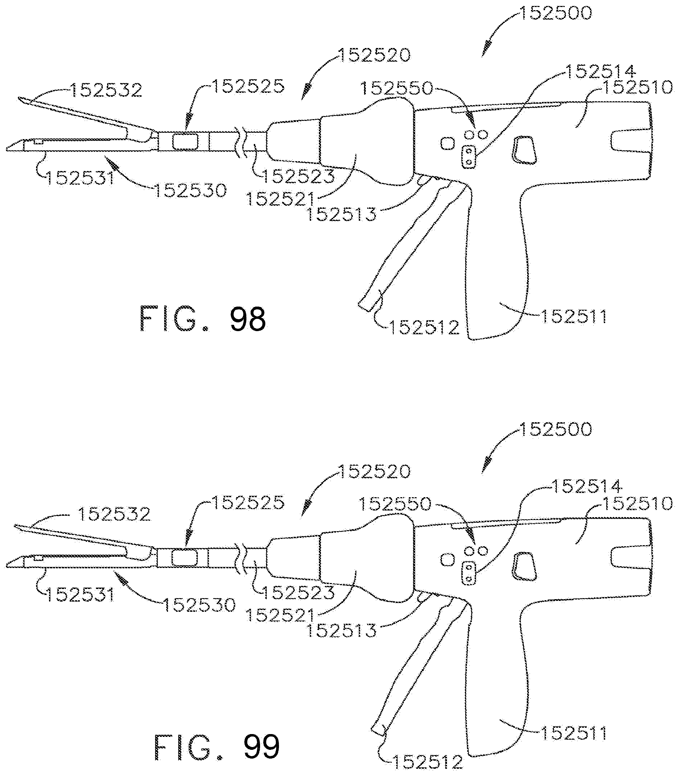

FIGS. 98-101 depict a surgical stapling assembly 152500 comprising a handle 152510, a shaft assembly 152520 extending from the handle 152510, and an end effector 152530 articulatable relative to the shaft assembly 152520 by way of an articulation joint 152525. The handle 152510 comprises articulation controls 152514 configured to be actuated by a user of the stapling assembly 152500 to articulate the end effector 152530. The shaft assembly 152520 comprises a nozzle 152521 and an elongate shaft portion 152523. The end effector 152530 comprises a cartridge channel jaw 152531 and an anvil jaw 152532 movable relative to the cartridge channel jaw 152531 between a fully-unclamped position (FIG. 204) and a fully-clamped position (FIG. 207). The handle 152510 further comprises a pistol grip portion 152511, a closure trigger 152512 configured to be actuated by a user of the stapling assembly 152500 to move the anvil jaw 152532 from the fully-unclamped position (FIG. 204) to the fully-clamped position (FIG. 207), and a firing trigger 152513 that is actuatable to fire the end effector 152530. In various alternative embodiments, the cartridge channel jaw 152531 is movable relative to the anvil jaw 152532 and the closure trigger is actuatable to move the cartridge channel jaw 152531 from a fully-unclamped position to a fully-clamped position.

The surgical stapling assembly 152500 further comprises a visual indicator 152550 configured to indicate whether or not the end effector 152530 is still articulatable based on the closure state of the end effector 152530. In various embodiments, the visual indicator 152550 comprises an LED in electrical communication with an output gate of a control circuit processor that is illuminated when the end effector is articulatable, i.e., that the end effector has not been closed beyond the partial closure threshold position. When the control circuit processor detects that the end effector has been closed beyond the partial closure threshold position, the control circuit processor de-illuminates the visual indicator 152550 to indicate that the end effector 152530 can't be articulated. As discussed further below, the control circuit comprises at least one sensor in communication with an input gate of the control circuit processor that provides data to the control circuit processor regarding the closure state of the end effector.

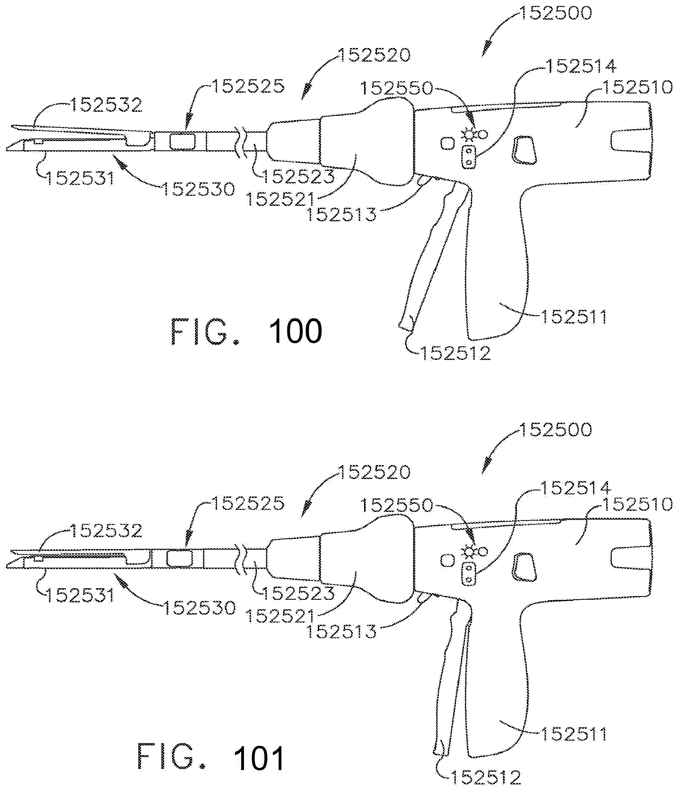

In various alternative embodiments, the LED of the visual indicator 152550 is not illuminated when the end effector 152530 is articulatable and is illuminated when the end effector 152530 is not articulatable. In at least one such embodiment, the control circuit processor flashes the visual indicator 152550 when the clutch decouples the articulation drive from the staple firing drive which disables the user's ability to articulate the end effector 152530. FIG. 98 illustrates the anvil jaw 152532 in a fully open, or unclamped, position and the visual indicator 152550 in an unilluminated condition. FIG. 99 illustrates the anvil jaw 152532 in a first partially-clamped position and the visual indicator 152550 in an unilluminated condition. FIG. 100 illustrates the anvil jaw 152532 in a second partially-clamped position. The second partially-clamped position comprises the partially-clamped threshold position and, thus, the visual indicator 152550 is activated. FIG. 101 illustrates the anvil jaw 152532 in a fully-clamped position and the visual indicator 152550 in an activated condition. Consistent with the deactivation of the visual indicator 152500 in FIGS. 98 and 99, the end effector 152530 is articulatable in the conditions illustrated in FIGS. 98 and 99. The end effector 152530 is not articulatable in the positions illustrated in FIGS. 100 and 101. This inability to articulate the end effector 152530 is indicated by the activation of the visual indicator 152550.

In various embodiments, the visual indicator 152550 comprises two or more lights. In at least one embodiment, one light of the visual indicator 152550 is illuminated green if the end effector 152530 is still capable of being articulated and the anvil jaw 152532 has not been closed past the partially-clamped threshold position. Another light of the visual indicator 152550 will illuminate a different color, such as red, for example, when the anvil jaw 152532 reaches and/or passes the partially-clamped threshold position indicating that the end effector 152530 is no longer capable of being articulated. In at least one instance, one light of the visual indicator 152550 will pulsate with a gradually changing frequency corresponding to the position of the anvil jaw 152532. For example, a light of the visual indicator 152550 will blink slowly when the anvil jaw 152532 is fully open. As the anvil jaw 152532 approaches the partially-clamped threshold position, the light of the visual indicator 152550 will pulsate faster and faster and, when the anvil jaw 152532 reaches and/or passes the partially-clamped threshold position, the light of the visual indicator 152550 will stop flashing and remain solidly illuminated. In at least one instance, the light of the visual indicator 152550 will change colors when the anvil jaw 152532 passes the partially-clamped threshold position.

While the anvil jaw 152532 is in the fully-clamped position, in at least one embodiment, the light of the visual indicator 152550 may blink red slowly indicating that the end effector 152530 is far from being able to be articulated. As the anvil jaw 152532 is opened from the fully-clamped position, the slow-pulsating red light pulses faster and faster as the anvil jaw 152532 approaches the partially-clamped threshold position. The light flips to solid green just after passing the partially-unclamped threshold position.

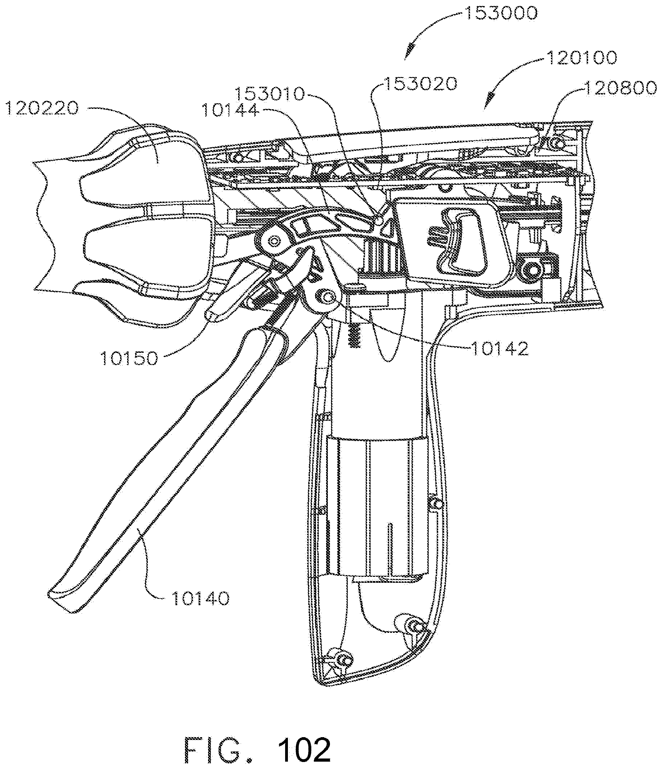

FIG. 102 depicts a handle 153000 of a surgical stapling instrument which is similar in many respects to the surgical instrument handles disclosed herein. The handle 153000 comprises a sensor 153020 and a sensor input device 153010 detectable by the sensor 153020. In at least one instance, the sensor 153020 comprises a Hall Effect sensor and the sensor input device 153010 comprises a magnet, although any suitable arrangement could be used. The magnet 153010 is mounted on the top portion 10144 of the closure trigger 10140. The Hall Effect sensor 153020 is mounted directly on the control board 120800 and is in communication with the control circuit of the surgical stapling instrument. The Hall Effect sensor 153020 is configured to detect the position of the magnet 153010 and, thus, detect the rotational position of the closure trigger 10140. Given that the rotational position of the anvil jaw 152532 is linked to the rotational position of the closure trigger 10140, the control circuit can use the detected rotational position of the closure trigger 10140 as a proxy for the rotational position of the anvil jaw 152532. In such embodiments, a sensor down in the end effector 152530, and the signal path back to the handle 153000, can be avoided.

The surgical instrument systems described herein are motivated by an electric motor; however, the surgical instrument systems described herein can be motivated in any suitable manner. In certain instances, the motors disclosed herein may comprise a portion or portions of a robotically controlled system. U.S. Patent No. 9,072,535, for example, discloses several examples of a robotic surgical instrument system in greater detail. The disclosures of International Patent Publication No. WO 2017/083125, International Patent Publication No. WO 2017/083126, International Patent Publication No. WO 2015/153642, U.S. Patent Application Publication No. 2017/0265954, U.S. Patent Application Publication No. 2017/0265865, and U.S. Patent Publication No. 2017/0290586are relevant.

The surgical instrument systems described herein have been described in connection with the deployment and deformation of staples; however, the embodiments described herein are not so limited. Various embodiments are envisioned which deploy fasteners other than staples, such as clamps or tacks, for example. Moreover, various embodiments are envisioned which utilize any suitable means for sealing tissue. For instance, an end effector in accordance with various embodiments can comprise electrodes configured to heat and seal the tissue. Also, for instance, an end effector in accordance with certain embodiments can apply vibrational energy to seal the tissue.

Although various devices have been described herein in connection with certain embodiments, modifications and variations to those embodiments may be implemented. Particular features, structures, or characteristics may be combined in any suitable manner in one or more embodiments. Thus, the particular features, structures, or characteristics illustrated or described in connection with one embodiment may be combined in whole or in part, with the features, structures or characteristics of one ore more other embodiments without limitation. Also, where materials are disclosed for certain components, other materials may be used. Furthermore, according to various embodiments, a single component may be replaced by multiple components, and multiple components may be replaced by a single component, to perform a given function or functions. The foregoing description and following claims are intended to cover all such modification and variations.

The devices disclosed herein can be designed to be disposed of after a single use, or they can be designed to be used multiple times. In either case, however, a device can be reconditioned for reuse after at least one use. Reconditioning can include any combination of the steps including, but not limited to, the disassembly of the device, followed by cleaning or replacement of particular pieces of the device, and subsequent reassembly of the device. In particular, a reconditioning facility and/or surgical team can disassemble a device and, after cleaning and/or replacing particular parts of the device, the device can be reassembled for subsequent use. Those skilled in the art will appreciate that reconditioning of a device can utilize a variety of techniques for disassembly, cleaning/replacement, and reassembly. Use of such techniques, and the resulting reconditioned device, are all within the scope of the present application.

The devices disclosed herein may be processed before surgery. First, a new or used instrument may be obtained and, when necessary, cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, and/or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta radiation, gamma radiation, ethylene oxide, plasma peroxide, and/or steam.

## Claims

1. A surgical instrument assembly (150000), comprising:
a handle (120100) comprising articulation control actuators (10160);
a shaft assembly (120200) rotatable relative to said handle (120100), wherein said shaft assembly (120200) comprises:
an elongate shaft;
an articulation joint (120500); and
an end effector (110400) articulatable relative to said elongate shaft by way of said articulation joint; and
a control circuit configured to:
identify an orientation state of said shaft assembly (120200) relative to said handle (120100);
map an output of said control circuit in response to an input from said articulation control actuators (10160) based on said identified orientation state; and
alert a user as to how said output has been mapped;
wherein said handle (120100) comprises an indicator light (150020) in communication with said control circuit that is illuminated by said control circuit corresponding to how said output has been mapped, **characterized in that** either:
said indicator light (150020) is configured to flash with a gradually changing speed where, the closer said control circuit is to re-mapping said output, the faster said indicator light (150020) flashes; or
said control circuit is configured to illuminate said indicator light (150020) with different colors corresponding to how close said control circuit is to re-mapping said output.

2. The surgical instrument assembly of Claim 1, further comprising a sensor (151320) configured to detect a change in the orientation state of said shaft assembly (120200).

3. The surgical instrument assembly of Claim 1, wherein alerting a user as to how the output of said control circuit is mapped to inputs from said articulation control actuators (10160) comprises displaying a state of said articulation control actuators (10160) on a surgical hub display.

4. The surgical instrument assembly of claim 1, wherein:
said handle (120100) comprises a first lateral side and a second lateral side opposite said first lateral side;
said articulation control actuators (10160) comprise a first articulation control actuator and a second articulation control actuator in communication with said control circuit;
said shaft assembly (120200) is rotatable into a plurality of orientation states;
wherein said control circuit is further configured such that:
being configured to map the output of said control circuit comprises being configured to automatically switch the output response of said control circuit to coincide with the identified orientation state such that said end effector (110400) is always articulated toward said first lateral side of said handle (120100) when said first articulation actuator (10160) is actuated and always articulated toward said second lateral side of said handle (120100) when said second articulation actuator (10160) is actuated regardless of the orientation state of said shaft assembly (120200) relative to said handle (120100); and
being configured to alert a user as to how said output has been mapped comprises being configured to alert said user of said surgical instrument that the output response of said control circuit has been switched to compensate for the identified orientation state of said shaft assembly (120200).

5. The surgical instrument assembly of Claim 4, wherein said control circuit is configured to illuminate said indicator light (150020) when the output response of said control circuit has been switched to compensate for the identified orientation state of said shaft assembly (120200).

6. The surgical instrument assembly of Claim 5, further comprising a sensor in communication with said control circuit to detect when said shaft assembly (120200) is:
at a threshold orientation in which the output response of said control circuit is switched;
in an orientation just before said threshold orientation; and
in an orientation just past said threshold orientation.

7. The surgical instrument assembly of Claim 6 when said indicator light (150020) is configured to flash with a gradually changing speed, wherein said control circuit is configured to:
Illuminate said indicator light (150020) in a pulsing manner when said shaft assembly (120200) is in an orientation just before said threshold orientation;
Illuminate said indicator light (150020) in a constant manner when said shaft assembly (120200) is at said threshold orientation and just past said threshold orientation;
Illuminate said indicator light (150020) in a constant manner when the output response of said control circuit has been switched; and
not illuminate said indicator light (150020) when the output response of said control circuit has not been switched.

8. The surgical instrument assembly of Claim 7, wherein said control circuit is configured to increase the rate in which said indicator light (150020) is pulsed as said shaft assembly (120200) approaches closer to said threshold orientation.

9. The surgical instrument assembly of Claim 6 when said control circuit is configured to illuminate said indicator light (150020) with different colors, wherein said control circuit is configured to:
Illuminate said indicator light (150020) in a first color when said shaft assembly (120200) is in an orientation just before said threshold orientation; and
Illuminate said indicator light (150020) in a second color when said shaft assembly (120200) is at said threshold orientation and just past said threshold orientation;
Illuminate said indicator light (150020) in said second color when the output response of said control circuit has been switched; and
illuminate said indicator light (150020) in a third color when the output response of said control circuit has not been switched, wherein said first color, said second color, and said third color are different.

10. The surgical instrument assembly of Claim 6, wherein said sensor (151320) comprises a Hall Effect sensor.

11. The surgical instrument assembly of Claim 10, further comprising a ring magnet (151310) configured to change the output of said Hall Effect sensor (151320) as the orientation state of said end effector (110400) changes.

12. The surgical instrument assembly of Claim 4, wherein alerting a user as to whether the output response of said control circuit has been switched based on the detected orientation of said shaft assembly (120200) comprises displaying a state of said articulation control actuators on a surgical hub display.

## Patentansprüche

1. Chirurgische Instrumentenanordnung (150000), umfassend:
einen Griff (120100), der Gelenksteueraktuatoren (10160) umfasst;
eine Schaftanordnung (120200), die relativ zu dem Griff (120100) drehbar ist, wobei die Schaftanordnung (120200) umfasst:
einen länglichen Schaft;
eine Gelenkverbindung (120500); und
einen Endeffektor (110400), der relativ zu dem länglichen Schaft mittels des Gelenks gelenkig bewegbar ist; und
eine Steuerschaltung, die konfiguriert ist zum:
Identifizieren eines Ausrichtungszustands der Schaftanordnung (120200) relativ zum Griff (120100);
Zuordnen einer Ausgabe der Steuerschaltung als Reaktion auf eine Eingabe von den Gelenksteueraktuatoren (10160) basierend auf dem identifizierten Ausrichtungszustand; und
Benachrichtigen eines Benutzers darüber, wie die Ausgabe zugeordnet wurde;
wobei der Griff (120100) eine Anzeigeleuchte (150020) umfasst, die mit der Steuerschaltung in Verbindung steht und von der Steuerschaltung entsprechend der Zuordnung des Ausgabesignals beleuchtet wird,
**dadurch gekennzeichnet, dass** entweder:
die Anzeigeleuchte (150020) konfiguriert ist, um mit einer sich allmählich ändernden Geschwindigkeit zu blinken, wobei die Anzeigeleuchte (150020) umso schneller blinkt, je näher die Steuerschaltung an der Neuzuordnung der Ausgabe ist; oder
die Steuerschaltung konfiguriert ist, um die Anzeigeleuchte (150020) in unterschiedlichen Farben aufleuchten zu lassen, je nachdem, wie nahe die Steuerschaltung daran ist, die Ausgabe neu zuzuordnen.

2. Chirurgische Instrumentenanordnung nach Anspruch 1, ferner umfassend einen Sensor (151320), der konfiguriert ist, um eine Änderung des Ausrichtungszustands der Schaftanordnung (120200) zu erkennen.

3. Chirurgische Instrumentenanordnung nach Anspruch 1, wobei das Informieren eines Benutzers darüber, wie die Ausgabe der Steuerschaltung zu Eingaben von den Gelenksteueraktuatoren (10160) zugeordnet wird, das Anzeigen eines Zustands der Gelenksteueraktuatoren (10160) auf einer Anzeige des chirurgischen Hubs umfasst.

4. Chirurgische Instrumentenanordnung nach Anspruch 1, wobei:
der Griff (120100) eine erste laterale Seite und eine der ersten lateralen Seite gegenüberliegende zweite laterale Seite umfasst;
die Gelenksteueraktuatoren (10160) einen ersten Gelenksteueraktuator und einen zweiten Gelenksteueraktuator umfassen, die mit der Steuerschaltung in Verbindung stehen;
die Schaftanordnung (120200) in eine Vielzahl von Ausrichtungszuständen drehbar ist;
wobei die Steuerschaltung ferner konfiguriert ist, sodass:
das Konfigurieren zum Zuordnen der Ausgabe der Steuerschaltung das Konfigurieren zum automatischen Umschalten der Ausgabereaktion der Steuerschaltung umfasst, damit sie mit dem identifizierten Ausrichtungszustand übereinstimmt, sodass der Endeffektor (110400) immer in Richtung der ersten lateralen Seite des Griffs (120100) gelenkig bewegt wird, wenn der erste Gelenkaktuator (10160) betätigt wird, und immer in Richtung der zweiten lateralen Seite des Griffs (120100) gelenkig bewegt wird, wenn der zweite Gelenkaktuator (10160) betätigt wird, und zwar unabhängig vom Ausrichtungszustand der Schaftanordnung (120200) relativ zum Griff (120100); und
das Konfigurieren zum Benachrichtigen eines Benutzers darüber, wie die Ausgabe zugeordnet wurde, das Konfigurieren zum Benachrichtigen des Benutzers des chirurgischen Instruments umfasst, dass die Ausgabereaktion der Steuerschaltung umgeschaltet wurde, um den identifizierten Ausrichtungszustand der Schaftanordnung (120200) zu kompensieren.

5. Chirurgische Instrumentenanordnung nach Anspruch 4, wobei die Steuerschaltung konfiguriert ist, um die Anzeigeleuchte (150020) aufleuchten zu lassen, wenn die Ausgabereaktion der Steuerschaltung umgeschaltet wurde, um den identifizierten Ausrichtungszustand der Schaftanordnung (120200) zu kompensieren.

6. Chirurgische Instrumentenanordnung nach Anspruch 5, ferner umfassend einen Sensor, der mit der Steuerschaltung in Verbindung steht, um zu erkennen, wenn die Schaftanordnung (120200)
bei einer Schwellenausrichtung ist, bei der die Ausgabereaktion der Steuerschaltung umgeschaltet wird;
in einer Ausrichtung unmittelbar vor der Schwellenausrichtung ist; und
in einer Ausrichtung knapp über der Schwellenausrichtung ist.

7. Chirurgische Instrumentenanordnung nach Anspruch 6, wobei die Anzeigeleuchte (150020) konfiguriert ist, um mit einer sich allmählich ändernden Geschwindigkeit zu blinken, wobei die Steuerschaltung konfiguriert ist zum:
pulsierenden Erleuchten der Anzeigeleuchte (150020), wenn sich die Schaftanordnung (120200) in einer Ausrichtung unmittelbar vor der Schwellenausrichtung befindet;
konstantes Erleuchten der Anzeigeleuchte (150020), wenn sich die Schaftanordnung (120200) in der Schwellenausrichtung befindet oder die Schwellenausrichtung gerade überschritten hat;
konstantes Erleuchten der Anzeigeleuchte (150020), wenn die Ausgabereaktion der Steuerschaltung umgeschaltet wurde; und
Nicht Erleuchten der Anzeigeleuchte (150020), wenn die Ausgabereaktion der Steuerschaltung nicht umgeschaltet wurde.

8. Chirurgische Instrumentenanordnung nach Anspruch 7, wobei die Steuerschaltung konfiguriert ist, um die Frequenz zu erhöhen, mit der die Anzeigeleuchte (150020) gepulst wird, während sich die Schaftanordnung (120200) der Schwellenausrichtung nähert.

9. Chirurgische Instrumentenanordnung nach Anspruch 6, wobei die Steuerschaltung konfiguriert ist, um die Anzeigeleuchte (150020) in unterschiedlichen Farben aufleuchten zu lassen, wobei die Steuerschaltung konfiguriert ist zum:
Erleuchten der Anzeigeleuchte (150020) in einer ersten Farbe, wenn sich die Schaftanordnung (120200) in einer Ausrichtung unmittelbar vor der Schwellenausrichtung befindet; und
Erleuchten der Anzeigeleuchte (150020) in einer zweiten Farbe, wenn sich die Schaftanordnung (120200) in der Schwellenausrichtung befindet und diese Schwellenausrichtung gerade überschritten hat;
Erleuchten der Anzeigeleuchte (150020) in der zweiten Farbe, wenn die Ausgabereaktion der Steuerschaltung umgeschaltet wurde; und
Erleuchten der Anzeigeleuchte (150020) in einer dritten Farbe, wenn die Ausgabereaktion der Steuerschaltung nicht umgeschaltet wurde, wobei die erste Farbe, die zweite Farbe und die dritte Farbe unterschiedlich sind.

10. Chirurgische Instrumentenanordnung nach Anspruch 6, wobei der zweite Sensor (151320) einen Hall-Effekt-Sensor umfasst.

11. Chirurgische Instrumentenanordnung nach Anspruch 10, ferner umfassend einen Ringmagneten (151310), der konfiguriert ist, um die Ausgabe des Hall-Effekt-Sensors (151320) zu ändern, wenn sich der Ausrichtungszustand des Endeffektors (110400) ändert.

12. Chirurgische Instrumentenanordnung nach Anspruch 4, wobei das Benachrichtigen eines Benutzers, ob die Ausgabereaktion der Steuerschaltung basierend auf der identifizierten Ausrichtung der Schaftanordnung (120200) umgeschaltet wurde, das Anzeigen eines Zustands der Gelenksteueraktuatoren auf einer Anzeige des chirurgischen Hubs umfasst.

## Revendications

1. Ensemble instrument chirurgical (150000), comprenant :
une poignée (120100) comprenant des actionneurs de commande d'articulation (10160) ;
un ensemble tige (120200) rotatif par rapport à ladite poignée (120100), dans lequel ledit ensemble tige (120200) comprend :
une tige allongée ;
une jonction articulée (120500) ; et
un effecteur terminal (110400) articulable par rapport à ladite tige allongée au moyen de ladite jonction articulée ; et
un circuit de commande configuré pour :
identifier un état d'orientation dudit ensemble tige (120200) par rapport à ladite poignée (120100) ;
mapper une sortie dudit circuit de commande en réponse à une entrée provenant desdits actionneurs de commande d'articulation (10160) en fonction dudit état d'orientation identifié ; et
alerter un utilisateur concernant la manière dont ladite sortie a été mappée ;
dans lequel ladite poignée (120100) comprend un indicateur lumineux (150020) en communication avec ledit circuit de commande qui est illuminé par ledit circuit de commande en correspondance avec la manière dont ladite sortie a été mappée, **caractérisé en ce que** soit :
ledit indicateur lumineux (150020) est configuré pour clignoter avec une vitesse changeant progressivement où, plus ledit circuit de commande est proche de remapper ladite sortie, plus ledit indicateur lumineux (150020) clignote rapidement ; ou
ledit circuit de commande est configuré pour illuminer ledit indicateur lumineux (150020) avec des couleurs différentes correspondant à la manière dont ledit circuit de commande est proche de remapper ladite sortie.

2. Ensemble instrument chirurgical selon la revendication 1, comprenant en outre un capteur (151320) configuré pour détecter un changement dans l'état d'orientation dudit ensemble tige (120200).

3. Ensemble instrument chirurgical selon la revendication 1, dans lequel le fait d'alerter un utilisateur concernant la manière dont la sortie dudit circuit de commande est mappée à des entrées provenant desdits actionneurs de commande d'articulation (10160) comprend l'affichage d'un état desdits actionneurs de commande d'articulation (10160) sur un affichage de contrôleur chirurgical central.

4. Ensemble instrument chirurgical selon la revendication 1, dans lequel :
ladite poignée (120100) comprend un premier côté latéral et un second côté latéral opposé audit premier côté latéral ;
lesdits actionneurs de commande d'articulation (10160) comprennent un premier actionneur de commande d'articulation et un second actionneur de commande d'articulation en communication avec ledit circuit de commande ;
ledit ensemble tige (120200) peut effectuer une rotation dans une pluralité d'états d'orientation ;
dans lequel ledit circuit de commande est configuré en outre de telle sorte que :
le fait d'être configuré pour mapper la sortie dudit circuit de commande comprend le fait d'être configuré pour commuter automatiquement la réponse de sortie dudit circuit de commande pour qu'elle coïncide avec l'état d'orientation identifié de telle sorte que ledit effecteur terminal (110400) est toujours articulé en direction dudit premier côté latéral de ladite poignée (120100) lorsque ledit premier actionneur d'articulation (10160) est actionné et toujours articulé en direction dudit second côté latéral de ladite poignée (120100) lorsque ledit second actionneur d'articulation (10160) est actionné indépendamment de l'état d'orientation dudit ensemble tige (120200) par rapport à ladite poignée (120100) ; et
le fait d'être configuré pour alerter un utilisateur concernant la manière dont ladite sortie a été mappée comprend le fait d'être configuré pour alerter ledit utilisateur dudit instrument chirurgical que la réponse de sortie dudit circuit de commande a été commutée pour compenser l'état d'orientation identifié dudit ensemble tige (120200).

5. Ensemble instrument chirurgical selon la revendication 4, dans lequel ledit circuit de commande est configuré pour illuminer ledit indicateur lumineux (150020) lorsque la réponse de sortie dudit circuit de commande a été commutée pour compenser l'état d'orientation identifié dudit ensemble tige (120200).

6. Ensemble instrument chirurgical selon la revendication 5, comprenant en outre un capteur en communication avec ledit circuit de commande pour détecter le moment où ledit ensemble tige (120200) est :
à une orientation seuil dans laquelle la réponse de sortie dudit circuit de commande est commutée ;
dans une orientation juste avant ladite orientation seuil ; et
dans une orientation juste au-delà de ladite orientation seuil.

7. Ensemble instrument chirurgical selon la revendication 6 lorsque ledit indicateur lumineux (150020) est configuré pour clignoter avec une vitesse changeant progressivement, dans lequel ledit circuit de commande est configuré pour :
illuminer ledit indicateur lumineux (150020) d'une manière pulsée lorsque ledit ensemble tige (120200) est dans une orientation juste avant ladite orientation seuil ;
illuminer ledit indicateur lumineux (150020) d'une manière constante lorsque ledit ensemble tige (120200) est à ladite orientation seuil et juste au-delà de ladite orientation seuil ;
illuminer ledit indicateur lumineux (150020) d'une manière constante lorsque la réponse de sortie dudit circuit de commande a été commutée ; et
ne pas illuminer ledit indicateur lumineux (150020) lorsque la réponse de sortie dudit circuit de commande n'a pas été commutée.

8. Ensemble instrument chirurgical selon la revendication 7, dans lequel ledit circuit de commande est configuré pour augmenter la fréquence à laquelle ledit indicateur lumineux (150020) est pulsé à mesure que ledit ensemble tige (120200) se rapproche de ladite orientation seuil.

9. Ensemble instrument chirurgical selon la revendication 6 lorsque ledit circuit de commande est configuré pour illuminer ledit indicateur lumineux (150020) avec des couleurs différentes, dans lequel ledit circuit de commande est configuré pour :
illuminer ledit indicateur lumineux (150020) dans une première couleur lorsque ledit ensemble tige (120200) est dans une orientation juste avant ladite orientation seuil ; et
illuminer ledit indicateur lumineux (150020) dans une deuxième couleur lorsque ledit ensemble tige (120200) est à ladite orientation seuil et juste au-delà de ladite orientation seuil ;
illuminer ledit indicateur lumineux (150020) dans ladite deuxième couleur lorsque la réponse de sortie dudit circuit de commande a été commutée ; et
illuminer ledit indicateur lumineux (150020) dans une troisième couleur lorsque la réponse de sortie dudit circuit de commande n'a pas été commutée, dans lequel ladite première couleur, ladite deuxième couleur et ladite troisième couleur sont différentes.

10. Ensemble instrument chirurgical selon la revendication 6, dans lequel ledit capteur (151320) comprend un capteur à effet Hall.

11. Ensemble instrument chirurgical selon la revendication 10, comprenant en outre un aimant annulaire (151310) configuré pour changer la sortie dudit capteur à effet Hall (151320) à mesure que l'état d'orientation dudit effecteur terminal (110400) change.

12. Ensemble instrument chirurgical selon la revendication 4, dans lequel le fait d'alerter un utilisateur quant à savoir si la réponse de sortie dudit circuit de commande a été commutée en fonction de l'orientation détectée dudit ensemble tige (120200) comprend l'affichage d'un état desdits actionneurs de commande d'articulation sur un affichage de contrôleur chirurgical central.
